# EUROPEAN PATENT APPLICATION

(11) **EP 1 125 931 A1**
(43) Date of publication of application: **22.08.2001**
(21) Application number: 00301260.6
(22) Date of filing: 17.02.2000
(51) Int. Cl.: C07D 251/54, C07D 251/34, C07C 323/47, C07D 213/53, C07D 239/26, C07D 307/52, C07D 307/81, C07D 207/32, C07D 213/61, C07D 213/62, C07D 307/42, C07D 409/12, C07D 405/12, C07D 333/22, C07D 333/58, A01N 43/40, A01N 35/10

(54) **Biocidal alkyl-substituted-(hetero)aryl-ketoxime-O-ethers and the production method thereof**

(71) Applicant: Hunan Research Institute of Chemical Industry, Hunan Sheng, 410007 (CN)
(72) Inventor: Liu, Aiping, Changsha, Hunan 410007 (CN); Long, Shengyou, Changsha, Hunan 410007 (CN); Ou, Xiaoming, Changsha, Hunan 410007 (CN); Ren, Xunhe, Changsha, Hunan 410007 (CN); Liu, Shudong, Changsha, Hunan 410007 (CN); Wang, Yongjiang, Changsha, Hunan 410007 (CN); Hou, Zhongke, Changsha, Hunan 410007 (CN); Yu, Zhengying, Changsha, Hunan 410007 (CN); Huang, Mingzhi, Changsha, Hunan 410007 (CN); Xu, Jianbing, Changsha, Hunan 410007 (CN)
(74) Representative: Buckley, Guy Julian

(57) **Abstract**

This invention provides a series of alkyl-substituted-(hetero)aryl-ketoxime-o-ether compounds represented by the general formula (I), a series of intermediate ketones and oxime compounds represented by the general formula (II) and general formula (III), their production method and biological activities.

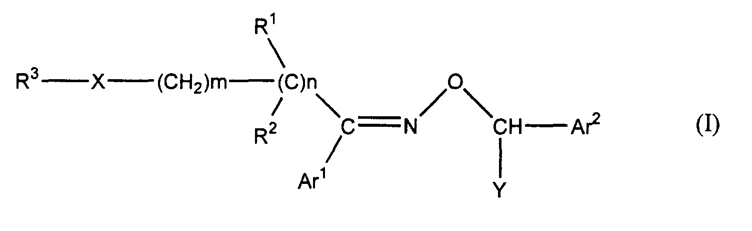

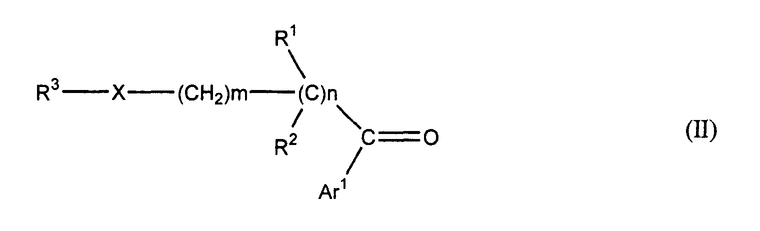

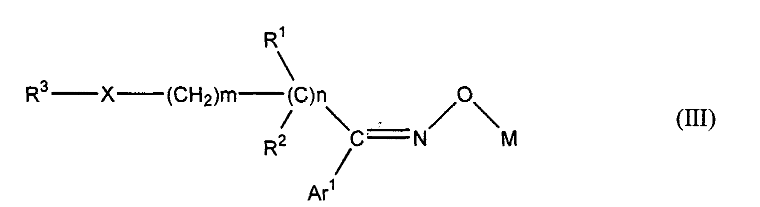

The compounds represented by general formula (I) have excellent biological activity for controlling insects, fungi, and weeds. They show particularly high activity in insect pest control such as in agriculture, horticulture, flower cultivation, and hygiene field. The compounds represented by the general formula (I) have quick and persistent insecticidal activity, without harming the crops. The compounds represented by general formula (II) and (III) that serve as the intermediates also have fairly good biological activity.

## Description

This invention relates to a series of biocidal alkyl-substituted-(hetero)aryl-ketoxime-o-ethers, intermediate ketones, and oxime compounds, and their production methods.

There are numerous domestic and foreign literature about the study and application of alkyl-substituted-(hetero)aryl-ketoxime-o-ethers as agricultural chemicals, and some of them have been offered as agricultural chemical products. Related literature and patents are as follows : Bull. M. J., Davics, J. H., Searle, J.G., Henry, A.G., Pestic. Sci., II, 249(1980); Paul, Jill Helaine, et al., Eur. Pat. Appl. 4,754, US Appl. 891,991; Jpn. Kokai Tokkyo Koho 80, 115,864; B. Kuhn, G. Zabek, et al., CN 1077709A(DE P4213149.9); CN 1059515A; DE 4442730A; DE 2806664A; T. Nishioka, et al. Jpn. Kokai Tokkyo Koho, 55-17323, 54-13852. Agricultural chemical products are as follows : Oxime ether pyrethrum esters (CA Registration No. 69043-27-2), and so on. Alkyl-substituted-(hetero)aryl-ketoxime-o-ethers have excellent biocidal activity. They have, not only many characteristic features such as broad spectrum, high efficiency and low toxicity, but also the pyrethrum type biological activity such as a quick knockout power. The alkyl-substituted-(hetero)aryl-ketoxime-o-ethers can be regarded as a new type of agricultural chemical and deserve an extensive development and application. In order to find a new alkyl-substituted-(hetero)aryl-ketoxime-o-ether type agricultural chemical species, the Hunan Chemical and Industrial Research Institute has conducted an extensive study on this type of compounds and has filed a patent application for some of the new biologically active compounds (Application No. 98-112665.0). Through further investigation, we have discovered a series of new alkyl-substituted-(hetero)aryl-ketoxime-o-ether compounds that have excellent agricultural chemical property, and particularly have excellent insecticidal property.

This invention relates to a series of alkyl-substituted-(hetero)aryl-ketoxime-o-ethers represented by the general formula (I), and a series of intermediate ketones and oxime compounds represented by general formula (II) and general formula (III). 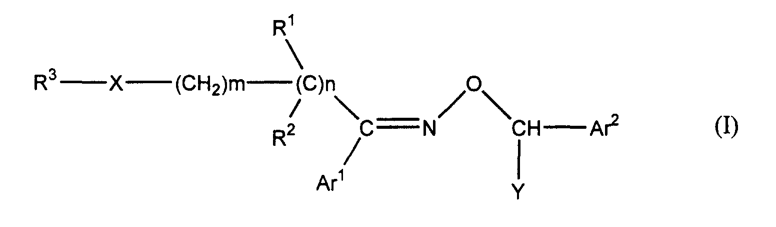 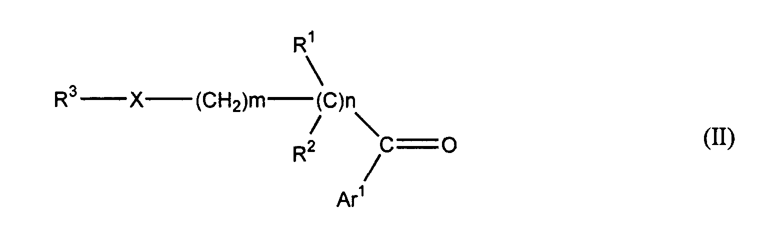 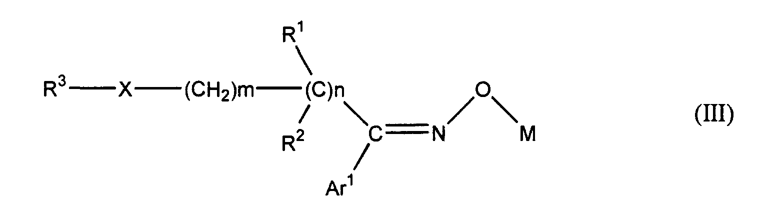 In the general formula (I), (II) and (III) :
I. Ar1 and Ar2 may be identical or different, and they represent:
   (a) (C₆ - C₁₂) aryl group or heteroaryl group having no more than 10 carbon atoms, or
   (b) the group defined in I.(a) and, if necessary, it is substituted by no more than 5 identical or different substituting groups described below: halogens, nitro group, cyano group, (C₁ - C₆) alkyl groups, (C₁ - C₆) halogen-substituted alkyl groups, cyano-(C₁ - C₆) alkyl groups, (C₁ - C₆) alkoxy-(C₁ - C₆) alkyl groups, (C₁ - C₆)-alkylthio group-(C₁ - C₆) alkyl groups, (C₁ - C₆) alkoxy groups, (C₁ - C₆) alkoxy-(C₁ - C₆) alkoxy groups, (C₁ - C₆) halogen-substituted alkoxy groups, (C₁ - C₆) halogen-substituted-alkoxy-alkyl groups, (C₁ - C₆)alkylthio groups, (C₁ - C₆) halogen-substituted alkylthio groups, (C₁ - C₆) alkyl-sulfonyl groups, (C₁ - C₆) alkyl-sulfinyl groups, (C₁ - C₆) alkoxyl-carbonyl groups, (C₁ - C₆) alkylamino group, 2-(C₁ - C₆) alkylamino groups, linear (C₂ - C₆) alkenyl groups, linear (C₂ - C₆) alkenoxy groups, linear (C₂ - C₆) alkenoxyalkyl groups, linear (C₂ - C₆) halogen-substituted alkenyl groups, linear (C₂ - C₆) halogen-substituted alkenoxy group, linear (C₂ - C₆) halogen-substituted alkenoxy-alkyl groups, (C₂ - C₆) alkynyl groups, (C₂ - C₆) alkynoxy groups, (C₂ - C₆) halogen-substituted alkynyl groups, (C₂ - C₆) halogen-substituted alkynoxy groups, (C₃ - C₈) cycloalkyl groups, (C₃ - C₈) cycloalkyloxy groups, (C₃ - C₈) cycloalkylamino groups, (C₆ - C₁₂) aryl groups, (C₆ - C₁₂) aryl-thio groups, (C₆ -C₁₂) aryl-(C₁ - C₄) alkyl groups, (C₆ - C₁₂) aryloxy-carbonyl groups, (C₆ - C₁₂) aryl-sulfonyl groups, (C₆ - C₁₂) aryl-sulfinyl groups, (C₆ - C₁₂ arylamino group, heteroaryl groups, heteroaryloxy groups, heteroaryl-(C₆ - C₁₂) alkyl group, heteroaryl-thio groups, heteroaryloxy-carbonyl groups, heteroaryl-sulfonyl groups, heteroaryl-sulfinyl groups, and
      1) when the substituting group in I.(b) is an aryl group or heteroaryl group, it may be substituted by one or more identical or different group(s) selected from (C₁ - C₆) alkyl groups, (C₁ - C₆) alkoxy groups, (C₁ - C₆) halogen-substituted alkyl groups, (C₁ - C₆) halogen-substituted alkoxy group and halogens, and the heteroaryl group is the group having no more than 10 carbon atoms,
      2) the cycloalkyl group described in I.(b) may be substituted by no more than five identical or different groups selected from halogens and (C₁ - C₄) alkyl groups,
      3) two of the substituting groups described in I.(b) represent methylene dioxy group and ethylene dioxide group, which may have one or two identical or different substituting group(s) selected from halogens and (C₁ - C₆) alkyl groups.
      4) the aryl group and the heteroaryl group defined in I.(a) and I.(b) may be hydrogenated partially or entirely, and one or two CH₂ groups may be substituted by CO,
II. R₁ and R₂ may be identical or different, representing the following: hydrogen, halogens, (C₁ - C₆) alkyl groups, (C₁ - C₆) halogenated-alkyl groups, cyano-(C₁ - C₆) alkyl groups, (C₁ - C₆) alkoxy-(C₁ - C₆) alkyl groups, (C₁ - C₆) alkylthio-(C₁ - C₆) alkyl groups, (C₁ - C₆) alkoxy-(C₁ - C₆) alkoxy groups, (C₁ -C₆) alkoxy groups, (C₁ - C₆) alkylthio groups, (C₁ - C₆) alkyl-sulfonyl groups, (C₁ - C₆) alkyl-sulfinyl groups, (C₁ - C₆) alkoxy-carbonyl groups, (C₁ - C₆) alkylamino groups, 2-(C₁ - C₆) alkylamino groups, (C₁ - C₆) halogen-substituted alkoxy groups, linear (C₂- C₆) alkenyl groups, linear (C₂ - C₆) alkenoxy groups, linear (C₂ - C₆) halogenated- alkenyl groups, linear (C₂ - C₆) halogenated-alkenoxy groups, (C₂ - C₆) alkynyl groups, (C₂ - C₆) alkynoxy groups, (C₂ - C₆) halogenated-alkynyl groups, (C₂ - C₆) halogenated-alkynoxy groups, (C₃ - C₈) cycloalkyl groups, (C₃ - C₈) cycloalkyloxy groups, (C₃ - C₈) cycloalkylamino groups, (C₆ - C₁₂) aryl groups, (C₆ - C₁₂) aryloxy groups, (C₆ - C₁₂) aryl-(C₁ - C₄) alkyl groups, (C₆ - C₁₂) arylthio groups, (C₆ - C₁₂) aryloxy-carbonyl groups, (C₆ - C₁₂) aryl-sulfonyl groups, (C₆ - C₁₂) aryl-sulfinyl groups, (C₆ - C₁₂) arylamino groups, heteroaryl groups, heteroaryloxy groups, heteroaryl-(C₁ - C₄) alkyl groups, heteroarylthio groups, heteroaryloxy-carbonyl groups, heteroaryl sulfonyl groups, and heteroaryl sulfinyl groups,
III. R₃ represents the following:
   (a) hydrogen,
   (b) (C₁ - C₆) alkyl groups, (C₆ - C₁₂) aryl groups, heteroaryl groups with no more than 10 carbon atoms, linear (C₂ - C₆) alkenyl groups, (C₂ - C₆) alkynyl groups, and (C₁ - C₄) acyl groups. However, the groups described in III.(b) may be substituted by identical or different groups selected from halogens, (C₁ - C₄) alkyl groups, (C₁ - C₄) alkoxy groups, (C₁ - C₄) halogen-substituted alkyl groups, (C₁ - C₄) alkyl-thio groups, and (C₁ - C₄) halogenated alkoxy groups,
IV.X represents, O, S, SO, SO₂, NH, or NR^{a} (where, R^{a} represents a (C₁ - C₄) alkyl group),
V. Y represents hydrogen, cyano group, halogens, (C₁ - C₄) alkyl group, or (C₁ - C₄) alkoxy group,
VI.M is a hydrogen or a monobasic metallic atom,
VII. m or n independently represent 0, 1, 2 or 3.

The above-described alkyl groups, linear alkenyl groups and alkynyl groups may be a straight chain or branched. Meantime, they may be applied to the groups derived from them, such as alkyloxy groups, alkyloxy carbonyl groups, alkylthio groups, halogenated alkyl groups and arylalkyl groups originated from the alkyl group.

Halogenated alkyl group, halogenated linear alkenyl group and halogenated alkynyl group are the alkyl groups, linear alkenyl groups and alkynyl groups where one, more or all hydrogen atoms have been substituted by halogens. Meantime, they may be applied to the groups derived from them. For example, the groups derived from halogenated alkyl group are halogenated alkyloxy groups, halogenated alkylthio groups, halogenated alkyloxy carbonyl groups and aryl halogenated alkyl groups.

Halogens are fluorine, chlorine, bromine or iodine, and preferred halogens are fluorine, chlorine and bromine.

Preferred (C₆ - C₁₂) aryl groups are phenyl group and the group derived from phenyl group, such as naphthyl group, biphenyl group and so on.

Preferred heteroaryl group having no more than 10 carbon atoms are mono or bicyclic aryl group, where the formula contains at least one N, O, or S, such as thiophene group, benzothiophene group, furan group, benzofuran group, pyrrole group, indole group, imidazole group, pyrazole group, pyridine group, pyrazine group, pyrimidine group, diazine group, oxazole group, isoxazole group, thiazole group and isothiazole group.

(C₆ - C₁₂) aryl group and heteroaryl group having no more than 10 carbon atoms may be partially or entirely hydrogenated. And, one or two CH₂ group(s) is substituted by CO, such as cyclohexene group, cyclohexadione group and so on.

In the general formula (I), (II) and (III), Ar1 is preferably the following groups : 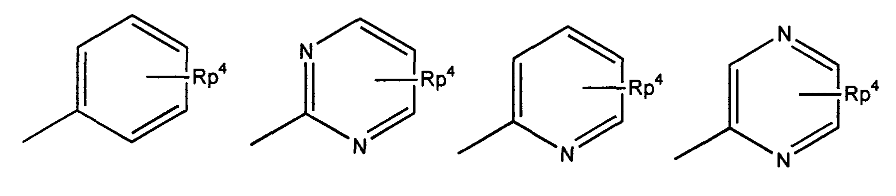 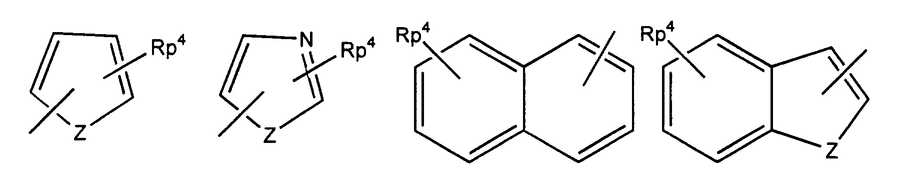
Z is O, S or NR⁵, and R⁵ is a hydrogen, (C₁ - C₆) alkyl groups, (C₁ - C₆) halogen-substituted alkyl groups, (C₁ - C₆) alkoxy-(C₁ - C₆) alkyl groups, (C₁ - C₆) alkylthio-(C₁ - C₆) alkyl groups, cyano-(C₁ - C₆) alkyl groups, (C₁ - C₆) alkyloxy carbonyl groups, linear (C₂ - C₆) alkenyl groups, linear (C₂ - C₆) halogenated alkenyl groups, (C₂ - C₆) alkynyl groups, (C₂ - C₆) halogenated alkynyl groups, (C₃ - C₈) cycloalkyl groups, the cycloalkyl group may be substituted by no more than five identical or different groups selected from halogens and (C₁ - C₄) alkyl groups, (C₆ - C₁₂) aryl groups, (C₆ - C₁₂) aryl-(C₁ - C₄) alkyl group, (C₆ - C₁₂) aryloxy carbonyl group, and heteroaryl groups, heteroaryloxy carbonyl group, and heteroaryl-(C₁ - C₄) alkyl groups having no more than 10 carbons,
P is an integer from 0 to 5,
R⁴ is identical or different, and represents, halogens, nitro group, cyano group, (C₁ - C₆) alkyl groups, (C₁ - C₆) halogenated alkyl groups, cyano-(C₁ - C₆) alkyl groups, (C₁ - C₆) alkoxy-(C₁ - C₆) alkyl groups, (C₁ - C₆) alkylthio-(C₁ - C₆) alkyl groups, (C₁- C₆) alkoxy groups, (C₁ - C₆) alkoxy-(C₁ - C₆) alkoxy groups, (C₁ - C₆) halogenated alkoxy groups, (C₁ - C₆) halogenated alkoxy-alkyl groups, (C₁ - C₆) alkylthio groups, (C₁ - C₆) halogenated alkylthio groups, (C₁ - C₆) alkyl-sulfonyl groups, (C₁ - C₆) alkyl-sulfinyl groups, (C₁ - C₆) alkoxy-carbonyl groups, (C₁ - C₆) alkylamino groups, 2-(C₁ - C₆) alkylamino groups, linear (C₂ - C₆) alkenyl groups, linear (C₂ - C₆) alkenoxy groups, linear (C₂ - C₆) alkenoxy alkyl groups, linear (C₂ - C₆) halogenated alkenyl groups, linear (C₂ - C₆) halogenated alkenyloxy groups, linear (C₂ - C₆) halogenated alkenoxy-alkyl group, (C₂ - C₆) alkynyl groups, (C₂ - C₆) alkenoxy groups, (C₂ - C₆) halogenated alkynyl groups, (C₂ - C₆) halogenated alkynoxy groups, (C₃ - C₈) cycloalkyl groups, (C₃ - C₈) cycloalkyloxy groups, (C₃ - C₈) cycloalkylamino groups, (C₆ - C₁₂) aryl groups, (C₆ - C₁₂) aryloxy groups, (C₆ - C₁₂) arylthio groups, (C₆ - C₁₂) aryl-(C₁ - C₄) alkyl groups, (C₆ - C₁₂) aryloxy carbonyl groups, (C₆ - C₁₂) aryl-sulfonyl groups, (C₆ - C₁₂) arylsulfinyl groups, (C₆ - C₁₂) arylamino groups, heteroaryl groups, heteroaryloxy groups, heteroaryl-(C₁ - C₄) alkyl groups, heteroaryl-thio groups, heteroaryloxy carbonyl groups, heteroaryl-sulfonyl groups and heteroaryl-sulfinyl groups, and
Preferred R⁴ group is a hydrogen, halogens, (C₁ - C₆) alkyl groups, (C₁ - C₆) halogenated alkyl groups, (C₁ - C₆) alkoxy groups, (C₁ - C₆) alkoxy-(C₁ - C₆) alkyl groups, (C₁ - C₆) alkoxy-(C₁ - C₆) alkoxy groups, (C₁ - C₆) halogenated alkoxy groups, (C₁ - C₆) alkyl-sulfonyl groups, linear (C₂ - C₆) alkenyl groups, linear (C₂ - C₆) alkenoxy groups, linear (C₂ - C₆) halogenated alkenyl groups, linear (C₂ - C₆) halogenated alkenoxy groups, (C₂ - C₆) alkynyl groups, (C₂ - C₆) alkynoxy groups, (C₂ - C₆) halogenated alkynyl groups, (C₂ - C₆) halogenated alkynoxy groups, (C₃ - C₈) cycloalkyl groups, (C₃ - C₈) cycloalkyloxy groups, (C₃ - C₈) halogenated cycloalkyl groups, (C₆ - C₁₂) aryl groups, (C₆ - C₁₂) aryloxy groups, (C₆- C₁₂) aryl-(C₁ - C₄) alkyl groups, (C₆ - C₁₂) arylthio groups, heteroaryl groups having no more than 10 carbon atoms, heteroaryloxy groups, heteroaryl-(C₁ - C₄) alkyl groups and so on, and
   1) when the substituting group in R⁴ is an aryl group or a hetero-aryl group, it may be substituted by one or more identical or different group(s) selected from (C₁ - C₆) alkyl groups, (C₁ - C₆) alkoxy groups, (C₁ - C₆) halogen-substituted alkyl groups, (C₁ - C₆) halogenated alkoxy groups and halogens, and the heteroaryl group is the one having no more than 10 carbon atoms,
   2) the cycloalkyl group described in R⁴ may be substituted by no more than five identical or different groups selected from halogens and (C₁ - C₄) alkyl groups,
   3) two of the substituting groups described in R⁴ represent methylene dioxy group or ethylene dioxy group, which may have one or two identical or different substituting group(s) selected from halogens and (C₁ - C₆) alkyl groups.

In the general formula (I), (II) and (III), the Ar1 is preferably a group described in the following.

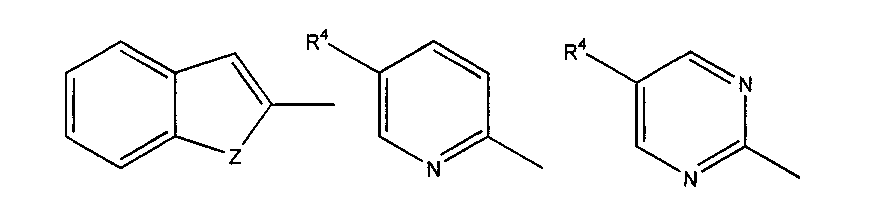

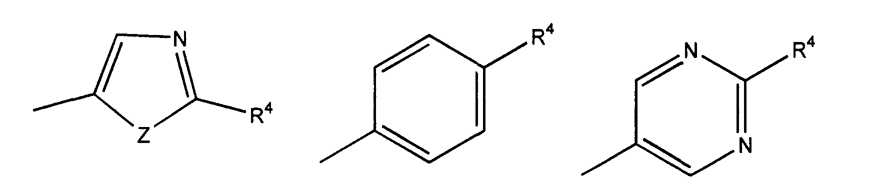

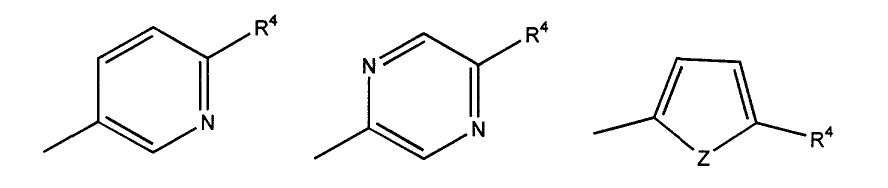

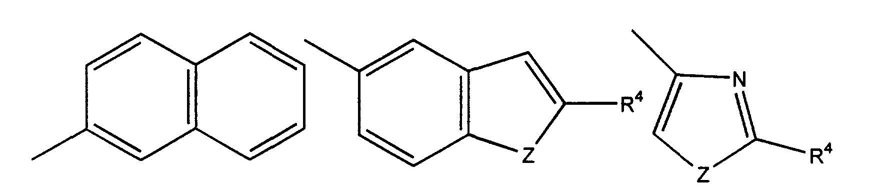

In these formula, Z is O, S or NR⁵, where R⁵ is a hydrogen, (C₁ - C₆) alkyl groups, (C₁ - C₆) halogenated alkyl groups, (C₁ - C₆) alkoxy-(C₁ - C₆) alkyl groups, (C₁ - C₆) alkoxy carbonyl groups, linear (C₂ - C₆) alkenyl groups, linear (C₂ - C₆) halogenated alkenyl groups, linear (C₂ - C₆) halogenated alkenyl groups, (C₂ - C₆) alkynyl groups, and (C₂ - C₆) halogenated alkynyl groups.

R⁴ is a hydrogen, halogens, (C₁ - C₆) alkyl groups, (C₁ - C₆) halogenated alkyl groups, (C₁ - C₆) alkoxy-(C₁ - C₆) alkyl groups, (C₁ - C₆) alkoxy-(C₁ - C₆) alkoxy groups, (C₁ - C₆) halogenated alkoxy groups, (C₁ - C₆) halogenated alkoxy groups, (C₁ - C₆) alkyl-sulfonyl groups, linear (C₂ - C₆) alkenyl groups, linear (C₂ - C₆) alkenoxy groups, linear (C₂ - C₆) halogenated alkenyl groups, linear (C₂ - C₆) halogenated alkenoxy groups, (C₂ - C₆) alkynyl groups, (C₂ - C₆) alkynoxy groups, (C₂ - C₆) halogenated alkynyl groups, (C₂ - C₆) halogenated alkynoxy groups, (C₃ - C₈) cycloalkyl groups, and (C₃ - C₈) halogenated cycloalkyl groups.

Two of the substituting groups in R⁴ represent methylene dioxide group and ethylene dioxide group, which may have one or two identical or different groups selected from halogens and (C₁ - C₆) alkyl groups.

The R₁ and R₂ in the general formula (I), (II), and (III) are identical or different, and preferably are hydrogen, halogens, (C₁ - C₄) alkyl groups, (C₁ - C₄) alkoxyl groups, (C₁ - C₄) halogenated alkyl groups, (C₁ - C₄) alkylthio groups, linear (C₁ - C₄) alkenyl groups, (C₁ - C₄) alkynyl groups, linear (C₁ - C₄) halogenated alkenyl groups, (C₁ - C₄) halogenated alkynyl groups, (C₁ - C₄) alkyl-sulfonyl groups, (C₁ - C₄) alkyl-sulfinyl groups, and (C₁ - C₄) alkylamino groups.

The R₃ in the general formula (I), (II) and (III) are preferably hydrogen, (C₁ - C₄) alkyl groups, (C₁ - C₄) halogenated alkyl groups, linear (C₁ - C₄) alkenyl groups, (C₁ - C₄) alkynyl groups, linear (C₁ - C₄) halogenated alkenyl groups, (C₁ - C₄) halogenated alkynyl groups, and (C₆ - C₁₂) aryl groups.

In the general formula (I), (II), and (III), m is preferably 0, and n is preferably 1.

In the general formula (I), (II), and (III), and preferred Ar2 is the following: 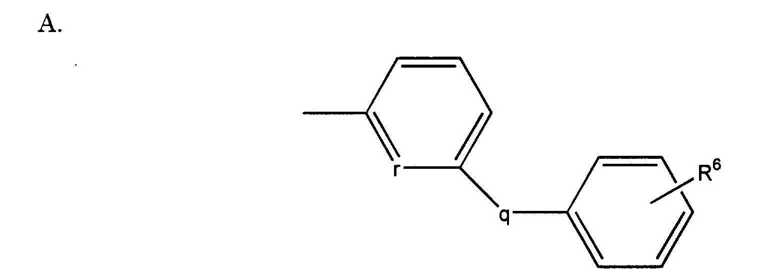 r represents N or CH, and q is O, S, NH or CH₂, preferably O or CH₂.

And, R⁶ is a hydrogen, halogens, acryloxy group or propargyloxy . 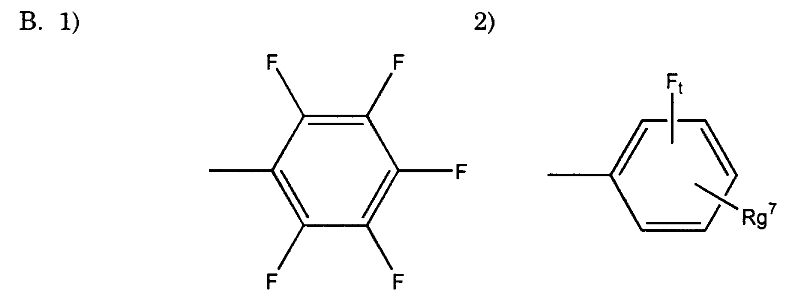
F represents fluorine,
t and g are identical or different, and they represent 0, 1, 2, 3, or 4, and sum of t and g is no larger than 5,
R⁷ may be identical or different, it represents a hydrogen, halogens, (C₁ - C₆) alkyl groups, (C₁ - C₆) halogenated alkyl groups, (C₁ - C₆) alkoxy groups, (C₁ - C₆) alkoxy-(C₁ - C₆) alkyl groups, (C₁ - C₆) alkoxy-(C₁ - C₆) alkoxy groups, (C₁ - C₆) halogenated alkoxy groups, (C₁ - C₆) alkyl-sulfonyl groups, linear (C₂ - C₆) alkenyl groups, linear (C₂ - C₆) alkenoxy groups, linear (C₂ - C₆) alkenoxy-alkyl groups, linear (C₂ - C₆) halogenated alkenyl groups, linear (C₂ - C₆) halogenated alkenoxy groups, linear (C₂ - C₆) halogenated alkenoxy-alkyl groups, (C₂ - C₆) alkynyl groups, (C₂ - C₆) alkenoxy groups, (C₂ - C₆) halogenated alkynyl groups, (C₂ - C₆) halogenated alkynoxy groups, (C₃ - C₈) cycloalkyl groups, (C₃ - C₈) cycloalkyloxy groups, (C₃ - C₈) halogenated cycloalkyl groups, (C₆ - C₁₂) aryl groups, (C₆ - C₁₂) aryloxy groups, (C₆ - C₁₂) aryl-(C₁ - C₄) alkyl groups, (C₆ - C₁₂) aryl-thio groups, heteroaryl groups having no more than 10 carbon atoms, heteroaryloxy groups, heteroaryl-(C₁ - C₄) alkyl groups,

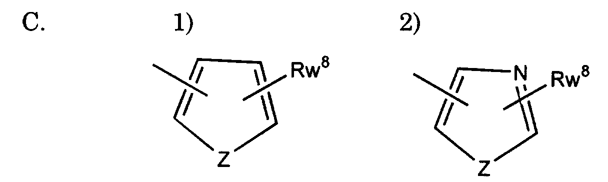

In the above formula, w is 1, 2, or 3, and
Z is O, S, or NR⁵ and R⁵ represents a hydrogen, (C₁ - C₆) alkyl groups, (C₁ - C₆) halogenated alkyl groups, (C₁ - C₆) alkoxy- (C₁ - C₆) alkyl groups, (C₁ - C₆)alkylthio-(C₁ - C₆)alkyl groups, cyano-(C₁ - C₆) alkyl groups, (C₁ - C₆) alkoxy-carbonyl groups, linear (C₂ - C₆) alkenyl groups, linear (C₂ - C₆) halogenated alkenyl groups, (C₂ - C₆) alkynyl groups, (C₂ - C₆) halogenated alkynyl groups, and (C₃ - C₈) cycloalkyl groups which, however, may be substituted by no more than five identical or different substituting groups selected from halogens and (C₁ - C₄) alkyl groups, (C₆ - C₁₂) aryl groups, (C₆ - C₁₂) aryl-(C₁ - C₄) alkyl groups, (C₆ - C₁₂) aryloxy-carbonyl groups, heteroaryl groups having no more than 10 carbon atoms, heteroaryl-carbonyl groups, and heteroaryl-(C₁ - C₄) alkyl groups.
R⁸ may be identical or different, and it represents a hydrogen, halogens, (C₁ - C₆) alkyl groups, (C₁ - C₆) halogenated alkyl groups, (C₁ - C₆) alkoxy groups, (C₁ - C₆) alkoxy-(C₁ - C₆) alkyl groups, (C₁ - C₆) alkoxy-(C₁ - C₆) alkoxy groups, (C₁ - C₆) halogenated alkoxy groups, linear (C₂ - C₆) alkenyl groups, linear (C₂ - C₆) alkenyloxy groups, (C₂ - C₆) alkynyl groups, (C₂ - C₆) alkynoxy groups, (C₂ - C₆) halogenated alkynyl groups, (C₂ - C₆) halogenated alkynoxy groups, (C₆ - C₁₂) aryloxy groups, (C₆ - C₁₂) aryl-(C₁ - C₄) alkyl groups, (C₆ - C₁₂) arylthio groups, heteroaryloxy groups having no more than 10 carbon atoms, heteroaryl-thio groups, and heteroaryl-(C₁ - C₄) alkyl groups. And, the aryl group or the hetero-aryl group in the R⁸ may be selected from (C₁ - C₆) alkyl groups, (C₁ - C₆) alkoxy groups, (C₁ - C₆) halogenated alkyl groups, (C₁ - C₆) halogenated alkoxy groups, and halogens, which may be identical or different.

Preferred R⁸ is benzyl group, phenoxy group, acryl group, propargyl group, methyl group and trifluoromethyl group. 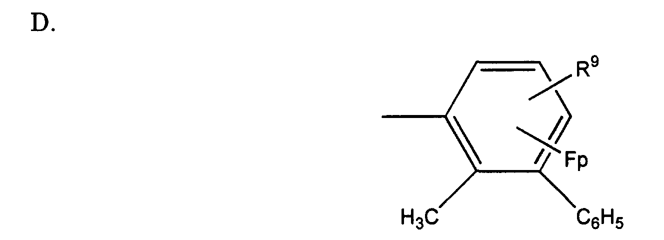
F represents fluorine,
R⁹ is a hydrogen, fluorine or a methyl group,
p is 0, 1, or 2.

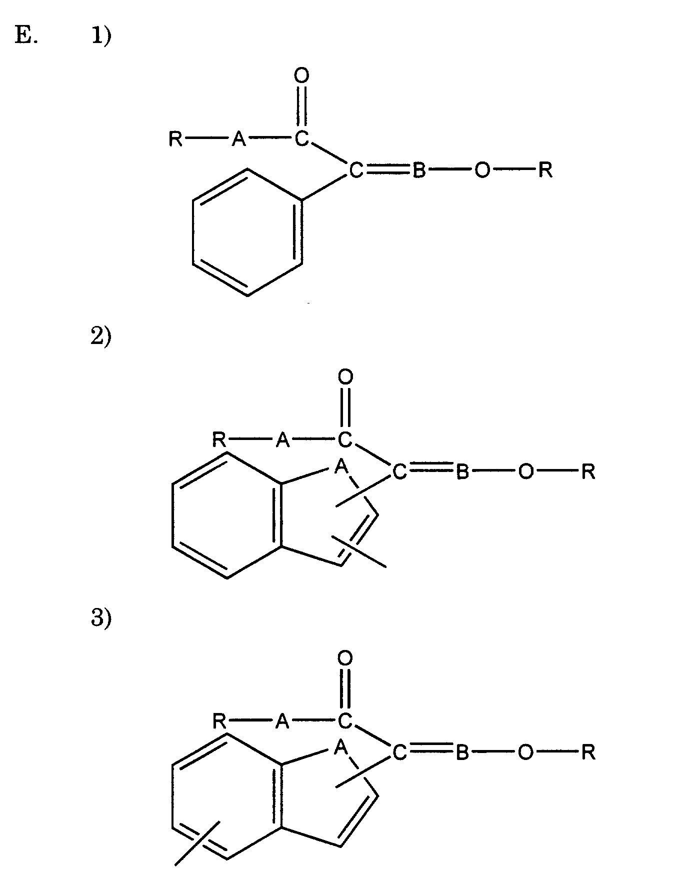

R is a (C₁ - C₆) alkyl group or a (C₁ - C₆) halogenated alkyl group, preferably a methyl group,
A is an oxygen, sulfur or NH.
B is a nitrogen or CH.

In the general formula (I), (II) and (III), preferred Ar2 is the following: 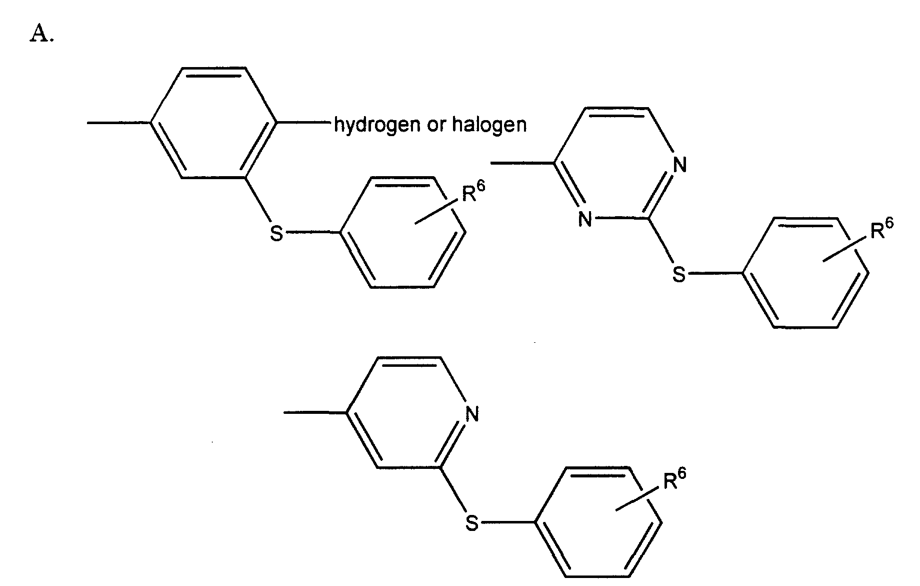
R⁶ is a hydrogen, 3-fluoro, 4- fluoro, or 4-chloro group.
s is O, S, NH or CH₂, preferably O or CH₂.

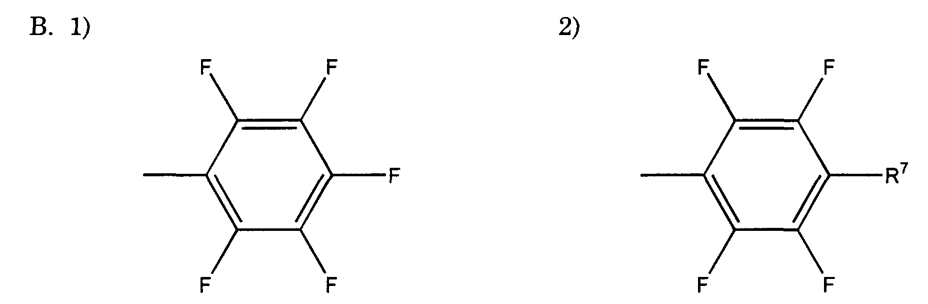

R⁷ is (C₁- C₆) alkyl groups, preferably CH₃, (C₁ - C₆) halogenated alkyl groups, (C₁ - C₆) alkoxy groups, and (C₁ - C₆) halogenated alkoxy groups.

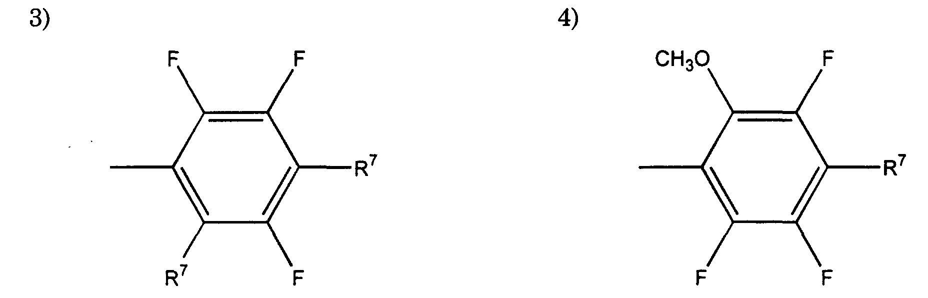

R⁷ is H, CH₃, or OCH₃.

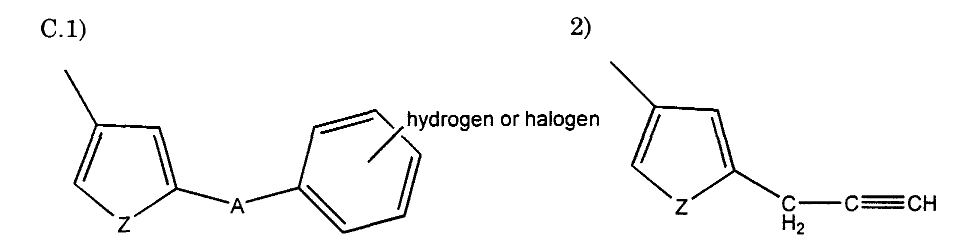

A is CH₂ or O, and Z is O or S.

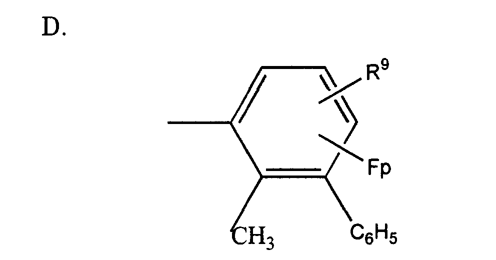

F is fluorine.

R⁹ is a hydrogen, fluorine or a methyl group.

p is 0, 1, or 2.

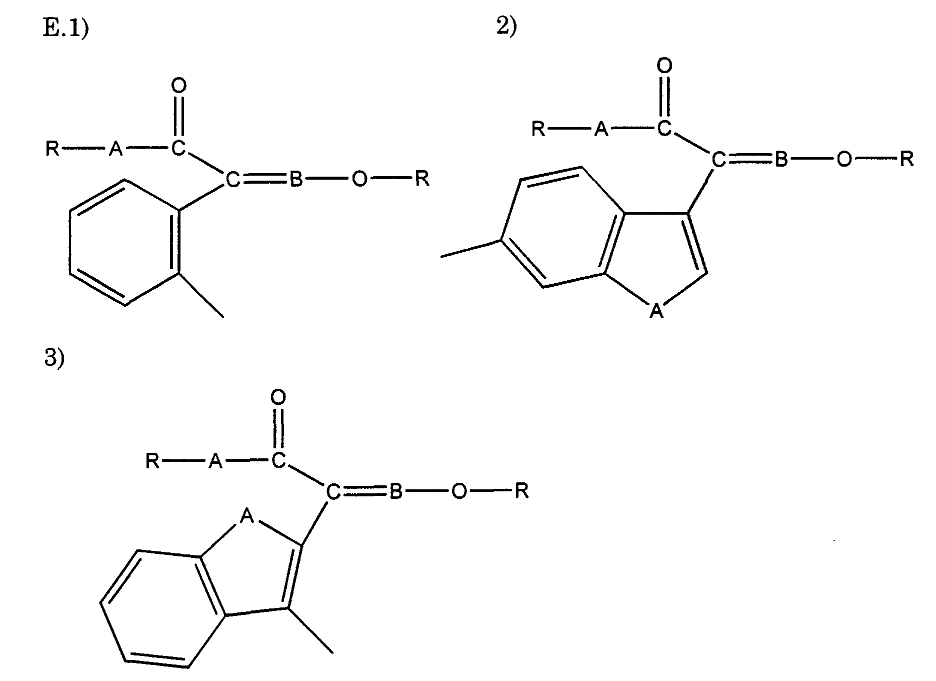

R is a methyl group.

A is an oxygen, sulfur or NH.

B is a nitrogen or CH.

Particularly preferred examples of the compounds for the formula (I), (II) and (III) are as follows: Ar1 is a substituted or unsubstituted aryl or heteroaryl group and Ar2 is the particularly preferred Ar2 described above. R₁ and R₂ are hydrogen, methyl group or ethyl group. R₃ is a methyl group. m is 0, n is 1, and X is S, O, or SO₂.

The compounds represented by the formula (I) and (III) may exist in two types of geometric isomers : "cis" form (formula Z) and "trans" form (formula E). With "cis" form (formula Z), the oxime-oxygen and the aryl group or hetero-aryl group Ar1 are located on the same side of C=N double bond. With the "trans" form (formula E), the oxime-oxygen and the aryl group or heteroaryl group Ar1 are located on different side of the C=N double bond. Thus, it does not change its geometric structure when it is transformed. Thus, with the compound of the formula (III), "cis" type will yield a corresponding "cis" type (I) oxime ether. With the "trans" type (formula E) (III), it will yield a corresponding "trans" type (I) (formula E) oxime ether (formula E).

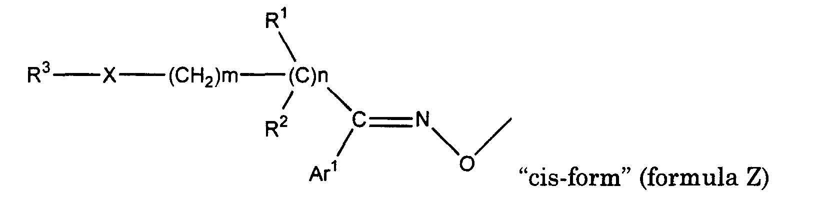

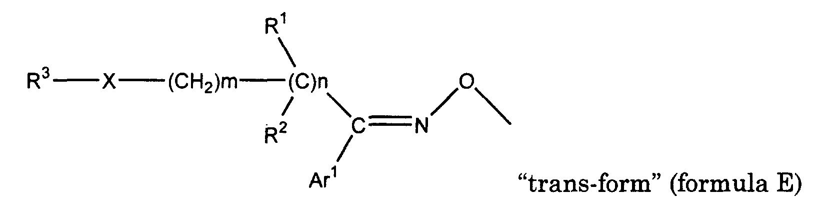

Beside, if the alkyl-substituted-(hetero)aryl-ketoxime-o-ether described in the general formula (I), intermediate ketone and oxime compound described in the general formula (II) and (III) contain one or more asymmetric carbon atom(s), it may be a racemate, a diastereoisomer or a pure optical isomer.

The alkyl-substituted-(hetero)aryl-ketoxime-o-ether described in the general formula (I) and the intermediate oxime described in the general formula (III) refer, not only to a cis-form (formula Z), but also to a trans-form (formula E) as well as a mixture of both.

The alkyl-substituted-(hetero)aryl-ketoxime-o-ether described in the general formula (I), the intermediate ketone, and oxime compound described in the general formula (II) and (III) refer, not only to a racemate and diastereoisomer, but also to a pure optical isomer.

The alkyl-substituted-(hetero)aryl-ketoxime-o-ether described in the general formula (I), the intermediate ketone, and oxime compound described in the general formula (II) and (III) refer, not only to a pure isomer, but also to the mixtures of various isomers.

The alkyl-substituted-(hetero)aryl-ketoxime-o-ethers of this invention described in the formula (I), the intermediate ketone, and oxime compound described in the general formula (II) and (III) are indicated in Table 1 where the group Ar2 is indicated simply as G^{k} (k = 1-35). However, compounds of the formula (I), (II) and (III) are not limited to those indicated in Table 1.

Production methods of the alkyl-substituted-(hetero)aryl-ketoxime-o-ethers of this invention represented by formula (I), the intermediate ketones, and oxime compounds represented by general formula (II) and (III) are described in the following:
a) Using the compound illustrated by the formula (III) and the compound illustrated by the formula (IV).

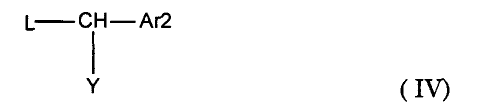

In the formula (IV), L represents a releasable group. Thus, if M in the formula (III) is a hydrogen, it can be obtained, in the presence of an appropriate base such as alkaline metal hydroxide, alkaline metal carbonate, alkaline metal bicarbonate, alkaline metal alcohol, alkaline metal, alkaline metal hydride, pyridine, or tertiary amine, by adding an appropriate solvent such as the one selected from one or two of water, tetrahydrofuran, toluene, benzene, acetonitrile and/or dimethylsulfoxide, and so on, and adding a phase transfer catalyst PTC such as tetrabutyl ammonium iodide, tetrabutyl ammonium bromide, tetraethyl ammonium bromide, or 18-crown ether-6,, and running the reaction under atmospheric pressure at 0 - 120°C for 1 - 20 hours.

If M in the formula (III) is an alkaline metal atom such as sodium, alkylation of the oxime salt is carried out under the same condition, and presence of an alkali is not required. Or.
the compound indicated by the formula (II) is reacted with the compound indicated by the formula (V) or its salt, in the presence of an appropriate base, if necessary.

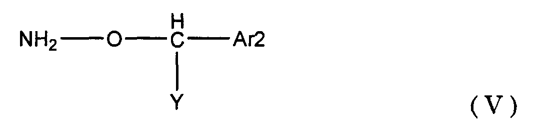

The compound (III) of this invention can be prepared by the following method. It is synthesized by reacting a compound represented by the formula (II) with hydroxylamine hydrochloride salt or hydroxylamine sulfate salt in an appropriate solvent, preferably water and/or alcohol, under atmospheric pressure at 0 - 100°C. If necessary, an appropriate base such as an alkaline metal hydroxide, alkaline metal carbonate, alkaline metal bicarbonate, or pyridine may be added to run the synthesis.

The compound (II) of this invention indicated by the formula (II) is prepared as follows. Compound of the formula (II) having different structures is synthesized by using appropriate starting materials and corresponding synthetic procedure. If X in the compound indicted in the general formula (II) is S, one can use the compound indicated by the formula (VI).

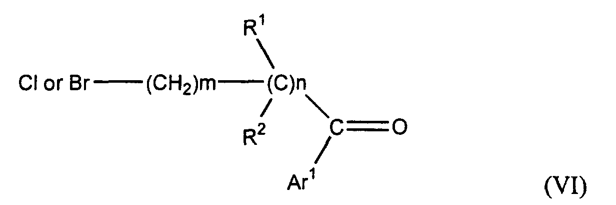

In the above formula, Ar1, R₁, R₂, n, and m are the same as defined in Claim 1, and this compound is obtained by reacting with the sodium salt of corresponding sulfate alcohol in an aqueous solvent under atmospheric pressure at 30 - 100°C for 2 - 10 hours.

The alkyl-substituted-(hetero)-aryl-ketoxime-o-ethers, the intermediate ketones, and oxime compounds proposed in this invention have biological activity, and some compounds have particularly excellent biological activity. They show outstanding activity in pest control field, particularly in agriculture, horticulture, flower cultivation, and hygienic field. They do not harm the crops, and show quick and persistent insecticidal effect in a satisfactory manner. In this place, the pests includes, but not limited, to the following.

Insect pests : Orthoptera such as roaches, thrips such as cotton thrips, rice plant thrips, and cucumber thrips; Homoptera such as leafhopper, planthopper, and aphid; Lepidoptera such as eastern army worm, cotton leaf worm, moth, and cabbage army worm; Hymenoptera such as larva of sawfly; Diptera such as mosquitoes and fly; mites, and so on.

Fungal pathogens : Powdery mildew, Downy mildew, Sclerotium, Venturia, Giberella, Fusarium, and so on.

Weeds : Barnyard grass, shortawn foxtail, wild sorghum; broadleaf weed such as fat hen (Chenopodium) and bittercress.

By using the alkyl-substituted-(hetero)aryl-ketoxime-o-ethers, the intermediate ketones, or the oxime compounds of this invention as the effective component of the controlling agent in agriculture, one can prepare any desired type of agent such as emulsion oil, wettable powder, suspending agent, granules, and so on. Appropriate aids include carrier (diluting agent) and other aids such as extender, emulsifier, wetting agents, dispersing agents, adhesive agents, and degrading agents.

### Examples for preparing controlling agents for agriculture :

Preparation of emulsion oil: Twenty parts (by weight) of the alkyl-substituted-(hetero)aryl-ketoxime-o-ether, intermediate ketone, or oxime compound of this invention, 73 parts of diluting agent such as toluene, and 7 parts of an appropriate aid were mixed homogeneously, to prepare the emulsion oil which was be diluted with water for application.

Preparation of wettable powder : Twenty parts (by weight) of the alkyl-substituted-(hetero)aryl-ketoxime-o-ether, intermediate ketone, or oxime compound, 53 parts of clay, 20 parts of bleached carbon black, 5 parts of lignin silicate, and 2 parts of polyoxy methylalkyl ether were mixed, ground, and pulverized to prepare a wettable powder.

Examples are provided in the following to further explain this invention.

### Example 1 Synthesis of 1-(4-chlorophenyl)-2-methylthio-O-((3-phenoxyphenyl) methyl) 1-propanone oxime - the compound No. 30 in the Table 1

1-(4-Chlorophenyl)-2-methylthio) 1-propanone oxime (a mixture of Z and E isomers, content = 90%) 1.7 g (0.007 mol) was weighed out and dissolved in 18 ml of water-free acetonitrile containing 0.20 g of metallic sodium. After reacting at 30 - 40°C for 2 - 3 hours, tetrabutyl ammonium iodide 0.3 g was added. Then, m-phenoxy benzyl chloride (content = 90%) 1.91 g (0.008 mol) was added dropwise. After the addition, temperature was raised slowly to 60 - 65°C and they were reacted for 8 - 9 hours. After the treatment, a crude oily product 2.85 g (a mixture of Z and E isomers) was obtained. It was purified with petroleum ether-methyl acetate, by reduced pressure column chromatography. Thus, a pure Z and E isomers were obtained. The product was a faintly yellow, viscous liquid. Total weight of the pure Z and E isomers was 1.49 g. Purity : Z isomer = 92%, E isomer = 93% (gas chromatography). Yield was 47%.
NMR (δ^{CDCl3} TMS) :
E isomer : 1.26 (d, 3H, CH₃), 2.05 (s, 3H, SCH₃), 4.76 (q, 1H, -CH-S-), 5.15 (s, 2H, C=N-O-CH₂-), 6.96 - 7.66 (m, 13H, proton on the benzene ring).
Z isomer : 1.27 (d, 3H, CH₃), 1.93 (s, 3H, SCH₃), 3.60 (q, 1H, -CH-S-), 5.02 (s, 2H, C=M-O-CH₂-), 6.88 - 7.38 (m, 13H, proton on the benzene ring).
Result of element analysis : (Found/calculated): C % :67.41/67.07 H % :5.06/5.35 N % : 3.32/3.40 S % 7.25/7.77

### Example 2 Synthesis 1-(4-chlorophenyl)-2-methylthio-O-((3-phenoxyphenyl) methyl ethanone oxime - the compound (1) No. 5 in Table 1

1-(4-Chlorophenyl)-2-methylthio ethanone oxime (E isomer, content = 90%) 1.7 g (0.007 mol), m-phenoxy benzyl chloride (content = 90%) 1.76 g (0.007 mol), tetrabutyl ammonium iodide 0.3 g, and toluene 4 ml were weighed and added in a reaction vessel. With constant agitation, an aqueous solution 4 ml (containing NaOH 0.63 g) was added batchwise. Temperature was raised slowly to 58 - 60°C and reaction was carried out for 7 - 8 hours. After the treatment, a crude oily product 2.67 g (E isomer), was obtained. It was purified with petroleum ether-ethyl acetate by reduced pressure column chromatography, to obtain a faintly yellow viscous liquid 1.39 g (E isomer). Purity was 94% (gas chromatography), and yield was 44%.
NMR δ^{CDCl3} TMS : 1.98 (s, 3H, SCH₃), 3.72 (s, 2H, -CH₂-S), 5.19 (s, 2H, C=N-O-CH₂-), 6.89 - 7.66 (m, 13H, proton on the benzene ring).
Result of element analysis (Found/calculated) : C % : 66.41/66.08 H% : 5.06/4.88 N% : 3.52/3.50 S% : 8.05/8.54
Mass spectrum : Ion peak M⁺ (397)

### Example 3 Synthesis of 1-(4-chlorophenyl)-2-methylthio ethanone oxime) - compound (III), No. 5 in Table 1

1-(4-chlorophenyl)-2-methylthio ethanone 27.0 g (0.13 mol), ethanol 35 ml, water 14 ml, and hydroxylamine hydrochloride 12.80 g (0.18 mol) were added in a 250 ml three-necked flask equipped with a reflux condenser, a thermometer, and magnetic stirrer. While being agitated, a solid NaOH 16.0 g (0.4 mol) was added batchwise. After the addition, it was kept at room temperature (about 25°C) to run the reaction for 15 - 20 minutes. Then, the temperature was raised slowly to 45°C to run the reaction for 45 minutes. Then, it was heated to a refluxing temperature to run the reaction for 5 - 6 hours. After cooling, it was treated, to obtain a grayish white needle-like crystal 29 g (a mixture of Z and E isomers). Purity was 90% (gas chromatography), yield was 90%, and melting point was 93.6 - 96.6°C.
Result of element analysis (Found/calculated) : C % : 50.42/50.11H % : 4.83/4.64 N % : 6.47/6.49 S % : 13.82/14.80
Mass spectrum : Ion peak M⁺ (215)

### Example 4 Synthesis of 1-(4-chlorophenyl)-2-methylthio ethanone (or 4'-chloro-2-methylthio acetophenone - compound (II), No. 5 in Table 1

Aqueous sodium methyl mercaptan solution (content = 37%) 41.0 g (0.217 mol) was added in a 250 ml three-necked flasks equipped with a reflux condenser, a thermometer, and magnetic stirrer. At room temperature, 1-(4-chlorophenyl)-2-chloroacetophenone (content = 95%) 24.75 g (0.124 mol) was added batchwise. Then, reaction was carried out under atmospheric pressure at 60 - 80°C for 3 - 5 hours. The reaction mixture was cooled, and then treated, to obtain an oily liquid product 27 g. Purity was 90% (gas chromatography) and yield was 97%.
Result of element analysis (Found/calculated): C% : 53.80/53.86 H% 4.30/4.52 S% : 15.28/15.97
Mass spectrum : Ion peak M⁺ (200)

### Example 5 Synthesis of 1-(4-chlorophenyl)-2-chloro ethanone (or 4'-chloro-2-chloro acetophenone

Chlorobenzene 135 g (1.2 mol) and anhydrous aluminum trichloride 88.8 g (0.667) were added in a 500 ml three-necked flask equipped with a dropping funnel, magnetic stirrer, a thermometer, and a reflux condenser packed with anhydrous calcium chloride desiccant tube (the desiccant tube and alkali absorbing liquid were connected). After adding chloroacetyl chloride 69.5 g (0.615 mol) dropwise and slowly from an automatic dropping funnel at 5 - 15°C, temperature was raised slowly to about 70°C to release the tail gas, and then the reaction mixture was cooled and treated to obtain a white needle-like crystal 118 g. Purity was 90% (gas chromatography), yield was 91%, and melting point was 97.9 - 99.0°C (m.p. reported in literature = 98 - 100°C).
Result of element analysis (Found/calculated) : C % : 50.38/50.82 H% : 3.02/3.19
Mass spectrum : Ion peak M⁺ (188).

Every compounds of the formula (I), (II) and (III) can be synthesized by the similar method. Every synthesized compounds were identified and verified by NMR analysis, infra-red analysis, mass spectral analysis and/or element analysis.

Example of determination of biological activity : (Z isomer and E isomer showed different biological activities. Generally, the biological activity of E isomer is higher than the Z isomer, and the activity data is based on the activity data of the E isomer or that of the mixture of Z and E isomers

### Example 6 Test for the biological activity against Mythimne separata

Emulsion oil or wettable powder of the alkyl-substituted-(hetero)aryl-ketoxime-o-ether, intermediate ketone, or the active oxime compound of this invention prepared by the procedure described in the Examples for preparing a controlling agent in agriculture described above was diluted with water to prepare a solution of agricultural chemical having a certain concentration.

Ten heads of Mythimne separata and 5 pieces of corn fragments were placed in a Petri dish, and it was sprayed with a certain amount of agricultural chemical solution. After the exposure, it was kept in a room for normal cultivation. After 24 hours, number of live and dead insects were counted. Test was run 3 times, and results were averaged. It was found that a series of compound (I) [Code No. 5, 6, 7, 8, 9, 10, 11, 12, 21, 22, 23, 30, 31, 32, 33, 34, 35, 36, 37, 46, 47, 48, 213, 216, 229, 230, 231, 238, 254, 629, 632, 645, 646, 647, 654, 657] gave a lethal rate exceeding 100% when its concentration was 500 ppm (calculated as the content of effective component). Some compounds (I) [such as Code No. 5, 8, and 30] shown in Table 1] gave 100% lethal rate even when the concentration was lower than 500 ppm.

### Example 7 Test for the biological activity against aphids Aphis fabae

Emulsion oil or wettable powder of the alkyl-substituted-(heteroaryl-ketoxime-o-ether, ketone intermediate or active oxime compound of this invention prepared by the method described in the Example for preparing a controlling agent in agriculture was diluted with water to prepare a solution of agricultural chemical having a certain concentration. Aphids were inoculated in the new seedlings (more than 15). Then, the seedlings, together with the aphids, were dipped in the solution of agricultural agent for 5 seconds, removed, and excess liquid was drained. They were placed in a water-absorbing sponge, and held by glass rods. After 24 hours, the number of live and dead aphids was examined. Test was run 3 times, and the results were averaged. With a series of compound (I) [Code No. 5, 8, 21, 22, 23, 30, 33, 46, 47, 48], the aphids were killed 100% when the concentration was 250 ppm (calculated as the content of effective component). Some compounds (I) [Code No. 5, 8, and 30 in Table 1] gave 100% lethal rate with the aphids, even at a lower concentration.

### Example 8 Test for the biological activity against leafhopper Nephotetlix cimeticeps

Emulsion oil or wettable powder of the alkyl-substituted-(hetero)aryl-ketoxime-o-ether, ketone intermediate, or active oxime compound of this invention prepared by the method described in the Example for preparing a controlling agent in agriculture was diluted with water to prepare a solution of agricultural chemical having a certain concentration. Rice seedlings (second semester) were dipped in the solution of agricultural agent for 5 seconds, and then removed. It was drained and then placed in a large test tube. Each test tube contained 20 seedlings. Then, 20 leafhoppers (5^{th} instar) were introduced in the tube, and the mouth of the tube was covered with a white cheesecloth. The tube was kept at room temperature, and number of live and dead leafhoppers was examined after 24 hours. Test was run 3 times, and results were averaged. It was found that a series of compounds (I) [Code No. 5, 6, 7, 8, 9, 10, 11, 12, 21, 22, 23, 30, 31, 32, 33, 34, 36, 37, 46, 47, 48, 188 in Table 1] killed 100% of the leafhoppers when the concentration was 250 ppm (calculated as the content of effective component). Some compounds (I) [Code No. 5, 8, 30, 33, 188 in Table 1] showed 100% kill rate even at a lower concentration.

All synthesized compounds showed excellent biological activity against other insect pests also.

| Biological activity of the compound (I) [Code No. 5 in Table 1] : | | |
|---|---|---|
| Spinach army worm | E-form | LC₅₀ (ppm) < 5 |
| Spotted cutworm | E-form | LC₅₀ (ppm) < 5 |
| American tobacco cutworm | E-form | LC₅₀ (ppm) < 10 |
| Green peach aphid | E-form | LC₅₀ (ppm) > 100 |

| Biological activity of the compound (I) [Code No. 30 in Table 1] | | |
|---|---|---|
| Spinach army worm | E-form | LC₅₀ (ppm) < 5 |
| | Z-form | LC₅₀ (ppm) > 150 |
| Spotted cutworm | E-form | LC₅₀ (ppm) < 1.5 |
| | Z-form | LC₅₀ (ppm) > 150 |
| American tobacco cutworm | E-form | LC₅₀ (ppm) < 5 |
| | Z-form | LC₅₀ (ppm) > 150 |
| Green peach aphid | E-form | LC₅₀ (ppm) < 15 |
| | Z-form | LC₅₀ (ppm) > 150 |

### Example 9 Test for antifungal activity

Alkyl-substituted-(hetero)aryl-ketoxime-o-ether, ketone intermediate, or active oxime compound of this invention was diluted with an appropriate diluting agent (such as propanone) to prepare a parent solution having a certain concentration. One ml of this solution was taken with a pipette into 50 ml of molten PDA media. After shaking thoroughly, it was poured into two Petri dishes, to create duplicate. After cooling, a mycelial pad (diameter = about 4 mm) was taken and inoculated on the center of the culture dish. The diluting agent was used as the control. After inoculation, the dish was kept in an incubator set at an appropriate temperature. After 72 hours of cultivation, length (diameter of the colony) of the growth of mycelia was measured, and fungal growth inhibition rate was calculated. It was found that a series of compounds (III) [Code No. 5 and 7] showed higher than 90% growth inhibition rate against one or more fungal pathogens when the concentration was 100 ppm (calculated as the content of effective component).

### Example 10 Test for herbicidal activity

Emulsion oil or wettable powder of the alkyl-substituted-(hetero)aryl-ketoxime-o-ether, ketone intermediate or active oxime compound prepared by the method described in the Example for preparing a controlling agent in agriculture described above was diluted with water, to prepare a parent solution having a prescribed concentration. Seedlings of weed (such as Echinochloa crusgalli, etc., height = about 0.3 cm) were placed on a filter paper in a 9 cm Petri dish. Then, the seedlings were covered with a piece of filter paper. A certain amount of the solution of agricultural agent was added with a Pipette into the dish. The dish was covered and placed in an illuminated incubator kept at an appropriate temperature. After 3 - 4 days, root length, height of the seedlings and its fresh weight were measured, and % inhibition was calculated. It was found that certain compounds showed growth inhibitory activity or growth enhancing activity against one or more monocotyledonic and dicotyledonic weeds when the concentration (calculated as the content of effective component) was 100 ppm.

The AR2 group in the formula (I) is simply indicated as G^{k} (K = 1-35). Thus, in Table 1, general formula (VII) can be used to express the compound having the general formula (I).

Partial formula (VII) is equivalent to the formula (II), and they, together with the compounds having the formula (II) and formula (III) are illustrated in Table 1. However, the compounds of the formula (I), formula (II) and formula (II) are not necessarily limited to the compounds illustrated in Table 1.

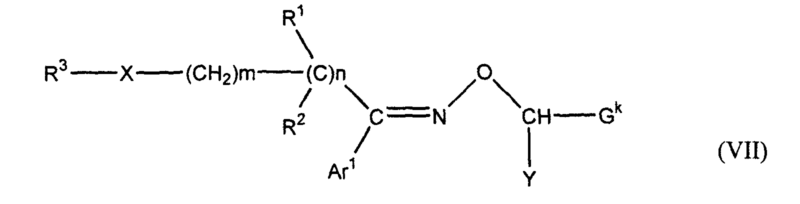

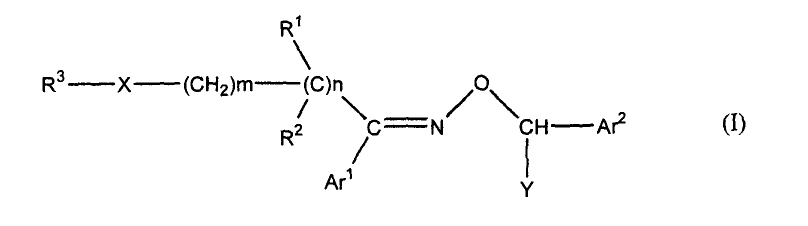

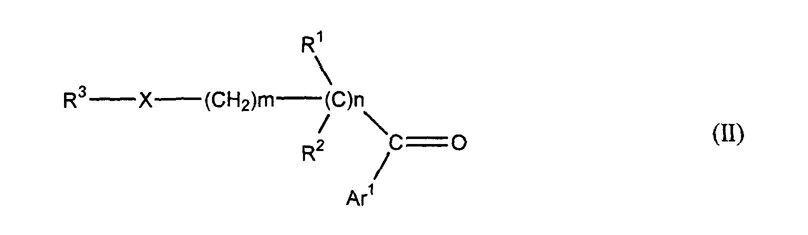

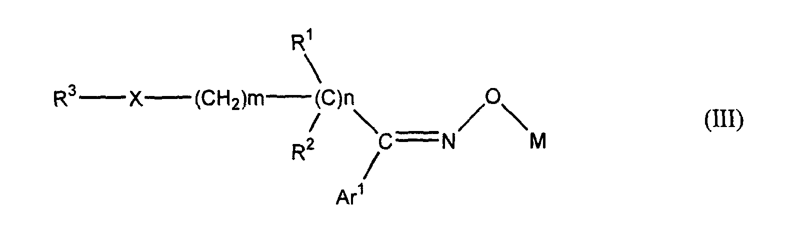

**Table 1**

| No. | R | R₁ | R₂ | R₃ | Ar¹ | X | m | n | G^{K} | Y |
|---|---|---|---|---|---|---|---|---|---|---|
| 1 | 4-Cl | H | Ethyl | CH₃ | phenyl | S | 0 | G¹ | G¹ | H |
| 2 | 4- OCH₂CH₃ | : | : | : | : | : | : | : | G¹ | : |
| 3 | 4- CH₃ | : | : | : | : | : | : | : | G¹ | : |
| 4 | 3,4-Cl₂ | : | : | : | : | : | | | G¹ | : |
| 5 | 4-Cl | : | H | : | : | : | : | : | G¹ | : |
| 6 | 4-F | : | : | : | : | : | : | : | G¹ | : |
| 7 | 4-Br | : | : | : | : | : | : | : | G¹ | : |
| 8 | 4- CH₃ | : | : | : | : | : | : | : | G¹ | : |
| 9 | 4-OCH₃ | : | : | : | : | : | : | : | G¹ | : |
| 10 | 4- CH₂CH₃ | : | : | : | : | : | : | : | G¹ | : |
| 11 | 4- OCH₂CH₃ | : | : | : | : | : | : | : | G¹ | : |
| 12 | 4- CH(CH₃)₂ | : | : | : | : | : | : | : | G¹ | : |
| 13 | 4- SCH₃ | : | : | : | : | : | : | : | G¹ | : |
| 14 | 4- C(CH₃)₃ | : | : | : | : | : | : | : | G¹ | : |
| 15 | 4- CH₂CH₂CH₃ | : | : | : | : | : | : | : | G¹ | : |
| 16 | 4- OCH(CH₃)₂ | : | : | : | : | : | : | : | G¹ | : |
| 17 | 4- OCH₂CH₂CH₃ | : | : | : | : | : | : | : | G¹ | : |
| 18 | 4- SOCH₃ | : | : | : | : | : | : | : | G¹ | : |
| 19 | 3,4-O-CH₂-O | : | : | : | : | : | : | : | G¹ | : |
| 20 | 4-Phenoxy | : | : | : | : | : | : | : | G¹ | : |
| 21 | 3,4-Cl₂ | : | : | : | : | : | : | : | G¹ | : |
| 22 | 3,4-(CH₃)₂ | : | : | : | : | : | : | : | G¹ | : |
| 23 | 3-Cl,4-CH₃ | : | : | : | : | : | : | : | G¹ | : |
| 24 | 4- CF₃ | : | : | : | : | : | : | : | G¹ | : |
| 25 | 4-OCF₃ | : | : | : | : | : | : | : | G¹ | : |
| 26 | 4-CF₂H | : | : | : | : | : | : | : | G¹ | : |
| 27 | 4-OCH₂CF₃ | : | : | : | : | : | : | : | G¹ | : |
| 28 | 3-Br,4-F | : | : | : | : | : | : | : | G¹ | : |
| 29 | 4-CH₂OCH₃ | : | : | : | : | : | : | : | G¹ | : |
| 30 | 4-Cl | H | CH₃ | CH₃ | phenyl | S | 0 | 1 | G¹ | H |
| 31 | 4-F | : | : | : | : | : | : | : | G¹ | : |
| 32 | 4-Br | : | : | : | : | : | : | : | G¹ | : |
| 33 | 4- CH₃ | : | : | : | : | : | : | : | G¹ | : |
| 34 | 4- OCH₃ | : | : | : | : | : | : | : | G¹ | : |
| 35 | 4- CH₂CH₃ | : | : | : | : | : | : | : | G¹ | : |
| 36 | 4- OCH₂CH₃ | : | : | : | : | : | : | : | G¹ | : |
| 37 | 4- CH(CH₃)₂ | : | : | : | : | : | : | : | G¹ | : |
| 38 | 4- SCH₃ | : | : | : | : | : | : | : | G¹ | : |
| 39 | 4- C(CH₃)₃ | : | : | : | : | : | : | : | G¹ | : |
| 40 | 4- CH₂CH₂CH₃ | : | : | : | : | : | : | : | G¹ | : |
| 41 | 4- OCH(CH₃)₂ | : | : | : | : | : | : | : | G¹ | : |
| 42 | 4- OCH₂CH₂CH₃ | : | : | : | : | : | : | : | G¹ | : |
| 43 | 4- SOCH₃ | : | : | : | : | : | : | : | G¹ | : |
| 44 | 3,4-OCH₂-O | : | : | : | : | : | : | : | G¹ | : |
| 45 | 4-Phenoxy | : | : | : | : | : | : | : | G¹ | : |
| 46 | 3,4-Cl₂ | : | : | : | : | : | : | : | G¹ | : |
| 47 | 3,4-(CH₃)₂ | : | : | : | : | ; | : | : | G¹ | : |
| 48 | 3-Cl,4-CH₃ | : | : | : | : | ; | : | : | G¹ | : |
| 49 | 4- CF₃ | : | : | : | : | : | : | : | G¹ | : |
| 50 | 4-OCF₃ | : | : | : | : | : | : | : | G¹ | : |
| 51 | 4-CF₂H | : | : | : | : | : | : | : | G¹ | : |
| 52 | 4-OCH₂CF₃ | : | : | : | : | : | : | : | G¹ | : |
| 53 | 3-Br,4-F | : | : | : | : | : | : | : | G¹ | : |
| 54 | 4-CH₂OCH₃ | : | : | : | : | : | : | : | | : |
| 55 | 4-OCF₂CF₂H | : | H | : | : | : | : | : | G¹ G¹ | : |
| 56 | 4-OCF₂H | : | : | : | : | : | : | : | G¹ | : |
| 57 | 4-OCH₂CCl=CH₂ | : | : | : | : | : | : | : | G¹ | : |
| 58 | 4-Ethoxy-ethoxy | : | : | : | : | : | : | : | G¹ | : |
| 59 | 4-OCH₂CH₂Cl | : | : | : | : | : | : | : | G¹ | : |
| 60 | 3,4,5-Trifluoro | H | H | CH₃ | phenyl | S | 0 | 1 | G¹ | H |
| 61 | 4-Acryloxymethyl | : | : | : | : | : | : | : | G¹ | : |
| 62 | 4-CH₂Cl | : | : | : | : | : | : | : | G¹ | : |
| 63 | 4-NO₂ | : | : | : | : | : | : | : | G¹ | : |
| 64 | 3-NO₂ | : | : | : | : | : | : | : | G¹ | : |
| 65 | 4-OCF₂CF₂H | : | CH₃ | : | ; | : | : | : | G¹ | : |
| 66 | 4-OCF₂H | : | : | : | : | : | : | : | G¹ | : |
| 67 | 4-OCH₂CCl=CH₂ | : | : | : | : | : | : | : | G¹ | : |
| 68 | 4-Ethoxy-ethoxy | : | : | : | : | : | : | : | G¹ | : |
| 69 | 4-OCH₂CH₂Cl | : | : | : | : | : | : | : | G¹ | : |
| 70 | 3,4,5-Trifluoro | : | : | : | : | : | : | : | G¹ | : |
| 71 | 4-Acryloxymethyl | : | : | : | : | : | : | : | G¹ | : |
| 72 | 4- CH₂Cl | : | : | : | : | : | : | : | G¹ | : |
| 73 | 4-Cl | : | H | : | : | : | : | 3 | G¹ | : |
| 74 | 4-CH₃ | : | : | : | : | : | : | 3 | G¹ | : |
| 75 | 2-Cl | : | : | : | : | : | : | 1 | G¹ | : |
| 76 | 3-Cl | : | : | : | : | : | : | : | G¹ | : |
| 77 | 3-CF₃ | : | : | : | : | : | : | : | G¹ | : |
| 78 | 3-Phenoxy | : | : | : | : | : | : | : | G¹ | : |
| 79 | 2-CH₃ | : | : | : | : | : | : | : | G¹ | : |
| 80 | 2-Cl | : | CH₃ | : | : | : | : | : | G¹ | : |
| 81 | 3-Cl | : | : | : | : | : | : | : | G¹ | : |
| 82 | 3-CF₃ | : | : | : | : | : | : | : | G¹ | : |
| 83 | 3-Phenoxy | : | : | : | : | : | : | : | G¹ | : |
| 84 | 2-CH₃ | : | : | : | : | : | : | : | G¹ | : |
| 85 | 4-Cl | CH₃ | : | : | : | : | : | : | G¹ | : |
| 86 | 4-F | : | : | : | : | : | : | : | G¹ | : |
| 87 | 4-Br | : | : | : | : | : | : | : | G¹ | : |
| 88 | 4- CH₃ | : | ; | : | : | : | : | : | G¹ | : |

| No. | R | R₁ | R₂ | R₃ | Ar¹ | X | m | n | G^{K} | : |
|---|---|---|---|---|---|---|---|---|---|---|
| 89 | 4- OCH₃ | CH₃ | CH₃ | CH₃ | Phenyl | S | 0 | 1 | G¹ | H |
| 90 | 4- CH₂CH₃ | : | : | : | : | : | : | : | G¹ | : |
| 91 | 4- OCH₂CH₃ | : | : | : | : | : | : | : | G¹ | : |
| 92 | 4-Cl | H | SCH₃ | : | : | : | : | : | G¹ | : |
| 93 | 4-F | : | : | : | : | : | : | : | G¹ | : |
| 94 | 4-Br | : | : | : | : | : | : | : | G¹ | : |
| 95 | 4- CH₃ | : | : | : | : | : | : | : | G¹ | : |
| 96 | 4- OCH₃ | : | : | : | : | : | : | : | G¹ | : |
| 97 | 4- CH₂CH₃ | : | : | : | : | : | : | : | G¹ | : |
| 98 | 4- OCH₂CH₃ | : | : | : | : | : | : | : | G¹ | : |
| 99 | 4-Cl | : | H | Ethyl | : | : | : | : | G¹ | : |
| 100 | 4-F | : | : | : | : | : | : | : | G¹ | : |
| 101 | 4-Br | : | : | : | : | : | : | : | G¹ | : |
| 102 | 4- CH₃ | : | : | : | : | : | : | : | G¹ | : |
| 103 | 4- OCH₂CH₃ | : | : | : | : | : | : | : | G¹ | : |
| 104 | 4-Cl | : | : | Isopropyl | : | : | : | : | G¹ | : |
| 105 | 4-F | : | : | : | : | : | : | : | G¹ | : |
| 106 | 4- OCH₃ | : | : | : | : | : | : | : | G¹ | : |
| 107 | 4- CH₃ | : | : | : | : | : | : | : | G¹ | : |
| 108 | 4- OCH₂CH₃ | : | : | : | : | : | : | : | G¹ | : |
| 109 | 4-Cl | : | : | *n*-Propyl | : | : | : | : | G¹ | : |
| 110 | 4-F | : | : | : | : | : | : | : | G¹ | : |
| 111 | 4- OCH₃ | : | : | : | : | : | : | : | G¹ | : |
| 112 | 4- OCH₂CH₃ | : | : | : | : | : | : | : | G¹ | : |
| 113 | 4-Cl | : | : | Phenyl | : | : | : | : | G¹ | : |
| 114 | 4-F | : | : | : | : | : | : | : | G¹ | : |
| 115 | 4- CH₃ | : | : | : | : | : | : | : | G¹ | : |
| 116 | 4- OCH₂CH₃ | : | : | : | : | : | : | : | G¹ | : |
| 117 | H | : | : | CH₃ | 2-pyridyl | : | : | : | G¹ | : |
| 118 | 6-Cl | : | : | : | : | : | : | : | G¹ | : |

| No. | R | R₁ | R₂ | R₃ | Ar¹ | X | m | n | G^{K} | Y |
|---|---|---|---|---|---|---|---|---|---|---|
| 119 | H | H | H | CH₃ | 4-pyridyl | S | 0 | 1 | G¹ | H |
| 120 | 6-H | : | : | : | 3-pyridyl | : | : | : | G¹ | : |
| 121 | 6-Cl | : | : | : | : | : | : | : | G¹ | : |
| 122 | 6- OCH₂CH₃ | : | : | : | : | : | : | : | G¹ | : |
| 123 | 6- OCH₂CF₃ | : | : | : | : | : | : | : | G¹ | : |
| 124 | 6- CH₃ | : | : | : | : | : | : | : | G¹ | : |
| 125 | 6- OCH₃ | : | : | : | : | : | : | : | G¹ | : |
| 126 | 2-H | : | : | : | 5-Pyrimidyl | : | : | : | G¹ | : |
| 127 | 2-Cl | : | : | : | : | : | : | : | G¹ | : |
| 128 | 2-OCH₂CH₃ | : | : | : | : | : | : | : | G¹ | : |
| 129 | H | : | : | : | 2-Furanyl | : | : | : | G¹ | : |
| 130 | 5-Cl | : | : | : | : | : | : | : | G¹ | : |
| 131 | H | : | : | : | 2-Thiophenyl | : | : | : | G¹ | : |
| 132 | 5-Cl | : | : | : | : | : | : | : | G¹ | : |
| 133 | H | : | : | : | 2-Pyrrolyl | : | : | : | G¹ | : |
| 134 | 5-Cl | : | : | : | : | : | : | : | G¹ | : |
| 135 | H | : | : | : | 2-Naphthyl | : | : | : | G¹ | : |
| 136 | H | : | : | : | 2-Benzofuranyl | : | : | : | G¹ | : |
| 137 | H | : | : | : | 2-Benzothiophenyl | : | : | : | G¹ | : |
| 138 | 4-Cl | : | : | : | phenyl | : | 1 | : | G¹ | : |
| 139 | 4-F | : | : | : | : | : | : | : | G¹ | : |
| 140 | 4-Br | : | : | : | : | : | : | : | G¹ | : |
| 141 | 4-CH₃ | : | : | : | : | : | : | : | G¹ | : |
| 142 | 4- OCH₃ | : | : | : | : | : | : | : | G¹ | : |
| 143 | 4- CH₂CH₃ | : | : | : | : | : | : | : | G¹ | : |
| 144 | 4-O CH₂CF₃ | : | : | : | : | : | : | : | G¹ | : |
| 145 | 4-Cl | CH₃ | CH₃ | : | : | : | : | : | G¹ | : |
| 146 | 4-F | : | : | : | : | : | : | : | G¹ | : |
| 147 | 4-Br | : | : | : | : | : | : | : | G¹ | : |
| 148 | 4- CH₃ | : | : | : | : | : | : | : | G¹ | : |
| 149 | 4- OCH₃ | CH₃ | CH₃ | CH₃ | Phenyl | S | 1 | 1 | G¹ | H |
| 150 | 4-O CH₂CF₃ | : | : | : | : | : | : | : | G¹ | : |
| 151 | 4-Cl | H | H | : | : | O | 0 | 1 | G¹ | : |
| 152 | 4-F | : | : | : | : | : | : | : | G¹ | : |
| 153 | 4-Br | : | : | : | : | : | : | : | G¹ | : |
| 154 | 4- CH₃ | : | : | : | : | : | : | : | G¹ | : |
| 155 | 4- OCH₃ | : | : | : | : | : | : | : | G¹ | : |
| 156 | 4- CH₂CH₃ | : | : | : | : | : | : | : | G¹ | : |
| 157 | 4-O CH₂CF₃ | : | : | : | : | : | : | : | G¹ | : |
| 158 | 4-Cl | : | CH₃ | : | : | O | : | : | G¹ | : |
| 159 | 4-F | : | : | : | : | : | : | : | G¹ | : |
| 160 | 4-Br | : | : | : | : | : | : | : | G¹ | : |
| 161 | 4- CH₃ | : | : | : | : | : | : | : | G¹ | : |
| 162 | 4- OCH₃ | : | : | : | : | : | : | : | G¹ | : |
| 163 | 4- CH₂CH₃ | : | : | : | : | : | : | : | G¹ | : |
| 164 | H | : | H | : | 2-Furanyl | : | : | : | G¹ | : |
| 165 | H | : | : | : | 2-Thiophenyl | : | : | : | G¹ | : |
| 166 | H | : | : | : | 2-pyridyl | : | : | : | G¹ | : |
| 167 | 4-Cl | : | : | : | Phenyl | SO₂ | : | : | G¹ | : |
| 168 | 4-F | : | : | : | : | : | : | : | G¹ | : |
| 169 | 4-Br | : | : | : | : | : | : | : | G¹ | : |
| 170 | 4- CH₃ | : | : | : | : | : | : | : | G¹ | : |
| 171 | 4- OCH₃ | : | : | : | : | : | : | : | G¹ | : |
| 172 | 4- CH₂CH₃ | : | : | : | : | : | : | : | G¹ | : |
| 173 | 4-O CH₂CF₃ | : | : | : | : | : | : | : | G¹ | : |
| 174 | 4-Cl | : | CH₃ | : | : | : | : | : | G¹ | : |
| 175 | 4-F | : | : | : | : | : | : | : | G¹ | : |
| 176 | 4-Br | : | : | : | : | : | : | : | G¹ | : |
| 177 | 4- CH₃ | : | : | : | : | : | : | : | G¹ | : |
| 178 | 4- OCH₃ | : | : | : | : | : | : | : | G¹ | : |
| 179 | 4-O CH₂CF₃ | H | CH₃ | CH₃ | Phenyl | SO₂ | 0 | 1 | G¹ | H |
| 180 | 4- CH₂CH₃ | : | : | : | : | : | : | : | G¹ | : |
| 181 | 4-Cl | : | H | : | : | SO | : | : | G¹ | : |
| 182 | 4-F | : | : | : | : | : | : | : | G¹ | : |
| 183 | 4-Br | : | : | : | : | : | : | : | G¹ | : |
| 184 | 4- CH₃ | : | : | : | : | : | : | : | G¹ | : |
| 185 | 4- OCH₃ | : | : | : | : | : | : | : | G¹ | : |
| 186 | 4- OCH₂CH₃ | : | : | : | : | : | : | : | G¹ | : |
| 187 | 4- OCH₃ | : | : | : | : | S | : | 3 | G¹ | : |
| 188 | H | : | CH₃ | : | : | : | : | 1 | G¹ | : |
| 189 | : | CH₃ | : | : | : | : | : | : | G¹ | : |
| 190 | 4- OCH₂CH₃ | H | H | : | : | : | 1 | : | G¹ | : |
| 191 | H | : | CH₃ | : | : | : | : | : | G¹ | : |
| 192 | : | CH₃ | : | : | : | : | : | : | G¹ | : |
| 193 | : | H | H | : | : | SO₂ | 0 | : | G¹ | : |
| 194 | : | : | : | : | : | SO | : | : | G¹ | : |
| 195 | H | : | : | : | : | S | : | 3 | G¹ | : |
| 196 | 4-F | : | : | : | : | : | : | : | G¹ | : |
| 197 | 4- OCH₂CH₃ | : | : | : | : | : | : | : | G¹ | : |
| 198 | H | : | : | : | : | : | : | 2 | G¹ | : |
| 199 | : | : | SCH₃ | : | : | : | : | 1 | G¹ | : |
| 200 | : | CH₃ | : | : | : | : | : | : | G¹ | : |
| 201 | : | H | Cl | : | : | : | : | : | G¹ | : |
| 202 | : | : | Eethyl | : | : | : | : | : | G¹ | : |
| 203 | ; | : | F | : | : | : | : | : | G¹ | : |
| 204 | : | Cl | Cl | : | : | : | : | : | G¹ | : |
| 205 | : | H | *n*-Propyl | : | : | : | : | : | G¹ | : |
| 206 | 2,4,5-Trichoro- | : | H | : | : | : | : | : | G¹ | : |
| 207 | 2,4,5-Trichloro- | : | CH₃ | : | : | : | : | : | G¹ | : |
| 208 | 4- SO₂CH₃ | : | H | : | : | : | : | : | G¹ | : |
| 209 | 4-Cl | H | Ethyl | CH₃ | phenyl | S | 0 | 1 | G⁸ | H |
| 210 | 4- OCH₂CH₃ | : | : | : | : | : | : | : | G⁸ | : |
| 211 | 4- CH₃ | : | : | : | : | : | : | : | G⁸ | : |
| 212 | 3,4-Cl₂ | : | : | : | : | : | : | : | G⁸ | : |
| 213 | 4-Cl | : | H | : | : | : | : | : | G⁸ | : |
| 214 | 4-F | : | : | : | : | : | : | : | G⁸ | : |
| 215 | 4-Br | : | : | : | : | : | : | : | G⁸ | : |
| 216 | 4- CH₃ | : | : | : | : | : | : | : | G⁸ | : |
| 217 | 4- OCH₃ | : | : | : | : | : | : | : | G⁸ | : |
| 218 | 4- CH₂CH₃ | : | : | : | : | : | : | : | G⁸ | : |
| 219 | 4- OCH₂CH₃ | : | : | : | : | : | : | : | G⁸ | : |
| 220 | 4- CH(CH₃)₂ | : | : | : | : | : | : | : | G⁸ | : |
| 221 | 4- SCH₃ | : | : | : | : | : | : | : | G⁸ | : |
| 222 | 4- C(CH₃)₃ | : | : | : | : | : | : | : | G⁸ | : |
| 223 | 4- CH₂CH₂CH₃ | : | : | : | : | : | : | : | G⁸ | : |
| 224 | 4- OCH(CH₃)₂ | : | : | : | : | : | : | : | G⁸ | : |
| 225 | 4- OCH₂CH₂CH₃ | : | : | : | : | : | : | : | G⁸ | : |
| 226 | 4- SOCH₃ | : | : | : | : | : | : | : | G⁸ | : |
| 227 | 3,4-O-CH₂-O | : | : | : | : | : | : | : | G⁸ | : |
| 228 | 4-Phenoxy | : | : | : | : | : | : | : | G⁸ | : |
| 229 | 3,4-Cl₂ | : | : | : | : | : | : | : | G⁸ | : |
| 330 | 3,4-(CH₃)₂ | : | : | : | : | : | : | : | G⁸ | : |
| 231 | 3-Cl,4-CH₃ | : | : | : | : | : | : | : | G⁸ | : |
| 232 | 4- CF₃ | : | : | : | : | : | : | : | G⁸ | : |
| 233 | 4-OCF₃ | : | : | : | : | : | : | : | G⁸ | : |
| 234 | 4-CF₂H | : | : | : | : | : | : | : | G⁸ | : |
| 235 | 4-OCH₂CF₃ | : | : | : | : | : | : | : | G⁸ | : |
| 236 | 3-Br,4-F | : | : | : | : | : | : | : | G⁸ | : |
| 237 | 4-CH₂OCH₃ | : | : | : | : | : | : | : | G⁸ | : |
| 238 | 4-Cl | H | CH₃ | CH₃ | phenyl | S | 0 | 1 | G⁸ | H |
| 239 | 4-F | : | : | : | : | : | : | : | G⁸ | : |
| 240 | 4-Br | : | : | : | : | : | : | : | G⁸ | : |
| 241 | 4- CH₃ | : | : | : | : | : | : | : | G⁸ | : |
| 242 | 4- OCH₃ | : | : | : | : | : | : | : | G⁸ | : |
| 243 | 4- CH₂CH₃ | : | : | : | : | : | : | : | G⁸ | : |
| 244 | 4- OCH₂CH₃ | : | : | : | : | : | : | : | G⁸ | : |
| 245 | 4- CH(CH₃)₂ | : | : | : | : | : | : | : | G⁸ | : |
| 246 | 4- SCH₃ | : | : | : | : | : | : | : | G⁸ | : |
| 247 | 4- C(CH₃)₃ | : | : | : | : | : | : | : | G⁸ | : |
| 248 | 4- CH₂CH₂CH₃ | : | : | : | : | : | : | : | G⁸ | : |
| 249 | 4- OCH(CH₃)₂ | : | : | : | : | : | : | : | G⁸ | : |
| 250 | 4- OCH₂CH₂CH₃ | : | : | : | : | : | : | : | G⁸ | : |
| 251 | 4- SOCH₃ | : | : | : | : | : | : | : | G⁸ | : |
| 252 | 3,4-O-CH₂-O | : | : | : | : | : | : | : | G⁸ | : |
| 253 | 4-Phenoxy | : | : | : | : | : | : | : | G⁸ | : |
| 254 | 3,4-Cl₂ | : | : | : | : | : | : | : | G⁸ | : |
| 255 | 3,4-(CH₃)₂ | : | : | : | : | : | : | : | G⁸ | : |
| 256 | 3-Cl,4-CH₃ | : | : | : | : | : | : | : | G⁸ | : |
| 257 | 4- CF₃ | : | : | : | : | : | : | : | G⁸ | : |
| 258 | 4-OCF₃ | : | : | : | : | : | : | : | G⁸ | : |
| 259 | 4-CF₂H | : | : | : | : | : | : | : | G⁸ | : |
| 260 | 4-OCH₂CF₃ | : | : | : | : | : | : | : | G⁸ | : |
| 261 | 3-Br,4-F | : | : | : | : | : | : | : | G⁸ | : |
| 262 | 4-CH₂OCH₃ | : | : | : | : | : | : | : | G⁸ | : |
| 263 | 4-OCF₂CF₂H | : | H | : | : | : | : | : | G⁸ | : |
| 264 | 4-OCF₂H | : | : | : | : | : | : | : | G⁸ | : |
| 265 | 4-OCH₂CCl=CH₂ | : | : | : | : | : | : | : | G⁸ | : |
| 266 | 4-Ethoxy-ethoxy | : | : | : | : | : | : | : | G⁸ | : |
| 267 | 4-OCH₂CH₂Cl | : | : | : | : | : | : | : | G⁸ | : |
| 268 | 3,4,5-Trifluoro | H | H | CH₃ | phenyl | S | 0 | 1 | G⁸ | H |
| 269 | 4-Acryloxymethyl | : | : | : | : | : | : | : | G⁸ | : |
| 270 | 4-CH₂Cl | : | : | : | : | : | : | : | G⁸ | : |
| 271 | 4-NO₂ | : | : | : | : | : | : | : | G⁸ | : |
| 272 | 3-NO₂ | : | : | : | : | : | : | : | G⁸ | : |
| 273 | 4-OCF₂CF₂H | : | CH₃ | : | : | : | : | : | G⁸ | : |
| 274 | 4-OCF₂H | : | : | : | : | : | : | : | G⁸ | : |
| 275 | 4-OCH₂CCl=CH₂ | : | : | : | : | : | : | : | G⁸ | : |
| 276 | 4-Ethoxy-ethoxy | : | : | : | : | : | : | : | G⁸ | : |
| 277 | 4-OCH₂CH₂Cl | : | : | : | : | : | : | : | G⁸ | : |
| 278 | 3,4,5-Trifluoro | : | : | : | : | : | : | : | G⁸ | : |
| 279 | 4-Acryloxymethyl | : | : | : | : | : | : | : | G⁸ | : |
| 280 | 4- CH₂Cl | : | : | : | : | : | : | : | G⁸ | : |
| 281 | 4-Cl | : | H | : | : | : | : | 3 | G¹ | : |
| 282 | 4-CH₃ | : | : | : | : | : | : | 3 | G¹ | : |
| 283 | 2-Cl | : | : | : | : | : | : | 1 | G¹ | : |
| 284 | 3-Cl | : | : | : | : | : | : | : | G⁸ | : |
| 285 | 3-CF₃ | : | : | : | : | : | : | : | G⁸ | : |
| 286 | 3-Phenoxy | : | : | : | : | : | : | : | G⁸ | : |
| 287 | 2-CH₃ | : | : | : | : | : | : | : | G⁸ | : |
| 288 | 2-Cl | : | CH₃ | : | : | : | : | : | G⁸ | : |
| 289 | 3-Cl | : | : | : | : | : | : | : | G⁸ | : |
| 290 | 3-CF₃ | : | : | : | : | : | : | : | G⁸ | : |
| 291 | 3-Phenoxy | : | : | : | : | : | : | : | G⁸ | : |
| 292 | 2-CH₃ | : | : | : | : | : | : | : | G⁸ | : |
| 293 | 4-Cl | CH₃ | : | : | : | : | : | : | G⁸ | : |
| 294 | 4-F | : | : | : | : | : | : | : | G⁸ | : |
| 295 | 4-Br | : | : | : | : | : | : | : | G⁸ | : |
| 296 | 4-CH₃ | : | : | : | : | : | : | : | G⁸ | : |
| 297 | 4- OCH₃ | CH₃ | CH₃ | CH₃ | Phenyl | S | 0 | 2 | G⁸ | H |
| 298 | 4- CH₂CH₃ | : | : | : | : | : | : | | G⁸ | : |
| 299 | 4- OCH₂CH₃ | : | : | : | : | : | : | : | G⁸ | : |
| 300 | 4-Cl | H | SCH₃ | : | : | : | : | : | G⁸ | : |
| 301 | 4-F | : | : | : | : | : | : | : | G⁸ | : |
| 302 | 4-Br | : | : | : | : | : | : | : | G⁸ | : |
| 303 | 4- CH₃ | : | : | : | : | : | : | : | G⁸ | : |
| 304 | 4- OCH₃ | : | : | : | : | : | : | : | G⁸ | : |
| 305 | 4- CH₂CH₃ | : | : | : | : | : | : | : | G⁸ | : |
| 306 | 4- OCH₂CH₃ | : | : | : | : | : | : | : | G⁸ | : |
| 307 | 4-Cl | : | H | Ethyl | : | : | : | : | G⁸ | : |
| 308 | 4-F | : | : | : | : | : | : | : | G⁸ | : |
| 309 | 4-Br | : | : | : | : | : | : | : | G⁸ | : |
| 310 | 4- CH₃ | : | : | : | : | : | : | : | G⁸ | : |
| 311 | 4- OCH₂CH₃ | : | : | : | : | : | : | : | G⁸ | : |
| 312 | 4-Cl | : | : | *i*-propyl | : | : | : | : | G⁸ | : |
| 313 | 4-F | : | : | : | : | : | : | : | G⁸ | : |
| 314 | 4- OCH₃ | : | : | : | : | : | : | : | G⁸ | : |
| 315 | 4- CH₃ | : | : | : | : | : | : | : | G⁸ | : |
| 316 | 4- OCH₂CH₃ | : | : | : | : | : | : | : | G⁸ | : |
| 317 | 4-Cl | : | : | *n*-Propyl | : | : | : | : | G⁸ | : |
| 318 | 4-F | : | : | : | : | : | : | : | G⁸ | : |
| 319 | 4- OCH₃ | : | : | : | : | : | : | : | G⁸ | : |
| 320 | 4- OCH₂CH₃ | : | : | : | : | : | : | : | G⁸ | : |
| 321 | 4-Cl | : | : | Phenyl | : | : | : | : | G⁸ | : |
| 322 | 4-F | : | : | : | : | : | : | : | G⁸ | : |
| 323 | 4- CH₃ | : | : | : | : | : | : | : | G⁸ | : |
| 324 | 4- OCH₂CH₃ | : | : | : | : | : | : | : | G⁸ | : |
| 325 | H | : | : | CH₃ | 2-pyridyl | : | : | : | G⁸ | : |
| 326 | 6-Cl | : | : | : | : | : | : | : | G⁸ | : |
| 327 | H | H | H | CH₃ | 4-pyridyl | S | 0 | 1 | G⁸ | H |
| 328 | 6-H | : | : | : | 3-pyridyl | : | : | : | G⁸ | : |
| 329 | 6-Cl | : | : | : | : | : | : | : | G⁸ | : |
| 330 | 6- OCH₂CH₃ | : | : | : | : | : | : | : | G⁸ | : |
| 331 | 6 - OCH₂CF₃ | : | : | : | : | : | : | : | G⁸ | : |
| 332 | 6- CH₃ | : | : | : | : | : | : | : | G⁸ | : |
| 333 | 6- OCH₃ | : | : | : | : | : | : | : | G⁸ | : |
| 334 | 2-H | : | : | : | : | : | : | : | G⁸ | : |
| 335 | 2-Cl | : | : | : | 5-Pyrimidyl | : | : | : | G⁸ | : |
| 336 | 2-OCH₂CH₃ | : | : | : | : | : | : | : | G⁸ | : |
| 337 | H | : | : | : | 2-Furanyl | : | : | : | G⁸ | : |
| 338 | 5-Cl | : | : | : | : | : | : | : | G⁸ | : |
| 339 | H | : | : | : | 2-Thiophenyl | : | : | : | G⁸ | : |
| 340 | 5-Cl | : | : | : | : | : | : | : | G⁸ | : |
| 341 | H | : | : | : | 2-Pyrrolyl | : | : | : | G⁸ | : |
| 342 | 5-Cl | : | : | : | : | : | : | : | G⁸ | : |
| 343 | H | : | : | : | 2-Naphthyl | : | : | : | G⁸ | : |
| 344 | H | : | : | : | 2-Benzofuranyl | : | : | : | G⁸ | : |
| 345 | H | : | : | : | 2-Benzothiophenyl | : | : | : | G⁸ | : |
| 346 | 4-Cl | : | : | : | phenyl | : | 1 | : | G⁸ | : |
| 347 | 4-F | : | : | : | : | : | : | : | G⁸ | : |
| 348 | 4-Br | : | : | : | : | : | : | : | G⁸ | : |
| 349 | 4- CH₃ | : | : | : | : | : | : | : | G⁸ | : |
| 350 | 4- OCH₃ | : | : | : | : | : | : | : | G⁸ | : |
| 351 | 4- CH₂CH₃ | : | : | : | : | : | : | : | G⁸ | : |
| 352 | 4-O CH₂CF₃ | : | : | : | : | : | : | : | G⁸ | : |
| 353 | 4-Cl | CH₃ | CH₃ | : | : | : | : | : | G⁸ | : |
| 354 | 4-F | : | : | : | : | : | : | : | G⁸ | : |
| 355 | 4-Br | : | : | : | : | : | : | : | G⁸ | : |
| 356 | 4- CH₃ | : | : | : | : | : | : | : | G⁸ | : |
| 357 | 4- OCH₃ | CH₃ | CH₃ | CH₃ | Phenyl | S | 1 | 1 | G⁸ | H |
| 358 | 4-O CH₂CF₃ | : | : | : | : | : | : | : | G⁸ | : |
| 359 | 4-Cl | H | H | : | : | O | 0 | 1 | G⁸ | : |
| 360 | 4-F | : | : | : | : | : | : | : | G⁸ | : |
| 361 | 4-Br | : | : | : | : | : | : | : | G⁸ | : |
| 362 | 4- CH₃ | : | : | : | : | : | : | : | G⁸ | : |
| 363 | 4- OCH₃ | : | : | : | : | : | : | : | G⁸ | : |
| 364 | 4- CH₂CH₃ | : | : | : | : | : | : | : | G⁸ | : |
| 365 | 4-O CH₂CF₃ | : | : | : | : | : | : | : | G⁸ | : |
| 366 | 4-Cl | : | CH₃ | : | : | O | : | : | G⁸ | : |
| 367 | 4-F | : | : | : | : | : | : | : | G⁸ | : |
| 368 | 4-Br | : | : | : | : | : | : | : | G⁸ | : |
| 369 | 4- CH₃ | : | : | : | : | : | : | : | G⁸ | : |
| 370 | 4- OCH₃ | : | : | : | : | : | : | : | G⁸ | : |
| 371 | 4- CH₂CH₃ | : | : | : | : | : | : | : | G⁸ | : |
| 372 | H | : | H | : | 2-Furanyl | : | : | : | G⁸ | : |
| 373 | H | : | : | : | 2-Thiophenyl | : | : | : | G⁸ | : |
| 374 | H | : | : | : | 2-pyridyl | : | : | : | G⁸ | : |
| 375 | 4-Cl | : | : | : | Phenyl | SO₂ | : | : | G⁸ | : |
| 376 | 4-F | : | : | : | : | : | : | : | G⁸ | : |
| 377 | 4-Br | : | : | : | : | : | : | : | G⁸ | : |
| 378 | 4- CH₃ | : | : | : | : | : | : | : | G⁸ | : |
| 379 | 4- OCH₃ | : | : | : | : | : | : | : | G⁸ | : |
| 380 | 4- CH₂CH₃ | : | : | : | : | : | : | : | G⁸ | : |
| 381 | 4-O CH₂CF₃ | : | : | : | : | : | : | : | G⁸ | : |
| 382 | 4-Cl | : | CH₃ | : | : | : | : | : | G⁸ | : |
| 383 | 4-F | : | : | : | : | : | : | : | G⁸ G⁸ | : |
| 384 | 4-Br | : | : | : | : | : | : | : | G⁸ | : |
| 385 | 4- CH₃ | : | : | : | : | : | : | : | G⁸ | : |
| 386 | 4- OCH₃ | : | : | : | : | : | : | : | G⁸ | : |
| 387 | 4-O CH₂CF₃ | H | CH₃ | CH₃ | Phenyl | SO₂ | : | 1 | G⁸ | H |
| 388 | 4- CH₂CH₃ | : | : | : | : | : | : | : | G⁸ | : |
| 389 | 4-Cl | : | H | : | : | SO | : | : | G⁸ | : |
| 390 | 4-F | : | : | : | : | : | : | : | G⁸ | : |
| 391 | 4-Br | : | : | : | : | : | : | : | G⁸ | : |
| 392 | 4- CH₃ | : | : | : | : | : | : | : | G⁸ | : |
| 393 | 4- OCH₃ | : | : | : | : | : | : | : | G⁸ | : |
| 394 | 4- OCH₂CH₃ | : | : | : | : | : | : | : | G⁸ | : |
| 395 | 4- OCH₃ | : | : | : | : | S | : | 3 | G⁸ | : |
| 396 | H | : | CH₃ | : | : | : | : | 1 | G⁸ | : |
| 397 | : | CH₃ | : | : | : | : | : | : | G⁸ | : |
| 398 | 4- OCH₂CH₃ | H | H | : | : | : | 1 | : | G⁸ | : |
| 399 | H | : | CH₃ | : | : | : | : | : | G⁸ | : |
| 400 | : | CH₃ | : | : | : | : | : | : | G⁸ | : |
| 401 | : | H | H | : | : | SO₂ | 0 | : | G⁸ | : |
| 402 | : | : | : | : | : | SO | : | : | G⁸ | : |
| 403 | H | : | : | : | : | S | : | 3 | G⁸ | : |
| 404 | 4-F | : | : | : | : | : | : | : | G⁸ | : |
| 405 | 4- OCH₂CH₃ | : | : | : | : | : | : | : | G⁸ | : |
| 406 | H | : | : | : | : | : | : | 2 | G⁸ | : |
| 407 | : | : | SCH₃ | : | : | : | : | 1 | G⁸ | : |
| 408 | : | CH₃ | : | : | : | : | : | : | G⁸ | : |
| 409 | : | H | Cl | : | : | : | : | : | G⁸ | : |
| 410 | : | : | Ethyl | : | : | : | : | : | G⁸ | : |
| 411 | : | : | F | : | : | : | : | : | G⁸ | : |
| 412 | : | Cl | Cl | : | : | : | : | : | G⁸ | : |
| 413 | : | H | *n*-Propyl | : | : | : | : | : | G⁸ | : |
| 414 | 2,4,5-Trichloro- | : | H | : | : | : | : | : | G⁸ | : |
| 415 | 2,4,5-Trichloro- | : | CH₃ | : | : | : | : | : | G⁸ | : |
| 416 | 4- SO₂CH₃ | : | H | : | : | : | : | : | G⁸ | : |
| 417 | 4-Cl | H | Ethyl | CH₃ | phenyl | S | 0 | 1 | G¹⁵ | H |
| 418 | 4- OCH₂CH₃ | : | : | : | : | : | : | : | G¹⁵ | : |
| 419 | 4- CH₃ | : | : | : | : | : | : | : | G¹⁵ | : |
| 420 | 3,4-Cl₂ | : | : | : | : | : | : | : | G¹⁵ | : |
| 421 | 4-Cl | : | H | : | : | : | : | : | G¹⁵ | : |
| 422 | 4-F | : | : | : | : | : | : | : | G¹⁵ | : |
| 423 | 4-Br | : | : | : | : | : | : | : | G¹⁵ | : |
| 424 | 4- CH₃ | : | : | : | : | : | : | : | G¹⁵ | : |
| 425 | 4- OCH₃ | : | : | : | : | : | : | : | G¹⁵ | : |
| 426 | 4- CH₂CH₃ | : | : | : | : | : | : | : | G¹⁵ | : |
| 427 | 4- OCH₂CH₃ | : | : | : | : | : | : | : | G¹⁵ | : |
| 428 | 4- CH(CH₃)₂ | : | : | : | : | : | : | : | G¹⁵ | : |
| 429 | 4- SCH₃ | : | : | : | : | : | : | : | G¹⁵ | : |
| 430 | 4- C(CH₃)₃ | : | : | : | : | : | : | : | G¹⁵ | : |
| 431 | 4- CH₂CH₂CH₃ | : | : | : | : | : | : | : | G¹⁵ | : |
| 432 | 4- OCH(CH₃)₂ | : | : | : | : | : | : | : | G¹⁵ | : |
| 433 | 4- OCH₂CH₂CH₃ | : | : | : | : | : | : | : | G¹⁵ | : |
| 434 | 4- SOCH₃ | : | : | : | : | : | : | : | G¹⁵ | : |
| 435 | 3,4-O-CH₂-O | : | : | : | : | : | : | : | G¹⁵ | : |
| 436 | 4-Phenoxy | : | : | : | : | : | : | : | G¹⁵ | : |
| 437 | 3,4-Cl₂ | : | : | : | : | : | : | : | G¹⁵ | : |
| 438 | 3,4-(CH₃)₂ | : | : | : | : | : | : | : | G¹⁵ | : |
| 439 | 3-C1,4-CH₃ | : | : | : | : | : | : | : | G¹⁵ | : |
| 440 | 4- CF₃ | : | : | : | : | : | : | : | G¹⁵ | : |
| 441 | 4-OCF₃ | : | : | : | : | : | : | : | G¹⁵ | : |
| 442 | 4-CF₂H | : | : | : | : | : | : | : | G¹⁵ | : |
| 443 | 4-OCH₂CF₃ | : | : | : | : | : | : | : | G¹⁵ | : |
| 444 | 3-Br,4-F | : | : | : | : | : | : | : | G¹⁵ | : |
| 445 | 4-CH₂OCH₃ | : | : | : | : | : | : | : | G¹⁵ | : |
| 446 | 4-Cl | H | CH₃ | CH₃ | : | S | 0 | 1 | G¹⁵ | H |
| 447 | 4-F | : | : | : | phenyl | : | : | : | : G¹⁵ | : |
| 448 | 4-Br | : | : | : | : | : | : | : | G¹⁵ | : |
| 449 | 4- CH₃ | : | : | : | : | : | : | : | G¹⁵ | : |
| 450 | 4- OCH₃ | : | : | : | : | : | : | : | G¹⁵ | : |
| 451 | 4- CH₂CH₃ | : | : : | : | : | : | : | : | G¹⁵ | : |
| 452 | 4- OCH₂CH₃ | : | : | : | : | : | : | : | G¹⁵ | : |
| 453 | 4- CH(CH₃)₂ | : | : | : | : | : | : | : | G¹⁵ | : |
| 454 | 4- SCH₃ | : | : | : | : | : | : | : | G¹⁵ | : |
| 455 | 4- C(CH₃)₃ | : | : | : | : | : | : | : | G¹⁵ | : |
| 456 | 4- CH₂CH₂CH₃ | : | : | : | : | : | : | : | G¹⁵ | : |
| 457 | 4- OCH(CH₃)₂ | : | : | : | : | : | : | : | G¹⁵ | : |
| 458 | 4- OCH₂CH₂CH₃ | : | : | : | : | : | : | : | G¹⁵ | : |
| 459 | 4- SOCH₃ | : | : | : | : | : | : | : | G¹⁵ | : |
| 460 | 3,4-O-CH₂-O | : | : | : | : | : | : | : | G¹⁵ | : |
| 461 | 4-Phenoxy | : | : | : | : | : | : | : | G¹⁵ | : |
| 462 | 3,4-Cl₂ | : | : | : | : | : | : | : | G¹⁵ | : |
| 463 | 3,4-(CH₃)₂ | : | : | : | : | : | : | : | G¹⁵ | : |
| 464 | 3-C1,4-CH₃ | : | : | : | : | : | : | : | G¹⁵ | : |
| 465 | 4- CF₃ | : | : | : | : | : | : | : | G¹⁵ | : |
| 466 | 4-OCF₃ | : | : | : | : | : | : | : | G¹⁵ | : |
| 467 | 4-CF₂H | : | : | : | : | : | : | : | G¹⁵ | : |
| 468 | 4-OCH₂CF₃ | : | : | : | : | : | : | : | G¹⁵ | : |
| 469 | 3-Br,4-F | : | : | : | : | : | : | : | G¹⁵ | : |
| 470 | 4-CH₂OCH₃ | : | : | : | : | : | : | : | G¹⁵ | : |
| 471 | 4-OCF₂CF₂H | : | H | : | : | : | : | : | G¹⁵ | : |
| 472 | 4-OCF₂H | : | : | : | : | : | : | : | G¹⁵ | : |
| 473 | 4-OCH₂CCl=CH₂ | : | : | : | : | : | : | : | G¹⁵ | : |
| 474 | 4-Ethoxy-ethoxy | : | : | : | : | : | : | : | G¹⁵ | : |
| 475 | 4-OCH₂CH₂Cl | : | : | : | : | : | : | : | G¹⁵ | : |
| 476 | 3,4,5-Trifluoro | H | H | CH₃ | phenyl | S | 0 | 1 | G¹⁵ | H |
| 477 | 4-Acryloxymethyl | : | : | : | : | : | : | : | G¹⁵ | : |
| 478 | 4-CH₂Cl | : | : | : | : | : | : | : | G¹⁵ | : |
| 479 | 4-NO₂ | : | : | : | : | : | : | : | G¹⁵ | : |
| 480 | 3-NO₂ | : | : | : | : | : | : | : | G¹⁵ | : |
| 481 | 4-OCF₂CF₂H | : | CH₃ | : | : | : | : | : | G¹⁵ | : |
| 482 | 4-OCF₂H | : | : | : | : | : | : | : | G¹⁵ | : |
| 483 | 4-OCH₂CCl=CH₂ | : | : | : | : | : | : | : | G¹⁵ | : |
| 484 | 4-Ethoxy-ethoxy | : | : | : | : | : | : | : | G¹⁵ | : |
| 485 | 4-OCH₂CH₂Cl | : | : | : | : | : | : | : | G¹⁵ | : |
| 486 | 3,4,5-Trifluoro | : | : | : | : | : | : | : | G¹⁵ | : |
| 487 | 4-Acryloxymethyl | : | : | : | : | : | : | : | G¹⁵ | : |
| 488 | 4- CH₂Cl | : | : | : | : | : | : | : | G¹⁵ | : |
| 489 | 4-Cl | : | H | : | : | : | : | 3 | G¹⁵ | : |
| 490 | 4-CH₃ | : | : | : | : | : | : | 3 | G¹⁵ | : |
| 491 | 2-Cl | : | : | : | : | : | : | 1 | G¹⁵ | : |
| 492 | 3-Cl | : | : | : | : | : | : | : | G¹⁵ | : |
| 493 | 3-CF₃ | : | : | : | : | : | : | : | G¹⁵ | : |
| 494 | 3-Phenoxy | : | : | : | : | : | : | : | G¹⁵ | : |
| 495 | 2-CH₃ | : | : | : | : | : | : | : | G¹⁵ | : |
| 496 | 2-Cl | : | CH₃ | : | : | : | : | : | G¹⁵ | : |
| 497 | 3-Cl | : | : | : | : | : | : | : | G¹⁵ | : |
| 498 | 3-CF₃ | : | : | : | : | : | : | : | G¹⁵ | : |
| 499 | 3-Phenoxy | : | : | : | : | : | : | : | G¹⁵ | : |
| 500 | 2-CH₃ | : | : | : | : | : | : | : | G¹⁵ | : |
| 501 | 4-Cl | CH₃ | : | : | : | : | : | : | G¹⁵ | : |
| 502 | 4-F | : | : | : | : | : | : | : | G¹⁵ | : |
| 503 | 4-Br | : | : | : | : | : | : | : | G¹⁵ | : |
| 504 | 4- CH₃ | : | : | : | : | : | : | : | G¹⁵ | : |

| No. | R | R₁ | R₂ | R₃ | Ar¹ | X | m | m | G^{K} | : |
|---|---|---|---|---|---|---|---|---|---|---|
| 505 | 4- OCH₃ | CH₃ | CH₃ | CH₃ | Phenyl | S | 0 | 1 | G¹⁵ | H |
| 506 | 4- CH₂CH₃ | : | : | : | : | : | : | : | G¹⁵ | : |
| 507 | 4- OCH₂CH₃ | : | : | : | : | : | : | : | G¹⁵ | : |
| 508 | 4-Cl | H | SCH₃ | : | : | : | : | : | G¹⁵ | : |
| 509 | 4-F | : | : | : | : | : | : | : | G¹⁵ | : |
| 510 | 4-Br | : | : | : | : | : | : | : | G¹⁵ | : |
| 511 | 4- CH₃ | : | : | : | : | : | : | : | G¹⁵ | : |
| 512 | 4- OCH₃ | : | : | : | : | : | : | : | G¹⁵ | : |
| 513 | 4- CH₂CH₃ | : | : | : | : | : | : | : | G¹⁵ | : |
| 514 | 4- OCH₂CH₃ | : | : | : | : | : | : | : | G¹⁵ | : |
| 515 | 4-Cl | : | H | Methyl | : | : | : | : | G¹⁵ | : |
| 516 | 4-F | : | : | : | : | : | : | : | G¹⁵ | : |
| 517 | 4-Br | : | : | : | : | : | : | : | G¹⁵ | : |
| 518 | 4- CH₃ | : | : | : | : | : | : | : | G¹⁵ | : |
| 519 | 4- OCH₂CH₃ | : | : | : | : | : | : | : | G¹⁵ | : |
| 520 | 4-Cl | : | : | *i*-propyl | : | : | : | : | G¹⁵ | : |
| 521 | 4-F | : | : | : | : | : | : | : | G¹⁵ | : |
| 522 | 4- OCH₃ | : | : | : | : | : | : | : | G¹⁵ | : |
| 523 | 4- CH₃ | : | : | : | : | : | : | : | G¹⁵ | : |
| 524 | 4- OCH₂CH₃ | : | : | : | : | : | : | : | G¹⁵ | : |
| 525 | 4-Cl | : | : | *n*-Propyl | : | : | : | : | G¹⁵ | : |
| 526 | 4-F | : | : | : | : | : | : | : | G¹⁵ | : |
| 527 | 4- OCH₃ | : | : | : | : | : | : | : | G¹⁵ | : |
| 528 | 4- OCH₂CH₃ | : | : | : | : | : | : | : | G¹⁵ | : |
| 529 | 4-Cl | : | : | Phenyl | : | : | : | : | G¹⁵ | : |
| 530 | 4-F | : | : | : | : | : | : | : | G¹⁵ | : |
| 531 | 4- CH₃ | : | : | : | : | : | : | : | G¹⁵ | : |
| 532 | 4- OCH₂CH₃ | : | : | : | : | : | : | : | G¹⁵ | : |
| 533 | H | : | : | CH₃ | 2-pyridyl | : | : | : | G¹⁵ | : |
| 534 | 6-Cl | : | : | : | : | : | : | : | G¹⁵ | : |

| No. | R | R₁ | R₂ | R₃ | Ar¹ | X | m | n | G^{K} | Y |
|---|---|---|---|---|---|---|---|---|---|---|
| 535 | H | H | H | CH₃ | 4-pyridyl | S | 0 | 1 | G¹⁵ | H |
| 536 | 6-H | : | : | : | 3-pyridyl | : | : | : | G¹⁵ | : |
| 537 | 6-Cl | : | : | : | : | : | : | : | G¹⁵ | : |
| 538 | 6- OCH₂CH₃ | : | : | : | : | : | : | : | G¹⁵ | : |
| 539 | 6 - OCH₂CF₃ | : | : | : | : | : | : | : | G¹⁵ | : |
| 540 | 6- CH₃ | : | : | : | : | : | : | : | G¹⁵ | : |
| 541 | 6- OCH₃ | : | : | : | : | : | : | : | G¹⁵ | : |
| 542 | 2-H | : | : | : | 5-Pyrimidyl | : | : | : | G¹⁵ | : |
| 543 | 2-Cl | : | : | : | : | : | : | : | G¹⁵ | : |
| 544 | 2-OCH₂CH₃ | : | : | : | : | : | : | : | G¹⁵ | : |
| 545 | H | : | : | : | 2-Furanyl | : | : | : | G¹⁵ | : |
| 546 | 5-Cl | : | : | : | : | : | : | : | G¹⁵ | : |
| 547 | H | : | : | : | 2-Thiophenyl : | : | : | : | G¹⁵ | : |
| 548 | 5-Cl | : | : | : | : | : | : | : | G¹⁵ | : |
| 549 | H | : | : | : | 2-Pyrrolyl | : | : | : | G¹⁵ | : |
| 550 | 5-Cl | : | : | : | : | : | : | : | G¹⁵ | : |
| 551 | H | : | : | : | 2-Naphthyl | : | : | : | G¹⁵ | : |
| 552 | H | : | : | : | 2-Benzofuranyl | : | : | : | G¹⁵ | : |
| 553 | H | : | : | : | 2-Benzothiophenyl | : | : | : | G¹⁵ | : |
| 554 | 4-Cl | : | : | : | phenyl | : | 1 | : | G¹⁵ | : |
| 555 | 4-F | : | : | : | : | : | : | : | G¹⁵ | : |
| 556 | 4-Br | : | : | : | : | : | : | : | G¹⁵ | : |
| 557 | 4- CH₃ | : | : | : | : | : | : | : | G¹⁵ | : |
| 558 | 4- OCH₃ | : | : | : | : | : | : | : | G¹⁵ | : |
| 559 | 4- CH₂CH₃ | : | : | : | : | : | : | : | G¹⁵ | : |
| 560 | 4-O CH₂CF₃ | : | : | : | : | : | : | : | G¹⁵ | : |
| 561 | 4-Cl | CH₃ | CH₃ | : | : | : | : | : | G¹⁵ | : |
| 562 | 4-F | : | : | : | : | : | : | : | G¹⁵ | : |
| 563 | 4-Br | : | : | : | : | : | : | : | G¹⁵ | : |
| 564 | 4- CH₃ | : | : | : | : | : | | | G¹⁵ | : |
| 565 | 4- OCH₃ | CH₃ | CH₃ | CH₃ | Phenyl | S | 1 | 1 | G¹⁵ | H |
| 566 | 4-O CH₂CF₃ | : | : | : | : | : | : | : | G¹⁵ | : |
| 567 | 4-Cl | H | H | : | : | O | 0 | 1 | G¹⁵ | : |
| 568 | 4-F | : | : | : | : | : | : | : | G¹⁵ | : |
| 569 | 4-Br | : | : | : | : | : | : | : | G¹⁵ | : |
| 570 | 4- CH₃ | : | : | : | : | : | : | : | G¹⁵ | : |
| 571 | 4- OCH₃ | : | : | : | : | : | : | : | G¹⁵ | : |
| 572 | 4- CH₂CH₃ | : | : | : | : | : | : | : | G¹⁵ | : |
| 573 | 4-O CH₂CF₃ | : | : | : | : | : | : | : | G¹⁵ | : |
| 574 | 4-Cl | : | CH₃ | : | : | O | : | : | G¹⁵ | : |
| 575 | 4-F | : | : | : | : | : | : | : | G¹⁵ | : |
| 576 | 4-Br | : | : | : | : | : | : | : | G¹⁵ | : |
| 577 | 4- CH₃ | : | : | : | : | : | : | : | G¹⁵ | : |
| 578 | 4- OCH₃ | : | : | : | : | : | : | : | G¹⁵ | : |
| 579 | 4- CH₂CH₃ | : | : | : | : | : | : | : | G¹⁵ | : |
| 580 | H | : | H | : | 2-Furanyl | : | : | : | G¹⁵ | : |
| 581 | H | : | : | : | 2-Thiophenyl | : | : | : | G¹⁵ | : |
| 582 | H | : | : | : | 2-pyridyl | : | : | : | G¹⁵ | : |
| 583 | 4-Cl | : | : | : | Phenyl | SO₂ | : | : | G¹⁵ | : |
| 584 | 4-F | : | : | : | : | : | : | : | G¹⁵ | : |
| 585 | 4-Br | : | : | : | : | : | : | : | G¹⁵ | : |
| 586 | 4- CH₃ | : | : | : | : | : | : | : | G" | : |
| 587 | 4- OCH₃ | : | : | : | : | : | : | : | G¹⁵ | : |
| 588 | 4- CH₂CH₃ | : | : | : | : | : | : | : | G¹⁵ | : |
| 589 | 4-O CH₂CF₃ | : | : | : | : | : | : | : | G¹⁵ | : |
| 590 | 4-Cl | : | CH₃ | : | : | : | : | : | G¹⁵ | : |
| 591 | 4-F | : | : | : | : | : | : | : | G¹⁵ | : |
| 592 | 4-Br | : | : | : | : | : | : | : | G¹⁵ | : |
| 593 | 4- CH₃ | : | : | : | : | : | : | : | G¹⁵ | : |
| 594 | 4- OCH₃ | : | : | : | : | : | : | : | G¹⁵ | : |
| 595 | 4-O CH₂CF₃ | H | CH₃ | CH₃ | Phenyl | SO₂ | 0 | 1 | G¹⁵ | H |
| 596 | 4- CH₂CH₃ | : | : | : | : | : | : | : | G¹⁵ | : |
| 597 | 4-Cl | : | H | : | : | SO | : | : | G¹⁵ | : |
| 598 | 4-F | : | : | : | : | : | : | : | G¹⁵ | : |
| 599 | 4-Br | : | : | : | : | : | : | : | G¹⁵ | : |
| 600 | 4- CH₃ | : | : | : | : | : | : | : | G¹⁵ | : |
| 601 | 4- OCH₃ | : | : | : | : | : | : | : | G¹⁵ | : |
| 602 | 4- OCH₂CH₃ | : | : | : | : | : | : | : | G¹⁵ | : |
| 603 | 4- OCH₃ | : | : | : | : | S | : | 3 | G¹⁵ | : |
| 604 | H | : | CH₃ | : | : | : | : | 1 | G¹⁵ | : |
| 605 | : | CH₃ | : | : | : | : | : | : | G¹⁵ | : |
| 606 | 4- OCH₂CH₃ | H | H | : | : | : | 1 | : | G¹⁵ | : |
| 607 | H | : | CH₃ | : | : | : | : | : | G¹⁵ | : |
| 608 | : | CH₃ | : | : | : | : | : | : | G¹⁵ | : |
| 609 | : | H | : | : | : | SO₂ | 0 | : | G¹⁵ | : |
| 610 | : | : | H | : | : | SO | : | : | G¹⁵ | : |
| 611 | H | : | : | : | : | S | : | 3 | G¹⁵ | : |
| 612 | 4-F | : | : | : | : | : | : | : | G¹⁵ | : |
| 613 | 4- OCH₂CH₃ | : | : | : | : | : | : | : | G¹⁵ | : |
| 614 | H | : | : | : | : | : | : | 2 | G¹⁵ | : |
| 615 | : | : | SCH₃ | : | : | : | : | 1 | G¹⁵ | : |
| 616 | : | CH₃ | : | : | : | : | : | : | G¹⁵ | : |
| 617 | : | H | Cl | : | : | : | : | : | G¹⁵ | : |
| 618 | : | : | Ethyl | : | : | : | : | : | G¹⁵ | : |
| 619 | : | : | F | : | : | : | : | : | G¹⁵ | : |
| 620 | : | Cl | Cl | : | : | : | : | : | G¹⁵ | : |
| 621 | : | H | *n*-Propyl | : | : | : | : | : | G¹⁵ | : |
| 622 | 2-Trichloro- | : | H | : | : | : | : | : | G¹⁵ | : |
| 623 | 2,4,5-Trichloro- | : | CH₃ | : | : | : | : | : | G¹⁵ | : |
| 624 | 4- SO₂CH₃ | : | H | : | : | : | : | : | G¹⁵ | : |
| 625 | 4-Cl | H | Ethyl | CH₃ | phenyl | S | 0 | 1 | G¹¹ | H |
| 626 | 4- OCH₂CH₃ | : | : | : | : | : | : | : | G¹¹ | : |
| 627 | 4- CH₃ | : | : | : | : | : | : | : | G¹¹ | : |
| 628 | 3,4-Cl₂ | : | : | : | : | : | : | : | G¹¹ | : |
| 629 | 4-Cl | : | H | : | : | : | : | : | G¹¹ | : |
| 630 | 4-F | : | : | : | : | : | : | : | G¹¹ | : |
| 631 | 4-Br | : | : | : | : | : | : | : | G¹¹ | : |
| 632 | 4- CH₃ | : | : | : | : | : | : | : | G¹¹ | : |
| 633 | 4- OCH₃ | : | : | : | : | : | : | : | G¹¹ | : |
| 634 | 4- CH₂CH₃ | : | : | : | : | : | : | : | G¹¹ | : |
| 635 | 4- OCH₂CH₃ | : | : | : | : | : | : | : | G¹¹ | : |
| 636 | 4- CH(CH₃)₂ | : | : | : | : | : | : | : | G¹¹ | : |
| 637 | 4- SCH₃ | : | : | : | : | : | : | : | G¹¹ | : |
| 638 | 4- C(CH₃)₃ | : | : | : | : | : | : | : | G¹¹ | : |
| 639 | 4- CH₂CH₂CH₃ | : | : | : | : | : | : | : | G¹¹ | : |
| 640 | 4- OCH(CH₃)₂ | : | : | : | : | : | : | : | G¹¹ | : |
| 641 | 4- OCH₂CH₂CH₃ | : | : | : | : | : | : | : | G¹¹ | : |
| 642 | 4- SOCH₃ | : | : | : | : | : | : | : | G¹¹ | : |
| 643 | 3,4-O-CH₂-O | : | : | : | : | : | : | : | G¹¹ | : |
| 644 | 4-Phenoxy | : | : | : | : | : | : | : | G¹¹ | : |
| 645 | 3,4-Cl₂ | : | : | : | : | : | : | : | G¹¹ | : |
| 646 | 3,4-(CH₃)₂ | : | : | : | : | : | : | : | G¹¹ | : |
| 647 | 3-C1,4-CH₃ | : | : | : | : | : | : | : | G¹¹ | : |
| 648 | 4- CF₃ | : | : | : | : | : | : | : | G¹¹ | : |
| 649 | 4-OCF₃ | : | : | : | : | : | : | : | G¹¹ | : |
| 650 | 4-CF₂H | : | : | : | : | : | : | : | G¹¹ | : |
| 651 | 4-OCH₂CF₃ | : | : | : | : | : | : | : | G¹¹ | : |
| 652 | 3-Br,4-F | : | : | : | : | : | : | : | G¹¹ | : |
| 653 | 4-CH₂OCH₃ | : | : | : | : | : | : | : | G¹¹ | : |
| 654 | 4-Cl | H | CH₃ | CH₃ | phenyl | S | 0 | 1 | G¹¹ | H |
| 655 | 4-F | : | : | : | : | : | : | : | G¹¹ | : |
| 656 | 4-Br | : | : | : : | : | : | : | : | G¹¹ | : |
| 657 | 4- CH₃ | : | : | : | : | : | : | : | G¹¹ | : |
| 658 | 4- OCH₃ | : | : | : | : | : | : | : | G¹¹ | : |
| 659 | 4- CH₂CH₃ | : | : | : | : | : | : | : | G¹¹ | : |
| 660 | 4- OCH₂CH₃ | : | : | : | : | : | : | : | G¹¹ | : |
| 661 | 4- CH(CH₃)₂ | : | : | : | : | : | : | : | G¹¹ | : |
| 662 | 4- SCH₃ | : | : | : | : | : | : | : | G¹¹ | : |
| 663 | 4-C(CH₃)₃ | : | : | : | : | : | : | : | G¹¹ | : |
| 664 | 4- CH₂CH₂CH₃ | : | : | : | : | : | : | : | G¹¹ | : |
| 665 | 4- OCH(CH₃)₂ | : | : | : | : | : | : | : | G¹¹ | : |
| 666 | 4- OCH₂CH₂CH₃ | : | : | : | : | : | : | : | G¹¹ | : |
| 667 | 4- SOCH₃ | : | : | : | : | : | : | : | G¹¹ | : |
| 668 | 3,4-O-CH₂-O | : | : | : | : | : | : | : | G¹¹ | : |
| 669 | 4-Phenoxy | : | : | : | : | : | : | : | G¹¹ | : |
| 670 | 3,4-Cl₂ | : | : | : | : | : | : | : | G¹¹ | : |
| 671 | 3,4-(CH₃)₂ | : | : | : | : | : | : | : | G¹¹ | : |
| 672 | 3-Cl,4-CH₃ | : | : | : | : | : | : | : | G¹¹ | : |
| 673 | 4- CF₃ | : | : | : | : | : | : | : | G¹¹ | : |
| 674 | 4-OCF₃ | : | : | : | : | : | : | : | G¹¹ | : |
| 675 | 4-CF₂H | : | : | : | : | : | : | : | G¹¹ | : |
| 676 | 4-OCH₂CF₃ | : | : | : | : | : | : | : | G¹¹ | : |
| 677 | 3-Br,4-F | : | : | : | : | : | : | : | G¹¹ | : |
| 678 | 4-CH₂OCH₃ | : | : | : | : | : | : | : | G¹¹ | : |
| 679 | 4-OCF₂CF₂H | : | H | : | : | : | : | : | G¹¹ | : |
| 680 | 4-OCF₂H | : | : | : | : | : | : | : | G¹¹ | : |
| 681 | 4-OCH₂CCl=CH₂ | : | : | : | : | : | : | : | G¹¹ | : |
| 682 | 4-Ethoxy-ethoxy | : | : | : | : | : | : | : | G¹¹ | : |
| 683 | 4-OCH₂CH₂Cl | : | : | : | : | : | : | : | G¹¹ | : |
| 684 | 3,4,5-Trifluoro | H | H | CH₃ | phenyl | S | 0 | 1 | G¹¹ | H |
| 685 | 4-Acryloxymethyl | : | : | : | : | : | : | : | G¹¹ | : |
| 686 | 4-CH₂Cl | : | : | : | : | : | : | : | G¹¹ | : |
| 687 | 4-NO₂ | : | : | : | : | : | : | : | G¹¹ | : |
| 688 | 3-NO₂ | : | : | : | : | : | : | : | G¹¹ | : |
| 689 | 4-OCF₂CF₂H | : | CH₃ | : | : | : | : | : | G¹¹ | : |
| 690 | 4-OCF₂H | : | : | : | : | : | : | : | G¹¹ | : |
| 691 | 4-OCH₂CCl=CH₂ | : | : | : | : | : | : | : | G¹¹ | : |
| 692 | 4-Ethoxy-ethoxy | : | : | : | : | : | : | : | G¹¹ | : |
| 693 | 4-OCH₂CH₂Cl | : | : | : | : | : | : | : | G¹¹ | : |
| 694 | 3,4,5-Trifluoro | : | : | : | : | : | : | : | G¹¹ | : |
| 695 | 4-Acryloxymethyl | : | : | : | : | : | : | : | G¹¹ | : |
| 696 | 4- CH₂Cl | : | : | : | : | : | : | : | G¹¹ | : |
| 697 | 4-Cl | : | H | : | : | : | : | 3 | G¹¹ | : |
| 698 | 4-CH₃ | : | : | : | : | : | : | 3 | G¹¹ | : |
| 699 | 2-Cl | : | : | : | : | : | : | 1 | G¹¹ | : |
| 700 | 3-Cl | : | : | : | : | : | : | : | G¹¹ | : |
| 701 | 3-CF₃ | : | : | : | : | : | : | : | G¹¹ | : |
| 702 | 3-Phenoxy | : | : | : | : | : | : | : | G¹¹ | : |
| 703 | 2-CH₃ | : | : | : | : | : | : | : | G¹¹ | : |
| 704 | 2-Cl | : | CH₃ | : | : | : | : | : | G¹¹ | : |
| 705 | 3-Cl | : | : | : | : | : | : | : | G¹¹ | : |
| 706 | 3-CF₃ | : | : | : | : | : | : | : | G¹¹ | : |
| 707 | 3-Phenoxy | : | : | : | : | : | : | : | G¹¹ | : |
| 708 | 2-CH₃ | : | : | : | : | : | : | : | G¹¹ | : |
| 709 | 4-Cl | CH₃ | : | : | : | : | : | : | G¹¹ | : |
| 710 | 4-F | : | : | : | : | : | : | : | G¹¹ | : |
| 711 | 4-Br | : | : | : | : | : | : | : | G¹¹ | : |
| 712 | 4- CH₃ | : | : | : | : | : | : | : | G¹¹ | : |
| 713 | 4- OCH₃ | CH₃ | CH₃ | CH₃ | Phenyl | S | 0 | 1 | G¹¹ | H |
| 714 | 4- CH₂CH₃ | : | : | : | : | : | : | : | G¹¹ | : |
| 715 | 4- OCH₂CH₃ | : | : | : | : | : | : | : | G¹¹ | : |
| 716 | 4-Cl | H | SCH₃ | : | : | : | : | : | G¹¹ | : |
| 717 | 4-F | : | : | : | : | : | : | : | G¹¹ | : |
| 718 | 4-Br | : | : | : | : | : | : | : | G¹¹ | : |
| 719 | 4- CH₃ | : | : | : | : | : | : | : | G¹¹ | : |
| 720 | 4- OCH₃ | : | : | : | : | : | : | : | G¹¹ | : |
| 721 | 4- CH₂CH₃ | : | : | : | : | : | : | : | G¹¹ | : |
| 722 | 4- OCH₂CH₃ | : | : | : | : | : | : | : | G¹¹ | : |
| 723 | 4-Cl | : | H | Ethyl | : | : | : | : | G¹¹ | : |
| 724 | 4-F | : | : | : | : | : | : | : | G¹¹ | : |
| 725 | 4-Br | : | : | : | : | : | : | : | G¹¹ | : |
| 726 | 4- CH₃ | : | : | : | : | : | : | : | G¹¹ | : |
| 727 | 4- OCH₂CH₃ | : | : | : | : | : | : | : | G¹¹ | : |
| 728 | 4-Cl | : | : | *i*-Propyl | : | : | : | : | G¹¹ | : |
| 729 | 4-F | : | : | : | : | : | : | : | G¹¹ | : |
| 730 | 4- OCH₃ | : | : | : | : | : | : | : | G¹¹ | : |
| 731 | 4- CH₃ | : | : | : | : | : | : | : | G¹¹ | : |
| 732 | 4- OCH₂CH₃ | : | : | : | : | : | : | : | G¹¹ | : |
| 733 | 4-Cl | : | : | *n*-Propyl | : | : | : | : | G¹¹ | : |
| 734 | 4-F | : | : | : | : | : | : | : | G¹¹ | : |
| 735 | 4- OCH₃ | : | : | : | : | : | : | : | G¹¹ | : |
| 736 | 4- OCH₂CH₃ | : | : | : | : | : | : | : | G¹¹ | : |
| 737 | 4-Cl | : | : | : | : | : | : | : | G¹¹ | : |
| 738 | 4-F | : | : | Phenyl | : | : | : | : | G¹¹ | : |
| 739 | 4- CH₃ | : | : | : | : | : | : | : | G¹¹ | : |
| 740 | 4- OCH₂CH₃ | : | : | : | : | : | : | : | G¹¹ | : |
| 741 | H | : | : | CH₃ | 2-pyridyl | : | : | : | G¹¹ | : |
| 742 | 6-Cl | : | : | : | : | : | : | : | G¹¹ | : |
| 743 | H | H | H | CH₃ | 4-pyridyl | S | 0 | 1 | G¹¹ | H |
| 744 | 6-H | : | : | : | 3-pyridyl | : | : | : | G¹¹ | : |
| 745 | 6-Cl | : | : | : | : | : | : | : | G¹¹ | : |
| 746 | 6- OCH₂CH₃ | : | : | : | : | : | : | : | G¹¹ | : |
| 747 | 6- OCH₂CF₃ | : | : | : | : | : | : | : | G¹¹ | : |
| 748 | 6- CH₃ | : | : | : | : | : | : | : | G¹¹ | : |
| 749 | 6- OCH₃ | : | : | : | : | : | : | : | G¹¹ | : |
| 750 | 2-H | : | : | : | 5-Pyrimidyl | : | : | : | G¹¹ | : |
| 751 | 2-Cl | : | : | : | : | : | : | : | G¹¹ | : |
| 752 | 2-OCH₂CH₃ | : | : | : | : | : | : | : | G¹¹ | : |
| 753 | H | : | : | : | 2-Furanyl | : | : | : | G¹¹ | : |
| 754 | 5-Cl | : | : | : | : | : | : | : | G¹¹ | : |
| 755 | H | : | : | : | 2-Thiophenyl | : | : | : | G¹¹ | : |
| 756 | 5-Cl | : | : | : | : | : | : | : | G¹¹ | : |
| 757 | H | : | : | : | 2-Pyrrolyl | : | : | : | G¹¹ | : |
| 758 | 5-Cl | : | : | : | : | : | : | : | G¹¹ | : |
| 759 | H | : | : | : | 2-Naphthyl | : | : | : | G¹¹ | : |
| 760 | H | : | : | : | 2-Benzofuranyl | : | : | : | _{G}11 | : |
| 761 | H | : | : | : | 2-Benzothiophenyl | : | : | : | G¹¹ | : |
| 762 | 4-Cl | : | : | : | phenyl | : | 1 | : | G¹¹ | : |
| 763 | 4-F | : | : | : | : | : | : | : | G¹¹ | : |
| 764 | 4-Br | : | : | : | : | : | : | : | G¹¹ | : |
| 765 | 4- CH₃ | : | : | : | : | : | : | : | G¹¹ | : |
| 766 | 4- OCH₃ | : | : | : | : | : | : | : | G¹¹ | : |
| 767 | 4- CH₂CH₃ | : | : | : | : | : | : | : | G¹¹ | : |
| 768 | 4-OCH₂CF₃ | : | : | : | : | : | : | : | G¹¹ | : |
| 769 | 4-Cl | CH₃ | CH₃ | : | : | : | : | : | G¹¹ | : |
| 770 | 4-F | : | : | : | : | : | : | : | G¹¹ | : |
| 771 | 4-Br | : | : | : | : | : | : | : | G¹¹ | : |
| 772 | 4- CH₃ | : | : | : | : | : | : | : | G¹¹ | : |
| 773 | 4- OCH₃ | CH₃ | CH₃ | CH₃ | Phenyl | S | 1 | 1 | G¹¹ | H |
| 774 | 4-O CH₂CF₃ | : | : | : | : | : | : | : | G¹¹ | : |
| 775 | 4-Cl | H | H | : | : | O | 0 | 1 | G¹¹ | : |
| 776 | 4-F | : | : | : | : | : | : | : | G¹¹ | : |
| 777 | 4-Br | : | : | : | : | : | : | : | G¹¹ | : |
| 778 | 4- CH₃ | : | : | : | : | : | : | : | G¹¹ | : |
| 779 | 4- OCH₃ | : | : | : | : | : | : | : | G¹¹ | : |
| 780 | 4- CH₂CH₃ | : | : | : | : | : | : | : | G¹¹ | : |
| 781 | 4-O CH₂CF₃ | : | : | : | : | : | : | : | G¹¹ | : |
| 782 | 4-Cl | : | CH₃ | : | : | O | : | : | G¹¹ | : |
| 783 | 4-F | : | : | : | : | : | : | : | G¹¹ | : |
| 784 | 4-Br | : | : | : | : | : | : | : | G¹¹ | : |
| 785 | 4- CH₃ | : | : | : | : | : | : | : | G¹¹ | : |
| 786 | 4- OCH₃ | : | : | : | : | : | : | : | G¹¹ | : |
| 787 | 4- CH₂CH₃ | : | : | : | : | : | : | : | G¹¹ | : |
| 788 | H | : | H | : | 2-Furanyl | : | : | : | G¹¹ | : |
| 789 | H | : | : | : | 2-Thiophenyl | : | : | : | G¹¹ | : |
| 790 | H | : | : | : | 2-pyridyl | : | : | : | G¹¹ | : |
| 791 | 4-Cl | : | : | : | Phenyl | SO₂ | : | : | G¹¹ | : |
| 792 | 4-F | : | : | : | : | : | : | : | G¹¹ | : |
| 793 | 4-Br | : | : | : | : | : | : | : | G¹¹ | : |
| 794 | 4- CH₃ | : | : | : | : | : | : | : | G¹¹ | : |
| 795 | 4- OCH₃ | : | : | : | : | : | : | : | G¹¹ | : |
| 796 | 4- CH₂CH₃ | : | : | : | : | : | : | : | G¹¹ | : |
| 797 | 4-O CH₂CF₃ | : | : | : | : | : | : | : | G¹¹ | : |
| 798 | 4-Cl | : | CH₃ | : | : | : | : | : | G¹¹ | : |
| 799 | 4-F | : | : | : | : | : | : | : | G¹¹ | : |
| 800 | 4-Br | : | : | : | : | : | : | : | G¹¹ | : |
| 801 | 4- CH₃ | : | : | : | : | : | : | : | G¹¹ | : |
| 802 | 4- OCH₃ | : | : | : | : | : | : | : | G¹¹ | : |
| 803 | 4-O CH₂CF₃ | H | CH₃ | CH₃ | Phenyl | SO₂ | 0 | 1 | G¹¹ | H |
| 804 | 4- CH₂CH₃ | : | : | : | : | : | : | : | G¹¹ | : |
| 805 | 4-Cl | : | : | : | : | SO | : | : | G¹¹ | : |
| 806 | 4-F | : | H | : | : | : | : | : | G¹¹ | : |
| 807 | 4-Br | : | : | : | : | : | : | : | G¹¹ | : |
| 808 | 4- CH₃ | : | : | : | : | : | : | : | G¹¹ | : |
| 809 | 4- OCH₃ | : | : | : | : | : | : | : | G¹¹ | : |
| 810 | 4- OCH₂CH₃ | : | : | : | : | : | : | : | G¹¹ | : |
| 811 | 4- OCH₃ | : | : | : | : | S | : | 3 | G¹¹ | : |
| 812 | H | : | CH₃ | : | : | : | : | 1 | G¹¹ | : |
| 813 | : | CH₃ | : | : | : | : | : | : | G¹¹ | : |
| 814 | 4- OCH₂CH₃ | H | H | : | : | : | 1 | : | G¹¹ | : |
| 815 | H | : | CH₃ | : | : | : | : | : | G¹¹ | : |
| 816 | : | CH₃ | : | : | : | : | : | : | G¹¹ | : |
| 817 | : | H | H | : | : | SO₂ | 0 | : | G¹¹ | : |
| 818 | : | : | : | : : | | SO | : | : | G¹¹ | : |
| 819 | H | : | : | : | : | S | : | 3 | G¹¹ | : |
| 820 | 4-F | : | : | : | : | : | : | : | G¹¹ | : |
| 821 | 4- OCH₂CH₃ | : | : | : | : | : | : | : | G¹¹ | : |
| 822 | H | : | : | : | : | : | : | 2 | G¹¹ | : |
| 823 | : | : | SCH₃ | : | : | : | : | 1 | G¹¹ | : |
| 824 | : | CH₃ | : | : | : | : | : | : | G¹¹ | : |
| 825 | : | H | Cl | : | : | : | : | : | G¹¹ | : |
| 826 | : | : | Ethyl | : | : | : | : | : | G¹¹ | : |
| 827 | : | : | F | : | : | : | : | : | G¹¹ | : |
| 828 | : | Cl | Cl | : | : | : | : | : | G¹¹ | : |
| 829 | : | H | *n*-Propyl | : | : | : | : | : | G¹¹ | : |
| 830 | 2,4,5-Trichloro- | : | H | : | : | : | : | : | G¹¹ | : |
| 831 | 2,4,5-Trichloro- | : | CH₃ | : | : | : | : | : | G¹¹ | : |
| 832 | 4- SO₂CH₃ | : | H | : | : | : | : | : | G¹¹ | : |
| 833 | 4-Cl | H | Ethyl | CH₃ | phenyl | S | 0 | 1 | G²⁴ | H |
| 834 | 4- OCH₂CH₃ | : | : | : | : | : | : | : | G²⁴ | : |
| 835 | 4- CH₃ | : | : | : | : | : | : | : | G²⁴ | : |
| 836 | 3,4-Cl₂ | : | : | : | : | : | : | : | G²⁴ | : |
| 837 | 4-Cl | : | H | : | : | : | : | : | G²⁴ | : |
| 838 | 4-F | : | : | : | : | : | : | : | G²⁴ | : |
| 839 | 4-Br | : | : | : | : | : | : | : | G²⁴ | : |
| 840 | 4-CH₃ | : | : | : | : | : | : | : | G²⁴ | : |
| 841 | 4- OCH₃ | : | : | : | : | : | : | : | G²⁴ | : |
| 842 | 4- CH₂CH₃ | : | : | : | : | : | : | : | G²⁴ | : |
| 843 | 4- OCH₂CH₃ | : | : | : | : | : | : | : | G²⁴ | : |
| 844 | 4- CH(CH₃)₂ | : | : | : | : | : | : | : | G²⁴ | : |
| 845 | 4- SCH₃ | : | : | : | : | : | : | : | G²⁴ | : |
| 846 | 4- C(CH₃)₃ | : | : | : | : | : | : | : | G²⁴ | : |
| 847 | 4- CH₂CH₂CH₃ | : | : | : | : | : | : | : | G²⁴ | : |
| 848 | 4- OCH(CH₃)₂ | : | : | : | : | : | : | : | G²⁴ | : |
| 849 | 4- OCH₂CH₂CH₃ | : | : | : | : | : | : | : | G²⁴ | : |
| 850 | 4- SOCH₃ | : | : | : | : | : | : | : | G²⁴ | : |
| 851 | 3,4-O-CH₂-O | : | : | : | : | : | : | : | G²⁴ | : |
| 852 | 4-Phenoxy | : | : | : | : | : | : | : | G²⁴ | : |
| 853 | 3,4-Cl₂ | : | : | : | : | : | : | : | G²⁴ | : |
| 854 | 3,4-(CH₃)₂ | : | : | : | : | : | : | : | G²⁴ | : |
| 855 | 3-Cl,4-CH₃ | : | : | : | : | : | : | : | G²⁴ | : |
| 856 | 4- CF₃ | : | : | : | : | : | : | : | G²⁴ | : |
| 857 | 4-OCF₃ | : | : | : | : | : | : | : | G²⁴ | : |
| 858 | 4-CF₂H | : | : | : | : | : | : | : | G²⁴ | : |
| 859 | 4-OCH₂CF₃ | : | : | : | : | : | : | : | G²⁴ | : |
| 860 | 3-Br,4-F | : | : | : | : | : | : | : | G²⁴ | : |
| 861 | 4-CH₂OCH₃ | : | : | : | : | : | : | : | G²⁴ | : |
| 862 | 4-Cl | H | CH₃ | CH₃ | phenyl | S | 0 | 1 | G²⁴ | H |
| 863 | 4-F | : | : | : | : | : | : | : | G²⁴ | : |
| 864 | 4-Br | : | : | : | : | : | : | : | G²⁴ | : |
| 865 | 4-CH₃ | : | : | : | : | : | : | : | G²⁴ | : |
| 866 | 4- OCH₃ | : | : | : | : | : | : | : | G²⁴ | : |
| 867 | 4- CH₂CH₃ | : | : | : | : | : | : | : | G²⁴ | : |
| 868 | 4- OCH₂CH₃ | : | : | : | : | : | : | : | G²⁴ | : |
| 869 | 4- CH(CH₃)₂ | : | : | : | : | : | : | : | G²⁴ | : |
| 870 | 4- SCH₃ | : | : | : | : | : | : | : | G²⁴ | : |
| 871 | 4-C(CH₃)₃ | : | : | : | : | : | : | : | G²⁴ | : |
| 872 | 4- CH₂CH₂CH₃ | : | : | : | : | : | : | : | G²⁴ | : |
| 873 | 4- OCH(CH₃)₂ | : | : | : | : | : | : | : | G²⁴ | : |
| 874 | 4- OCH₂CH₂CH₃ | : | : | : | : | : | : | : | G²⁴ | : |
| 875 | 4- SOCH₃ | : | : | : | : | : | : | : | G²⁴ | : |
| 876 | 3,4-O-CH₂-O | : | : | : | : | : | : | : | G²⁴ | : |
| 877 | 4-Phenoxy | : | : | : | : | : | : | : | G²⁴ | : |
| 878 | 3,4-Cl₂ | : | : | : | : | : | : | : | G²⁴ | : |
| 879 | 3,4-(CH₃)₂ | : | : | : | : | : | : | : | G²⁴ | : |
| 880 | 3-Cl,4-CH₃ | : | : | : | : | : | : | : | G²⁴ | : |
| 881 | 4- CF₃ | : | : | : | : | : | : | : | G²⁴ | : |
| 882 | 4-OCF₃ | : | : | : | : | : | : | : | G²⁴ | : |
| 883 | 4-CF₂H | : | : | : | : | : | : | : | G²⁴ | : |
| 884 | 4-OCH₂CF₃ | : | : | : | : | : | : | : | G²⁴ | : |
| 885 | 3-Br,4-F | : | : | : | : | : | : | : | G²⁴ | : |
| 886 | 4-CH₂OCH₃ | : | : | : | : | : | : | : | G²⁴ | : |
| 887 | 4-OCF₂CF₂H | : | H | : | : | : | : | : | G²⁴ | : |
| 888 | 4-OCF₂H | : | : | : | : | : | : | : | G²⁴ | : |
| 889 | 4-OCH₂CCl=CH₂ | : | : | : | : | : | : | : | G²⁴ | : |
| 890 | 4-Ethoxy-ethoxy | : | : | : | : | : | : | : | G²⁴ | : |
| 891 | 4-OCH₂CH₂Cl | : | : | : | : | : | : | : | G²⁴ | : |
| 892 | 3,4,5-Trifluoro | H | H | CH₃ | phenyl | S | 0 | 1 | G²⁴ | H |
| 893 | 4-Acryloxymethyl | : | : | : | : | : | : | : | G²⁴ | : |
| 894 | 4-CH₂Cl | : | : | : | : | : | : | : | G²⁴ | : |
| 895 | 4-NO₂ | : | : | : | : | : | : | : | G²⁴ | : |
| 896 | 3-NO₂ | : | : | : | : | : | : | : | G²⁴ | : |
| 897 | 4-OCF₂CF₂H | : | CH₃ | : | : | : | : | : | G²⁴ | : |
| 898 | 4-OCF₂H | : | : | : | : | : | : | : | G²⁴ | : |
| 899 | 4-OCH₂CCl=CH₂ | : | : | : | : | : | : | : | G²⁴ | : |
| 900 | 4-Ethoxy-ethoxy | : | : | : | : | : | : | : | G²⁴ | : |
| 901 | 4-OCH₂CH₂Cl | : | : | : | : | : | : | : | G²⁴ | : |
| 902 | 3,4,5-Trifluoro | : | : | : | : | : | : | : | G²⁴ | : |
| 903 | 4-Acryloxymethyl | : | : | : | : | : | : | : | G²⁴ | : |
| 904 | 4- CH₂Cl | : | : | : | : | : | : | : | G²⁴ | : |
| 905 | 4-Cl | : | H | : | : | : | : | 3 | G²⁴ | : |
| 906 | 4-CH₃ | : | : | : | : | : | : | 3 | G²⁴ | : |
| 907 | 2-Cl | : | : | : | : | : | : | 1 | G²⁴ | : |
| 908 | 3-Cl | : | : | : | : | : | : | : | G²⁴ | : |
| 909 | 3-CF₃ | : | : | : | : | : | : | : | G²⁴ | : |
| 910 | 3-Phenoxy | : | : | : | : | : | : | : | G²⁴ | : |
| 911 | 2-CH₃ | : | : | : | : | : | : | : | G²⁴ | : |
| 912 | 2-Cl | : | SCH₃ | : | : | : | : | : | G²⁴ | : |
| 913 | 3-Cl | : | : | : | : | : | : | : | G²⁴ | : |
| 914 | 3-CF₃ | : | : | : | : | : | : | : | G²⁴ | : |
| 915 | 3-Phenoxy | : | : | : | : | : | : | : | G²⁴ | : |
| 916 | 2-CH₃ | : | : | : | : | : | : | : | G²⁴ | : |
| 917 | 4-Cl | CH₃ | : | : | : | : | : | : | G²⁴ | : |
| 918 | 4-F | : | : | : | : | : | : | : | G²⁴ | : |
| 919 | 4-Br | : | : | : | : | : | : | : | G²⁴ | : |
| 920 | 4- CH₃ | : | : | : | : | : | : | : | G²⁴ | : |

| No. | R | R₁ | R₂ | R₃ | Ar¹ | X | m | n | G^{K} | : |
|---|---|---|---|---|---|---|---|---|---|---|
| 921 | 4- OCH₃ | CH₃ | CH₃ | CH₃ | Phenyl | S | 0 | 1 | G²⁴ | H |
| 922 | 4- CH₂CH₃ | : | : | : | : | : | : | : | G²⁴ | : |
| 923 | 4- OCH₂CH₃ | : | : | : | : | : | : | : | G²⁴ | : |
| 924 | 4-Cl | H | SCH₃ | : | : | : | : | : | G²⁴ | : |
| 925 | 4-F | : | : | : | : | : | : | : | G²⁴ | : |
| 926 | 4-Br | : | : | : | : | : | : | : | G²⁴ | : |
| 927 | 4- CH₃ | : | : | : | : | : | : | : | G²⁴ | : |
| 928 | 4- OCH₃ | : | : | : | : | : | : | : | G²⁴ | : |
| 929 | 4- CH₂CH₃ | : | : | : | : | : | : | : | G²⁴ | : |
| 930 | 4- OCH₂CH₃ | : | : | : | : | : | : | : | G²⁴ | : |
| 931 | 4-Cl | : | H | Ethyl | : | : | : | : | G²⁴ | : |
| 932 | 4-F | : | : | : | : | : | : | : | G²⁴ | : |
| 933 | 4-Br | : | : | : | : | : | : | : | G²⁴ | : |
| 934 | 4- CH₃ | : | : | : | : | : | : | : | G²⁴ | : |
| 935 | 4- OCH₂CH₃ | : | : | : | : | : | : | : | G²⁴ | : |
| 936 | 4-Cl | : | : | Isopropyl | : | : | : | : | G²⁴ | : |
| 937 | 4-F | : | : | : | : | : | : | : | G²⁴ | : |
| 938 | 4- OCH₃ | : | : | : | : | : | : | : | G²⁴ | : |
| 939 | 4- CH₃ | : | : | : | : | : | : | : | G²⁴ | : |
| 940 | 4- OCH₂CH₃ | : | : | : | : | : | : | : | G²⁴ | : |
| 941 | 4-Cl | : | : | *n*-Propyl | : | : | : | : | G²⁴ | : |
| 942 | 4-F | : | : | : | : | : | : | : | G²⁴ | : |
| 943 | 4- OCH₃ | : | : | : | : | : | : | : | G²⁴ | : |
| 944 | 4- OCH₂CH₃ | : | : | : | : | : | : | : | G²⁴ | : |
| 945 | 4-Cl | : | : | Phenyl | : | : | : | : | G²⁴ | : |
| 946 | 4-F | : | : | : | : | : | : | : | G²⁴ | : |
| 947 | 4- CH₃ | : | : | : | : | : | : | : | G²⁴ | : |
| 948 | 4- OCH₂CH₃ | : | : | : | : | : | : | : | G²⁴ | : |
| 949 | H | : | : | CH₃ | 2-pyridyl | : | : | : | G²⁴ | : |
| 950 | 6-Cl | : | : | : | : | : | : | : | G²⁴ | : |
| 951 | H | H | H | CH₃ | 4-pyridyl | S | 0 | 1 | G²⁴ | H |
| 952 | 6-H | : | : | : | 3-pyridyl | : | : | : | G²⁴ | : |
| 953 | 6-Cl | : | : | : | : | : | : | : | G²⁴ | : |
| 954 | 6- OCH₂CH₃ | : | : | : | : | : | : | : | G²⁴ | : |
| 955 | 6 - OCH₂CF₃ | : | : | : | : | : | : | : | G²⁴ | : |
| 956 | 6- CH₃ | : | : | : | : | : | : | : | G²⁴ | : |
| 957 | 6- OCH₃ | : | : | : | : | : | : | : | G²⁴ | : |
| 958 | 2-H | : | : | : | 5-Pyrimidyl | : | : | : | G²⁴ | : |
| 959 | 2-Cl | : | : | : | : | : | : | : | G²⁴ | : |
| 960 | 2-OCH₂CH₃ | : | : | : | : | : | : | : | G²⁴ | : |
| 961 | H | : | : | : | 2-Furanyl | : | : | : | G²⁴ | : |
| 962 | 5-Cl | : | : | : | : | : | : | : | G²⁴ | : |
| 963 | H | : | : | : | 2-Thiophenyl | : | : | : | G²⁴ | : |
| 964 | 5-Cl | : | : | : | : | : | : | : | G²⁴ | : |
| 965 | H | : | : | : | 2-Pyrrolyl | : | : | : | G²⁴ | : |
| 966 | 5-Cl | : | : | : | : | : | : | : | G²⁴ | : |
| 967 | H | : | : | : | 2-Naphthyl | : | : | : | G²⁴ | : |
| 968 | H | : | : | : | 2-Benzofuranyl | : | : | : | G²⁴ | : |
| 969 | H | : | : | : | 2-Benzothiophenyl | : | : | : | G²⁴ | : |
| 970 | 4-Cl | : | : | : | phenyl | 1 | 1 | : | G²⁴ | : |
| 971 | 4-F | : | : | : | : | : | : | : | G²⁴ | : |
| 972 | 4-Br | : | : | : | : | : | : | : | G²⁴ | : |
| 973 | 4- CH₃ | : | : | : | : | : | : | : | G²⁴ | : |
| 974 | 4- OCH₃ | : | : | : | : | : | : | : | G²⁴ | : |
| 975 | 4- CH₂CH₃ | : | : | : | : | : | : | : | G²⁴ | : |
| 976 | 4-O CH₂CF₃ | : | : | : | : | : | : | : | G²⁴ | : |
| 977 | 4-Cl | CH₃ | CH₃ | : | : | : | : | : | G²⁴ | : |
| 978 | 4-F | : | : | : | : | : | : | : | G²⁴ | : |
| 979 | 4-Br | : | : | : | : | : | : | : | G²⁴ | : |
| 980 | 4- CH₃ | : | : | : | : | : | : | : | G²⁴ | : |
| 981 | 4- OCH₃ | CH₃ | CH₃ | CH₃ | Phenyl | S | 1 | 1 | G²⁴ | H |
| 982 | 4-O CH₂CF₃ | : | : | : | : | : | : | : | G²⁴ | : |
| 983 | 4-Cl | H | H | : | : | O | 0 | 1 | G²⁴ | : |
| 984 | 4-F | : | : | : | : | : | : | : | G²⁴ | : |
| 985 | 4-Br | : | : | : | : | : | : | : | G²⁴ | : |
| 986 | 4- CH₃ | : | : | : | : | : | : | : | G²⁴ | : |
| 987 | 4- OCH₃ | : | : | : | : | : | : | : | G²⁴ | : |
| 988 | 4- CH₂CH₃ | : | : | : | : | : | : | : | G²⁴ | : |
| 989 | 4-O CH₂CF₃ | : | : | : | : | : | : | : | G²⁴ | : |
| 990 | 4-Cl | : | CH₃ | : | : | O | : | : | G²⁴ | : |
| 991 | 4-F | : | : | : | : | : | : | : | G²⁴ | : |
| 992 | 4-Br | : | : | : | : | : | : | : | G²⁴ | : |
| 993 | 4- CH₃ | : | : | : | : | : | : | : | G²⁴ | : |
| 994 | 4- OCH₃ | : | : | : | : | : | : | : | G²⁴ | : |
| 995 | 4- CH₂CH₃ | : | : | : | : | : | : | : | G²⁴ | : |
| 996 | H | : | H | : | 2-Furanyl | : | : | : | G²⁴ | : |
| 997 | H | : | : | : | 2-Thiophenyl | : | : | : | G²⁴ | : |
| 998 | H | : | : | : | 2-pyridyl | : | : | : | G²⁴ | : |
| 999 | 4-Cl | : | : | : | Phenyl | SO₂ | : | : | G²⁴ | : |
| 1000 | 4-F | : | : | : | : | : | : | : | G²⁴ | : |
| 1001 | 4-Br | : | : | : | : | : | : | : | G²⁴ | : |
| 1002 | 4- CH₃ | : | : | : | : | : | : | : | G²⁴ | : |
| 1003 | 4- OCH₃ | : | : | : | : | : | : | : | G²⁴ | : |
| 1004 | 4- CH₂CH₃ | : | : | : | : | : | : | : | G²⁴ | : |
| 1005 | 4-O CH₂CF₃ | : | : | : | : | : | : | : | G²⁴ | : |
| 1006 | 4-Cl | : | CH₃ | : | : | : | : | : | G²⁴ | : |
| 1007 | 4-F | : | : | : | : | : | : | : | G²⁴ | : |
| 1008 | 4-Br | : | : | : | : | : | : | : | G⁷ | : |
| 1009 | 4- CH₃ | : | : | : | : | : | : | : | G²⁴ | : |
| 1010 | 4- OCH₃ | : | : | : | : | : | : | : | G²⁴ | : |
| 1011 | 4-O CH₂CF₃ | H | CH₃ | CH₃ | Phenyl | SO₂ | 0 | 1 | G²⁴ | H |
| 1012 | 4- CH₂CH₃ | : | : | : | : | : | : | : | G²⁴ | : |
| 1013 | 4-Cl | : | : | : | : | SO | : | : | G²⁴ | : |
| 1014 | 4-F | : | : | : | : | : | : | : | G²⁴ | : |
| 1015 | 4-Br | : | H | : | : | : | : | : | G²⁴ | : |
| 1016 | 4-CH₃ | : | : | : | : | : | : | : | G²⁴ | : |
| 1017 | 4- OCH₃ | : | : | : | : | : | : | : | G²⁴ | : |
| 1018 | 4- OCH₂CH₃ | : | : | : | : | : | : | : | G²⁴ | : |
| 1019 | 4- OCH₃ | : | : | : | : | S | : | 3 | G²⁴ | : |
| 1020 | H | : | CH₃ | : | : | : | : | 1 | G²⁴ | : |
| 1021 | : | CH₃ | : | : | : | : | : | : | G²⁴ | : |
| 1022 | 4-Cl | H | H | : | : | : | 1 | : | G²⁴ | : |
| 1023 | H | : | CH₃ | : | : | : | : | : | G²⁴ | : |
| 1024 | : | CH₃ | : | : | : | : | : | : | G²⁴ | : |
| 1025 | : | H | H | : | : | SO₂ | 0 | : | G²⁴ | : |
| 1026 | : | : | : | : | : | SO | : | : | G²⁴ | : |
| 1027 | H | : | : | : | : | S | : | 3 | G²⁴ | : |
| 1028 | 4-F | : | : | : | : | : | : | : | G²⁴ | : |
| 1029 | 4-OCH₂CH₃ | : | : | : | : | : | : | : | G²⁴ | : |
| 1030 | H | : | : | : | : | : | : | 2 | G²⁴ | : |
| 1031 | : | : | SCH₃ | : | : | : | : | 1 | G²⁴ | : |
| 1032 | : | CH₃ | : | : | : | : | : | : | G²⁴ | : |
| 1033 | : | H | Cl | : | : | : | : | : | G²⁴ | : |
| 1034 | : | | Eethyl | : | : | : | : | : | G²⁴ | : |
| 1035 | : | | F | : | : | : | : | : | G²⁴ | : |
| 1036 | : | Cl | Cl | : | : | : | : | : | G²⁴ | : |
| 1037 | : | H | *n*-Propyl | : | : | : | : | : | G²⁴ | : |
| 1038 | 2,4,5-Trichloro- | : | H | : | : | : | : | : | G²⁴ | : |
| 1039 | 2,4,5-Trichloro- | : | CH₃ | : | : | : | : | : | G²⁴ | : |
| 1040 | 4- SO₂CH₃ | : | H | : | : | : | : | : | G²⁴ | : |
| 1041 | 4-Cl | H | Ethyl | CH₃ | phenyl | S | 0 | 1 | G² | H |
| 1042 | : | : | H | : | : | SO | : | : | G² | : |
| 1043 | : | : | : | : | : | SO₂ | 0 | : | G² | : |
| 1044 | : | : | : | : | : | O | : | : | G² | : |
| 1045 | 4-Cl | : | : | : | : | S | : | : | G² | : |
| 1046 | 4-F | : | : | : | : | : | : | : | G² | : |
| 1047 | 4-Br | : | : | : | : | : | : | : | G² | : |
| 1048 | 4- CH₃ | : | : | : | : | : | : | : | G² | : |
| 1049 | 4- OCH₃ | : | : | : | : | : | : | : | G² | : |
| 1050 | 4- OCH₂CH₃ | : | : | : | : | : | : | : | G² | : |
| 1051 | 4- OCH₂CF₃ | : | : | : | : | : | : | : | G² | : |
| 1052 | 3,4-Cl₂ | : | : | : | : | : | : | : | G² | : |
| 1053 | 4- CF₃ | : | : | : | : | : | : | : | G² | : |
| 1054 | 3-Cl,4-CH₃ | : | : | : | : | : | : | : | G² | : |
| 1055 | 3,4-(CH₃)₂ | : | : | : | : | : | : | : | G² | : |
| 1056 | 3,4-O-CH₂-O | : | : | : | : | : | : | : | G² | : |
| 1057 | 3,4-Cl₂ | : | CH₃ | : | : | : | : | : | G² | : |
| 1058 | 4-Cl | : | : | : | : | : | : | : | G² | : |
| 1059 | 4- CH₃ | : | : | : | : | : | : | : | G² | : |
| 1060 | 4- OCH₂CH₃ | : | : | : | : | : | : | : | G² | : |
| 1061 | 3,4- (CH₃)₂ | : | : | : | : | : | : | : | G² | : |
| 1062 | 4-Br | : | : | : | : | : | : | : | G² | : |
| 1063 | 4- OCH₂CF₃ | : | : | : | : | : | : | : | G² | : |
| 1064 | 3-Cl,4-CH₃ | : | : | : | : | : | : | : | G² | : |
| 1065 | 4-F | : | : | : | : | : | : | : | G² | : |
| 1066 | 4- CF₃ | : | : | : | : | : | : | : | G² | : |
| 1067 | 6- OCH₂CF₃ | : | : | : | 3-pyridyl | : | : | : | G² | : |
| 1068 | 6-Cl | : | : | : | : | : | : | : | G² | : |
| 1069 | H | : | : | : | : | : | : | : | G² | : |
| 1070 | 4-Cl | H | Ethyl | CH₃ | phenyl | S | 0 | 1 | G⁹ | H |
| 1071 | : | : | H | : | : | SO | : | : | G⁹ | : |
| 1072 | : | : | : | : | : | SO₂ | : | : | G⁹ | : |
| 1073 | : | : | : | : | : | O | : | : | G⁹ | : |
| 1074 | 4-Cl | : | : | : | : | : | : | : | G⁹ | : |
| 1075 | 4-F | : | : | : | : | : | : | : | G⁹ | : |
| 1076 | 4-Br | : | : | : | : | : | : | : | G⁹ | : |
| 1077 | 4- CH₃ | : | : | : | : | : | : | : | G⁹ | : |
| 1078 | 4- OCH₃ | : | : | : | : | : | : | : | G⁹ | : |
| 1079 | 4- OCH₂CH₃ | : | : | : | : | : | : | : | G⁹ | : |
| 1080 | 4- OCH₂CF₃ | : | : | : | : | : | : | : | G⁹ | : |
| 1081 | 3,4-Cl₂ | : | : | : | : | : | : | : | G⁹ | : |
| 1082 | 4- CF₃ | : | : | : | : | : | : | : | G⁹ | : |
| 1083 | 3-Cl,4-CH₃ | : | : | : | : | S | : | : | G⁹ | : |
| 1084 | 3,4-(CH₃)₂ | : | : | : | : | : | : | : | G⁹ | : |
| 1085 | 3,4-O-CH₂-O | : | : | : | : | : | : | : | G⁹ | : |
| 1086 | 3,4-Cl₂ | : | CH₃ | : | : | : | : | : | G⁹ | : |
| 1087 | 4-Cl | : | : | : | : | : | : | : | G⁹ | : |
| 1088 | 4-CH₃ | : | : | : | : | : | : | : | G⁹ | : |
| 1089 | 4- OCH₂CH₃ | : | : | : | : | : | : | : | G⁹ | : |
| 1090 | 3,4- (CH₃)₂ | : | : | : | : | : | : | : | G⁹ | : |
| 1091 | 4-Br | : | : | : | : | : | : | : | G⁹ | : |
| 1092 | 4- OCH₂CF₃ | : | : | : | : | : | : | : | G⁹ | : |
| 1093 | 3-Cl,4-CH₃ | : | : | : | : | : | : | : | G⁹ | : |
| 1094 | 4-F | : | : | : | : | : | : | : | G⁹ | : |
| 1095 | 4- CF₃ | : | : | : | : | : | : | : | G⁹ | : |
| 1096 | 6- OCH₂CF₃ | : | : | : | 3-pyridyl | : | : | : | G⁹ | : |
| 1097 | 6-Cl | : | : | : | : | : | : | : | G⁹ | : |
| 1098 | H | : | : | : | : | : | : | : | G⁹ | : |
| 1099 | 6- CH₃ | : | : | : | : | : | : | : | G⁹ | : |
| 1100 | 4-Cl | H | Ethyl | CH₃ | phenyl | S | 0 | 1 | G⁴ | H |
| 1101 | : | : | H | : | : | SO | : | : | G⁴ | : |
| 1102 | : | : | : | : | : | SO₂ | : | : | G⁴ | : |
| 1103 | : | : | : | : | : | O | : | : | G⁴ | : |
| 1104 | 4-Cl | : | : | : | : | S | : | : | G⁴ | : |
| 1105 | 4-F | : | : | : | : | : | : | : | G⁴ | : |
| 1106 | 4-Br | : | : | : | : | : | : | : | G⁴ | : |
| 1107 | 4- CH₃ | : | : | : | : | : | : | : | G⁴ | : |
| 1108 | 4- OCH₃ | : | : | : | : | : | : | : | G⁴ | : |
| 1109 | 4- OCH₂CH₃ | : | : | : | : | : | : | : | G⁴ | : |
| 1110 | 4- OCH₂CF₃ | : | : | : | : | : | : | : | G⁴ | : |
| 1111 | 3,4-Cl₂ | : | : | : | : | : | : | : | G⁴ | : |
| 1112 | 4- CF₃ | : | : | : | : | : | : | : | G⁴ | : |
| 1113 | 3-Cl,4-CH₃ | : | : | : | : | : | : | : | G⁴ | : |
| 1114 | 3,4-(CH₃)₂ | : | : | : | : | : | : | : | G⁴ | : |
| 1115 | 3,4-O-CH₂-O | : | : | : | : | : | : | : | G⁴ | : |
| 1116 | 3,4-Cl₂ | : | CH₃ | : | : | : | : | : | G⁴ | : |
| 1117 | 4-Cl | : | : | : | : | : | : | : | G⁴ | : |
| 1118 | 4- CH₃ | : | : | : | : | : | : | : | G⁴ | : |
| 1119 | 4- OCH₂CH₃ | : | : | : | : | : | : | : | G⁴ | : |
| 1120 | 3,4- (CH₃)₂ | : | : | : | : | : | : | : | G⁴ | : |
| 1121 | 4-Br | : | : | : | : | : | : | : | G⁴ | : |
| 1122 | 4- OCH₂CF₃ | : | : | : | : | : | : | : | G⁴ | : |
| 1123 | 3-Cl,4-CH₃ | : | : | : | : | : | : | : | G⁴ | : |
| 1124 | 4-F | : | : | : | : | : | : | : | G⁴ | : |
| 1125 | 4- CF₃ | : | : | : | : | : | : | : | G⁴ | : |
| 1126 | 6- OCH₂CF₃ | : | : | : | 3-pyridyl | : | : | : | G⁴ | : |
| 1127 | 6-Cl | : | : | : | : | : | : | : | G⁴ | : |
| 1128 | H | : | : | : | : | : | : | : | G⁴ | : |
| 1129 | 4-Cl | H | Ethyl | CH₃ | phenyl | S | : | : | G¹⁰ | H |
| 1130 | : | : | H | : | : | SO | : | : | G¹⁰ | : |
| 1131 | : | : | : | : | : | SO₂ | : | : | G¹⁰ | : |
| 1132 | : | : | : | : | : | O | : | : | G¹⁰ | : |
| 1133 | 4-Cl | : | : | : | : | S | : | : | G¹⁰ | : |
| 1134 | 4-F | : | : | : | : | : | : | : | G¹⁰ | : |
| 1135 | 4-Br | : | : | : | : | : | : | : | G¹⁰ | : |
| 1136 | 4- CH₃ | : | : | : | : | : | : | : | G¹⁰ | : |
| 1137 | 4- OCH₃ | : | : | : | : | : | : | : | G¹⁰ | : |
| 1138 | 4- OCH₂CH₃ | : | : | : | : | : | : | : | G¹⁰ | : |
| 1139 | 4- OCH₂CF₃ | : | : | : | : | : | : | : | G¹⁰ | : |
| 1140 | 3,4-Cl₂ | : | : | : | : | : | : | : | G¹⁰ | : |
| 1141 | 4- CF₃ | : | : | : | : | : | : | : | G¹⁰ | : |
| 1142 | 3-Cl,4-CH₃ | : | : | : | : | : | : | : | G¹⁰ | : |
| 1143 | 3,4-(CH₃)₂ | : | : | : | : | : | : | : | G¹⁰ | : |
| 1144 | 3,4-O-CH₂-O | : | : | : | : | : | : | : | G¹⁰ | : |
| 1145 | 3,4-Cl₂ | : | CH₃ | : | : | : | : | : | G¹⁰ | : |
| 1146 | 4-Cl | : | : | : | : | : | : | : | G¹⁰ | : |
| 1147 | 4- CH₃ | : | : | : | : | : | : | : | G¹⁰ | : |
| 1148 | 4- OCH₂CH₃ | : | : | : | : | : | : | : | G¹⁰ | : |
| 1149 | 3,4- (CH₃)₂ | : | : | : | : | : | : | : | G¹⁰ | : |
| 1150 | 4-Br | : | : | : | : | : | : | : | G¹⁰ | : |
| 1151 | 4- OCH₂CF₃ | : | : | : | : | : | : | : | G¹⁰ | : |
| 1152 | 3-Cl,4-CH₃ | : | : | : | : | : | : | : | G¹⁰ | : |
| 1153 | 4-F | : | : | : | : | : | : | : | G¹⁰ | : |
| 1154 | 4- CF₃ | : | : | : | : | : | : | : | G¹⁰ | : |
| 1155 | 6- OCH₂CF₃ | : | : | : | 3-pyridyl | : | : | : | G¹⁰ | : |
| 1156 | 6-Cl | : | : | : | : | : | : | : | G¹⁰ | : |
| 1157 | H | : | : | : | : | : | : | : | G¹⁰ | : |
| 1158 | 6- CH₃ | : | : | : | : | : | : | : | G¹⁰ | : |
| 1159 | 4-Cl | H | Ethyl | CH₃ | phenyl | S | 0 | 1 | G¹² | H |
| 1160 | : | : | H | : | : | SO | : | : | G¹² | : |
| 1161 | : | : | : | : | : | SO₂ | : | : | G¹² | : |
| 1162 | : | : | : | : | : | O | : | : | G¹² | : |
| 1163 | 4-Cl | : | : | : | : | : | : | : | G¹² | : |
| 1164 | 4-F | : | : | : | : | : | : | : | G¹² | : |
| 1165 | 4-Br | : | : | : | : | : | : | : | G¹² | : |
| 1166 | 4- CH₃ | : | : | : | : | : | : | : | G¹² | : |
| 1167 | 4- OCH₃ | : | : | : | : | : | : | : | G¹² | : |
| 1168 | 4- OCH₂CH₃ | : | : | : | : | : | : | : | G¹² | : |
| 1169 | 4- OCH₂CF₃ | : | : | : | : | : | : | : | G¹² | : |
| 1170 | 3,4-Cl₂ | : | : | : | : | S | : | : | G¹² | : |
| 1171 | 4- CF₃ | : | : | : | : | : | : | : | G¹² | : |
| 1172 | 3-Cl,4-CH₃ | : | : | : | : | : | : | : | G¹² | : |
| 1173 | 3,4-(CH₃)₂ | : | : | : | : | : | : | : | G¹² | : |
| 1174 | 3,4-O-CH₂-O | : | : | : | : | : | : | : | G¹² | : |
| 1175 | 3,4-Cl₂ | : | CH₃ | : | : | : | : | : | G¹² | : |
| 1176 | 4-Cl | : | : | : | : | : | : | : | G¹² | : |
| 1177 | 4- CH₃ | : | : | : | : | : | : | : | G¹² | : |
| 1178 | 4- OCH₂CH₃ | : | : | : | : | : | : | : | G¹² | : |
| 1179 | 3,4- (CH₃)₂ | : | : | : | : | : | : | : | G¹² | : |
| 1180 | 4-Br | : | : | : | : | : | : | : | G¹² | : |
| 1181 | 4- OCH₂CF₃ | : | : | : | : | : | : | : | G¹² | : |
| 1182 | 3-Cl,4-CH₃ | : | : | : | : | : | : | : | G¹² | : |
| 1183 | 4-F | : | : | : | : | : | : | : | G¹² | : |
| 1184 | 4- CF₃ | : | : | : | : | : | : | : | G¹² | : |
| 1185 | 6- OCH₂CF₃ | : | : | : | 3-pyridyl | : | : | : | G¹² | : |
| 1186 | 6-Cl | : | : | : | : | : | : | : | G¹² | : |
| 1187 | H | : | : | : | : | : | : | : | G¹² | : |
| 1188 | 6- CH₃ | : | : | : | : | : | : | : | G¹² | : |
| 1189 | 4-Cl | H | Ethyl | CH₃ | phenyl | S | 0 | 1 | G¹⁶ | H |
| 1190 | : | : | H | : | : | SO | : | : | G¹⁶ | : |
| 1191 | : | : | : | : | : | SO₂ | : | : | G¹⁶ | : |
| 1192 | : | : | : | : | : | O | : | : | G¹⁶ | : |
| 1193 | 4-Cl | : | : | : | : | S | : | : | G¹⁶ | : |
| 1194 | 4-F | : | : | : | : | : | : | : | G¹⁶ | : |
| 1195 | 4-Br | : | : | : | : | : | : | : | G¹⁶ | : |
| 1196 | 4- CH₃ | : | : | : | : | : | : | : | G¹⁶ | : |
| 1197 | 4- OCH₃ | : | : | : | : | : | : | : | G¹⁶ | : |
| 1198 | 4- OCH₂CH₃ | : | : | : | : | : | : | : | G¹⁶ | : |
| 1199 | 4- OCH₂CF₃ | : | : | : | : | : | : | : | G¹⁶ | : |
| 1200 | 3,4-Cl₂ | : | : | : | : | : | : | : | G¹⁶ | : |
| 1201 | 4- CF₃ | : | : | : | : | : | : | : | G¹⁶ | : |
| 1202 | 3-Cl,4-CH₃ | : | : | : | : | : | : | : | G¹⁶ | : |
| 1203 | 3,4-(CH₃)₂ | : | : | : | : | : | : | : | G¹⁶ | : |
| 1204 | 3,4-O-CH₂-O | : | : | : | : | : | : | : | G¹⁶ | : |
| 1205 | 3,4-Cl₂ | : | CH₃ | : | : | : | : | : | G¹⁶ | : |
| 1206 | 4-Cl | : | : | : | : | : | : | : | G¹⁶ | : |
| 1207 | 4- CH₃ | : | : | : | : | : | : | : | G¹⁶ | : |
| 1208 | 4- OCH₂CH₃ | : | : | : | : | : | : | : | G¹⁶ | : |
| 1209 | 3,4- (CH₃)₂ | : | : | : | : | : | : | : | G¹⁶ | : |
| 1210 | 4-Br | : | : | : | : | : | : | : | G¹⁶ | : |
| 1211 | 4- OCH₂CF₃ | : | : | : | : | : | : | : | G¹⁶ | : |
| 1212 | 3-Cl,4-CH₃ | : | : | : | : | : | : | : | G¹⁶ | : |
| 1213 | 4-F | : | : | : | : | : | : | : | G¹⁶ | : |
| 1214 | 4- CF₃ | : | : | : | : | : | : | : | G¹⁶ | : |
| 1215 | 6- OCH₂CF₃ | : | : | : | 3-pyridyl | : | : | : | G¹⁶ | : |
| 1216 | 6-Cl | : | : | : | : | : | : | : | G¹⁶ | : |
| 1217 | H | : | : | : | : | : | : | : | G¹⁶ | : |
| 1218 | 6- CH₃ | : | : | : | : | : | : | : | G¹⁶ | : |
| 1219 | 4-Cl | H | Ethyl | CH₃ | phenyl | S | 0 | 1 | G²² | H |
| 1220 | : | : | H | : | : | SO | : | : | G²² | : |
| 1221 | : | : | : | : | : | SO₂ | : | : | G²² | : |
| 1222 | : | : | : | : | : | O | : | : | G²² | : |
| 1223 | 4-Cl | : | : | : | : | S | : | : | G²² | : |
| 1224 | 4-F | : | : | : | : | : | : | : | G²² | : |
| 1225 | 4-Br | : | : | : | : | : | : | : | G²² | : |
| 1226 | 4- CH₃ | : | : | : | : | : | : | : | G²² | : |
| 1227 | 4- OCH₃ | : | : | : | : | : | : | : | G²² | : |
| 1228 | 4- OCH₂CH₃ | : | : | : | : | : | : | : | G²² | : |
| 1229 | 4- OCH₂CF₃ | : | : | : | : | : | : | : | G²² | : |
| 1230 | 3,4-Cl₂ | : | : | : | : | : | : | : | G²² | : |
| 1231 | 4- CF₃ | : | : | : | : | : | : | : | G²² | : |
| 1232 | 3-Cl,4-CH₃ | : | : | : | : | : | : | : | G²² | : |
| 1233 | 3,4-(CH₃)₂ | : | : | : | : | : | : | : | G²² | : |
| 1234 | 3,4-O-CH₂-O | : | : | : | : | : | : | : | G²² | : |
| 1235 | 3,4-Cl₂ | : | CH₃ | : | : | : | : | : | G²² | : |
| 1236 | 4-Cl | : | : | : | : | : | : | : | G²² | : |
| 1237 | 4-CH₃ | : | : | : | : | : | : | : | G²² | : |
| 1238 | 4- OCH₂CH₃ | : | : | : | : | : | : | : | G²² | : |
| 1239 | 3,4-(CH₃)₂ | : | : | : | : | : | : | : | G²² | : |
| 1240 | 4-Br | : | : | : | : | : | : | | G²² | : |
| 1241 | 4- OCH₂CF₃ | : | : | : | : | : | : | G²² | : | : |
| 1242 | 3-Cl,4-CH₃ | : | : | : | : | : | : | : G²² | : | : |
| 1243 | 4-F | : | : | : | : | : | : | G²² | : | : |
| 1244 | 4- CF₃ | : | : | : | : | : | : | G²² | : | : |
| 1245 | 6- OCH₂CF₃ | : | : | : | 3-pyridyl | : | : | G²² | : | : |
| 1256 | 6-Cl | : | : | : | : | : | : | G²² | : | : |
| 1247 | H | : | : | : | : | : | : | G²² | : | : |
| 1248 | 6- CH₃ | : | : | : | : | : | : | G²² | : | : |
| 1249 | 4-Cl | H | Ethyl | CH₃ | phenyl | S | 0 | 1 | G²⁸ | H |
| 1250 | : | : | H | : | : | SO | : | : | G²⁸ | : |
| 1251 | : | : | : | : | : | SO₂ | : | : | G²⁸ | : |
| 1252 | : | : | : | : | : | O | : | : | G²⁸ | : |
| 1253 | 4-Cl | : | : | : | : | S | : | : | G²⁸ | : |
| 1254 | 4-F | : | : | : | : | : | : | : | G²⁸ | : |
| 1255 | 4-Br | : | : | : | : | : | : | : | G²⁸ | : |
| 1256 | 4- CH₃ | : | : | : | : | : | : | : | G²⁸ | : |
| 1257 | 4- OCH₃ | : | : | : | : | : | : | : | G²⁸ | : |
| 1258 | 4- OCH₂CH₃ | : | : | : | : | : | : | : | G²⁸ | : |
| 1259 | 4- OCH₂CF₃ | : | : | : | : | : | : | : | G²⁸ | : |
| 1260 | 3,4-Cl₂ | : | : | : | : | : | : | : | G²⁸ | : |
| 1261 | 4- CF₃ | : | : | : | : | : | : | : | G²⁸ | : |
| 1262 | 3-Cl,4-CH₃ | : | : | : | : | : | : | : | G²⁸ | : |
| 1263 | 3,4-(CH₃)₂ | : | : | : | : | : | : | : | G²⁸ | : |
| 1264 | 3,4-O-CH₂-O | : | : | : | : | : | : | : | G²⁸ | : |
| 1265 | 3,4-Cl₂ | : | CH₃ | : | : | : | : | : | G²⁸ | : |
| 1266 | 4-Cl | : | : | : | : | : | : | : | G²⁸ | : |
| 1267 | 4- CH₃ | : | : | : | : | : | : | : | G²⁸ | : |
| 1268 | 4- OCH₂CH₃ | : | : | : | : | : | : | : | G²⁸ | : |
| 1269 | 3,4- (CH₃)₂ | : | : | : | : | : | : | : | G²⁸ | : |
| 1270 | 4-Br | : | : | : | : | : | : | : | G²⁸ | : |
| 1271 | 4- OCH₂CF₃ | : | : | : | : | : | : | : | G²⁸ | : |
| 1272 | 3-Cl,4-CH₃ | : | : | : | : | : | : | : | G²⁸ | : |
| 1273 | 4-F | : | : | : | : | : | : | : | G²⁸ | : |
| 1274 | 4- CF₃ | : | : | : | : | : | : | : | G²⁸ | : |
| 1275 | 6- OCH₂CF₃ | : | : | : | 3-pyridyl | : | : | : | G²⁸ | : |
| 1276 | 6-Cl | : | : | : | : | : | : | : | G²⁸ | : |
| 1277 | H | : | : | : | : | : | : | : | G²⁸ | : |
| 1278 | 6- CH₃ | : | : | : | : | : | : | : | G²⁸ | : |
| 1279 | 4-Cl | H | Ethyl | CH₃ | phenyl | S | 0 | 1 | G²⁹ | H |
| 1280 | : | : | H | : | : | SO | : | : | G²⁹ | : |
| 1281 | : | : | : | : | : | SO₂ | : | : | G²⁹ | : |
| 1282 | : | : | : | : | : | O | : | : | G²⁹ | : |
| 1283 | 4-Cl | : | : | : | : | S | : | : | G²⁹ | : |
| 1284 | 4-F | : | : | : | : | : | : | : | G²⁹ | : |
| 1285 | 4- CH₂CH₃ | : | : | : | : | : | : | : | G²⁹ | : |
| 1286 | 4- CH₃ | : | : | : | : | : | : | : | G²⁹ | : |
| 1287 | 4- OCH₃ | : | : | : | : | : | : | : | G²⁹ | : |
| 1288 | 4- OCH₂CH₃ | : | : | : | : | : | : | : | G²⁹ | : |
| 1289 | 4- OCH₂CF₃ | : | : | : | : | : | : | : | G²⁹ | : |
| 1290 | 3,4-Cl₂ | : | : | : | : | : | : | : | G²⁹ | : |
| 1291 | 4- CF₃ | : | : | : | : | : | : | : | G²⁹ | : |
| 1292 | 3-Cl,4-CH₃ | : | : | : | : | : | : | : | G²⁹ | : |
| 1293 | 3,4-(CH₃)₂ | : | : | : | : | : | : | : | G²⁹ | : |
| 1294 | 3,4-O-CH₂-O | : | : | : | : | : | : | : | G²⁹ | : |
| 1295 | 3,4-Cl₂ | : | CH₃ | : | : | : | : | : | G²⁹ | : |
| 1296 | 4-Cl | : | : | : | : | : | : | : | G²⁹ | : |
| 1297 | 4- CH₃ | : | : | : | : | : | : | : | G²⁹ | : |
| 1298 | 4- OCH₂CH₃ | : | : | : | : | : | : | : | G²⁹ | : |
| 1299 | 3,4- (CH₃)₂ | : | : | : | : | : | : | : | G²⁹ | : |
| 1300 | 4-Br | : | : | : | : | : | : | : | G²⁹ | : |
| 1301 | 4- OCH₂CF₃ | : | : | : | : | : | : | : | G²⁹ | : |
| 1302 | 3-Cl,4-CH₃ | : | : | : | : | : | : | : | G²⁹ | : |
| 1303 | 4-F | : | : | : | : | : | : | : | G²⁹ | : |
| 1304 | 4- CF₃ | : | : | : | : | : | : | : | G²⁹ | : |
| 1305 | 6- OCH₂CF₃ | : | : | : | 3-pyridyl | : | : | : | G²⁹ | : |
| 1306 | 6-Cl | : | : | : | : | : | : | : | G²⁹ | : |
| 1307 | H | : | : | : | : | : | : | : | G²⁹ | : |
| 1308 | 6- CH₃ | : | : | : | : | : | : | : | G²⁹ | : |
| 1309 | 4- OCH₂CH₃ | H | H | CH₃ | phenyl | S | 0 | 1 | G³ | H |
| 1310 | 4-Cl | : | : | : | : | : | : | : | G³ | : |
| 1311 | 4- CH₃ | : | : | : | : | : | : | : | G³ | : |
| 1312 | 4- OCH₂CH₃ | : | CH₃ | : | : | : | : | : | G³ | : |
| 1313 | 4-Cl | : | : | : | : | : | : | : | G³ | : |
| 1314 | H | : | : | : | 3-pyridyl | : | : | : | G³ | : |
| 1315 | 6-Cl | : | : | : | : | : | : | : | G³ | : |
| 1316 | 4-Cl | : | : | : | phenyl | O | : | : | G³ | : |
| 1317 | 4-Cl | : | : | : | : | SO₂ | : | : | G³ | : |
| 1318 | 4-Cl | : | : | : | : | SO | : | : | G³ | : |
| 1319 | 4- OCH₂CH₃ | H | H | CH₃ | phenyl | S | : | : | G³ | H |
| 1320 | 4-Cl | : | : | : | : | : | : | : | G⁵ | : |
| 1321 | 4- CH₃ | : | : | : | : | : | : | : | G⁵ | : |
| 1322 | 4-CH₃ | : | CH₃ | : | : | : | : | : | G⁵ | : |
| 1323 | 4-Cl | : | : | : | : | : | : | : | G⁵ | : |
| 1324 | H | : | : | : | 3-pyridyl | : | : | : | G⁵ | : |
| 1325 | 6-Cl | : | : | : | : | : | : | : | G⁵ | : |
| 1326 | 4-Cl | : | : | : | phenyl | O | : | : | G⁵ | : |
| 1327 | 4-Cl | : | : | : | : | SO₂ | : | : | G⁵ | : |
| 1328 | 4-Cl | : | : | : | : | SO | : | : | G⁵ | : |
| 1329 | 4- OCH₂CH₃ | H | H | CH₃ | phenyl | S | : | : | G⁶ | H |
| 1330 | 4-Cl | : | : | : | : | : | : | : | G⁶ | : |
| 1331 | 4- CH₃ | : | : | : | : | : | : | : | G⁶ | : |
| 1332 | 4- OCH₃ | : | CH₃ | : | : | : | : | : | G⁶ | : |
| 1333 | 4-Cl | : | : | : | : | : | : | : | G⁶ | : |
| 1334 | H | : | : | : | 3-pyridyl | : | : | : | G⁶ | : |
| 1335 | 6-Cl | : | : | : | : | : | : | : | G⁶ | : |
| 1336 | 4- Cl | : | : | : | phenyl | O | : | : | G⁶ | : |
| 1337 | 4- Cl | : | : | : | : | SO₂ | : | : | G⁶ | : |
| 1338 | 4-Cl | : | : | : | : | SO | : | : | G⁶ | : |
| 1339 | 4- OCH₂CH₃ | H | H | CH₃ | phenyl | S | 0 | 1 | G⁷ | H |
| 1340 | 4-Cl | : | : | : | : | : | : | : | G⁷ | : |
| 1341 | 4- CH₃ | : | : | : | : | : | : | : | G⁷ | : |
| 1342 | 4-CH₃ | : | CH₃ | : | : | : | : | : | G⁷ | : |
| 1343 | 4-Cl | : | : | : | : | : | : | : | G⁷ | : |
| 1344 | H | : | : | : | 3-pyridyl | : | : | : | G⁷ | : |
| 1345 | 6-Cl | : | : | : | : | : | : | : | G⁷ | : |
| 1346 | 4- Cl | : | : | : | phenyl | O | : | : | G⁷ | : |
| 1347 | 4- Cl | : | : | : | : | SO₂ | : | : | G⁷ | : |
| 1348 | 4- Cl | : | : | : | : | SO | : | : | G⁷ | : |
| 1349 | 4- OCH₂CH₃ | H | H | CH₃ | phenyl | S | : | : | G¹³ | H |
| 1350 | 4-Cl | : | : | : | : | : | : | : | G¹³ | : |
| 1351 | 4- CH₃ | : | : | : | : | : | : | : | G¹³ | : |
| 1352 | 4- CH₃ | : | CH₃ | : | : | : | : | : | G¹³ | : |
| 1353 | 4-Cl | : | : | : | : | : | : | : | G¹³ | : |
| 1354 | H | : | : | : | 3-pyridyl | : | : | : | G¹³ | : |
| 1355 | 6-Cl | : | : | : | : | : | : | : | G¹³ | : |
| 1356 | 4- Cl | : | : | : | phenyl | O | : | : | G¹³ | : |
| 1357 | 4- Cl | : | : | : | : | SO₂ | : | : | G¹³ | : |
| 1358 | 4- Cl | : | : | : | : | SO | : | : | G¹³ | : |
| 1359 | 4- OCH₂CH₃ | H | H | CH₃ | phenyl | S | : | : | G¹⁴ | H |
| 1360 | 4-Cl | : | : | : | : | : | : | : | G¹⁴ | : |
| 1361 | 4- CH₃ | : | : | : | : | : | : | : | G¹⁴ | : |
| 1362 | 4- CH₃ | : | CH₃ | : | : | : | : | : | G¹⁴ | : |
| 1363 | 4-Cl | : | : | : | : | : | : | : | G¹⁴ | : |
| 1364 | H | : | : | : | 3-pyridyl | : | : | : | G¹⁴ | : |
| 1365 | 6-Cl | : | : | : | : | : | : | : | G¹⁴ | : |
| 1366 | 4-Cl | : | : | : | phenyl | O | : | : | G¹⁴ | : |
| 1367 | 4- Cl | : | : | : | : | SO₂ | : | : | G¹⁴ | : |
| 1368 | 4- Cl | : | : | : | : | SO | : | : | G¹⁴ | : |
| 1369 | 4- OCH₂CH₃ | H | H | CH₃ | phenyl | S | 0 | 1 | G¹⁷ | H |
| 1370 | 4-Cl | : | : | : | : | : | : | : | G¹⁷ | : |
| 1371 | 4- CH₃ | : | : | : | : | : | : | : | G¹⁷ | : |
| 1372 | 4- CH₃ | : | CH₃ | : | : | : | : | : | G¹⁷ | : |
| 1373 | 4-Cl | : | : | : | : | : | : | : | G¹⁷ | : |
| 1374 | H | : | : | : | 3-pyridyl | : | : | : | G¹⁷ | : |
| 1375 | 6-Cl | : | : | : | : | : | : | : | G¹⁷ | : |
| 1376 | 4- Cl | : | : | : | phenyl | O | : | : | G¹⁷ | : |
| 1377 | 4- Cl | : | : | : | : | SO₂ | : | : | G¹⁷ | : |
| 1378 | 4- Cl | : | : | : | : | SO | : | : | G¹⁷ | : |
| 1379 | 4- OCH₂CH₃ | H | H | CH₃ | phenyl | S | : | : | G¹⁸ | H |
| 1380 | 4-Cl | : | : | : | : | : | : | : | G¹⁸ | : |
| 1381 | 4- CH₃ | : | : | : | : | : | : | : | G¹⁸ | : |
| 1382 | 4- CH₃ | : | CH₃ | : | : | : | : | : | G¹⁸ | : |
| 1383 | 4-Cl | : | : | : | : | : | : | : | G¹⁸ | : |
| 1384 | H | : | : | : | 3-pyridyl | : | : | : | G¹⁸ | : |
| 1385 | 6-Cl | : | : | : | : | : | : | : | G¹⁸ | : |
| 1386 | 4-Cl | : | : | : | phenyl | O | : | : | G¹⁸ | : |
| 1387 | 4-Cl | : | : | : | : | SO₂ | : | : | G¹⁸ | : |
| 1388 | 4-Cl | : | : | : | : | SO | : | : | G¹⁸ | : |
| 1389 | 4- OCH₂CH₃ | H | H | CH₃ | phenyl | S | : | : | G¹⁹ | H |
| 1390 | 4-Cl | : | : | : | : | : | : | : | G¹⁹ | : |
| 1391 | 4- CH₃ | : | : | : | : | : | : | : | G¹⁹ | : |
| 1392 | 4- CH₃ | : | CH₃ | : | : | : | : | : | G¹⁹ | : |
| 1393 | 4-Cl | : | : | : | : | : | : | : | G¹⁹ | : |
| 1394 | H | : | : | : | 3-pyridyl | : | : | : | G¹⁹ | : |
| 1395 | 6-Cl | : | : | : | : | : | : | : | G¹⁹ | : |
| 1396 | 4-Cl | : | : | : | phenyl | O | : | : | G¹⁹ | : |
| 1397 | 4-Cl | : | : | : | : | SO₂ | : | : | G¹⁹ | : |
| 1398 | 4-Cl | : | : | : | : | SO | : | : | G¹⁹ | : |
| 1399 | 4- OCH₂CH₃ | H | H | CH₃ | phenyl | S | 0 | 1 | G²⁰ | H |
| 1400 | 4-Cl | : | : | : | : | : | : | : | G²⁰ | : |
| 1401 | 4- CH₃ | : | : | : | : | : | : | : | G²⁰ | : |
| 1402 | 4- CH₃ | : | CH₃ | : | : | : | : | : | G²⁰ | : |
| 1403 | 4-Cl | : | : | : | : | : | : | : | G²⁰ | : |
| 1404 | H | : | : | : | 3-pyridyl | : | : | : | G²⁰ | : |
| 1405 | 6-Cl | : | : | : | : | : | : | : | G²⁰ | : |
| 1406 | 4-Cl | : | : | : | phenyl | O | : | : | G²⁰ | : |
| 1407 | 4-Cl | : | : | : | : | SO₂ | : | : | G²⁰ | : |
| 1408 | 4-Cl | : | : | : | : | SO | : | : | G²⁰ | : |
| 1409 | 4- OCH₂CH₃ | H | H | CH₃ | phenyl | S | : | : | G²¹ | H |
| 1410 | 4-Cl | : | : | : | : | : | : | : | G²¹ | : |
| 1411 | 4- CH₃ | : | : | : | : | : | : | : | G²¹ | : |
| 1412 | 4- CH₃ | : | CH₃ | : | : | : | : | : | G²¹ | : |
| 1413 | 4-Cl | : | : | : | : | : | : | : | G²¹ | : |
| 1414 | H | : | : | : | 3-pyridyl | : | : | : | G²¹ | : |
| 1415 | 6-Cl | : | : | : | : | : | : | : | G²¹ | : |
| 1416 | 4-Cl | : | : | : | phenyl | O | : | : | G²¹ | : |
| 1417 | 4-Cl | : | : | : | : | SO₂ | : | : | G²¹ | : |
| 1418 | 4-Cl | : | : | : | : | SO | : | : | G²¹ | : |
| 1419 | 4- OCH₂CH₃ | H | H | CH₃ | phenyl | S | : | : | G²³ | H |
| 1420 | 4-Cl | : | : | : | : | : | : | : | G²³ | : |
| 1421 | 4- CH₃ | : | : | : | : | : | : | : | G²³ | : |
| 1422 | 4- CH₃ | : | CH₃ | : | : | : | : | : | G²³ | : |
| 1423 | 4-Cl | : | : | : | : | : | : | : | G²³ | : |
| 1424 | H | : | : | : | 3-pyridyl | : | : | : | G²³ | : |
| 1425 | 6-Cl | : | : | : | : | : | : | : | G²³ | : |
| 1426 | 4-Cl | : | : | : | phenyl | O | : | : | G²³ | : |
| 1427 | 4-Cl | : | : | : | : | SO₂ | : | : | G²³ | : |
| 1428 | 4-Cl | : | : | : | : | SO | : | : | G²³ | : |
| 1429 | 4- OCH₂CH₃ | H | H | CH₃ | phenyl | S | 0 | 1 | G²⁵ | H |
| 1430 | 4-Cl | : | : | : | : | : | : | : | G²⁵ | : |
| 1431 | 4- CH₃ | : | : | : | : | : | : | : | G²⁵ | : |
| 1432 | 4- CH₃ | : | CH₃ | : | : | : | : | : | G²⁵ | : |
| 1433 | 4-Cl | : | : | : | : | : | : | : | G²⁵ | : |
| 1434 | H | : | : | : | 3-pyridyl | : | : | : | G²⁵ | : |
| 1435 | 6-Cl | : | : | : | : | : | : | : | G²⁵ | : |
| 1436 | 4-Cl | : | : | : | phenyl | O | : | : | G²⁵ | : |
| 1437 | 4-Cl | : | : | : | : | SO₂ | : | : | G²⁵ | : |
| 1438 | 4-Cl | : | : | : | : | SO | : | : | G²⁵ | : |
| 1439 | 4- OCH₂CH₃ | H | H | CH₃ | phenyl | S | : | : | G²⁶ | H |
| 1440 | 4-Cl | : | : | : | : | : | : | : | G²⁶ | : |
| 1441 | 4- CH₃ | : | : | : | : | : | : | : | G²⁶ | : |
| 1442 | 4- CH₃ | : | CH₃ | : | : | : | : | : | G²⁶ | : |
| 1443 | 4-Cl | : | : | : | : | : | : | : | G²⁶ | : |
| 1444 | H | : | : | : | 3-pyridyl | : | : | : | G²⁶ | : |
| 1445 | 6-Cl | : | : | : | : | : | : | : | G²⁶ | : |
| 1446 | 4-Cl | : | : | : | phenyl | O | : | : | G²⁶ | : |
| 1447 | 4-Cl | : | : | : | : | SO₂ | : | : | G²⁶ | : |
| 1448 | 4-Cl | : | : | : | : | SO | : | : | G²⁶ | : |
| 1449 | 4- OCH₃ | H | H | CH₃ | phenyl | S | : | : | G²⁷ | H |
| 1450 | 4-Cl | : | : | : | : | : | : | : | G²⁷ | : |
| 1451 | 4- CH₃ | : | : | : | : | : | : | : | G²⁷ | : |
| 1452 | 4- CH₃ | : | CH₃ | : | : | : | : | : | G²⁷ | : |
| 1453 | 4-Cl | : | : | : | : | : | : | : | G²⁷ | : |
| 1454 | H | : | : | : | 3-pyridyl | : | : | : | G²⁷ | : |
| 1455 | 6-Cl | : | : | : | : | : | : | : | G²⁷ | : |
| 1456 | 4-Cl | : | : | : | phenyl | O | : | : | G²⁷ | : |
| 1457 | 4-Cl | : | : | : | : | SO₂ | : | : | G²⁷ | : |
| 1458 | 4-Cl | : | : | : | : | SO | : | : | G²⁷ | : |
| 1459 | 4-Cl | H | Ethyl | CH₃ | phenyl | S | 0 | 1 | G¹ | - CN |
| 1460 | : | : | H | : | : | SO | : | : | G¹ | : |
| 1461 | : | : | : | : | : | SO₂ | : | : | G¹ | : |
| 1462 | : | : | : | : | : | O | : | : | G¹ | : |
| 1463 | 4-Cl | : | : | : | : | S | : | : | G¹ | : |
| 1464 | 4-F | : | : | : | : | : | : | : | G¹ | : |
| 1465 | 4-Br | : | : | : | : | : | : | : | G¹ | : |
| 1466 | 4- CH₃ | : | : | : | : | : | : | : | G¹ | : |
| 1467 | 4- OCH₃ | : | : | : | : | : | : | : | G¹ | : |
| 1468 | 4- OCH₂CH₃ | : | : | : | : | : | : | : | G¹ | : |
| 1469 | 4- OCH₃CF₃ | : | : | : | : | : | : | : | G¹ | : |
| 1470 | 3,4-Cl₂ | : | : | : | : | : | : | : | G¹ | : |
| 1471 | 4- CF₃ | : | : | : | : | : | : | : | G¹ | : |
| 1472 | 3-Cl,4-CH₃ | : | : | : | : | : | : | : | G¹ | : |
| 1473 | 3,4-(CH₃)₂ | : | : | : | : | : | : | : | G¹ | : |
| 1474 | 3,4-O-CH₂-O | : | : | : | : | : | : | : | G¹ | : |
| 1475 | 3,4-Cl₂ | : | CH₃ | : | : | : | : | : | G¹ | : |
| 1476 | 4-Cl | : | : | : | : | : | : | : | G¹ | : |
| 1477 | 4- CH₃ | : | : | : | : | : | : | : | G¹ | : |
| 1478 | 4- OCH₂CH₃ | : | : | : | : | : | : | : | G¹ | : |
| 1479 | 3,4- (CH₃)₂ | : | : | : | : | : | : | : | G¹ | : |
| 1480 | 4-Br | : | : | : | : | : | : | : | G¹ | : |
| 1481 | 4- OCH₂CF₃ | : | : | : | : | : | : | : | G¹ | : |
| 1482 | 3-Cl,4-CH₃ | : | : | : | : | : | : | : | G¹ | : |
| 1483 | 4-F | : | : | : | : | : | : | : | G¹ | : |
| 1484 | 4- CF₃ | : | : | : | : | : | : | : | G¹ | : |
| 1485 | 6- OCH₂CF₃ | : | : | : | 3-pyridyl | : | : | : | G¹ | : |
| 1486 | 6-Cl | : | : | : | : | : | : | : | G¹ | : |
| 1487 | H | : | : | : | : | : | : | : | G¹ | : |
| 1488 | 6- CH₃ | : | : | : | : | : | : | : | G¹ | : |
| 1489 | 4-Cl | H | Ethyl | CH₃ | phenyl | S | 0 | 1 | G¹ | -Cl |
| 1490 | : | : | H | : | : | SO | : | : | G¹ | : |
| 1491 | : | : | : | : | : | SO₂ | : | : | G¹ | : |
| 1492 | : | : | : | : | : | O | : | : | G¹ | : |
| 1493 | 4-Cl | : | : | : | : | S | : | : | G¹ | : |
| 1494 | 4-F | : | : | : | : | : | : | : | G¹ | : |
| 1495 | 4-Br | : | : | : | : | : | : | : | G¹ | : |
| 1496 | 4- CH₃ | : | : | : | : | : | : | : | G¹ | : |
| 1497 | 4- OCH₃ | : | : | : | : | : | : | : | G¹ | : |
| 1498 | 4- OCH₂CH₃ | : | : | : | : | : | : | : | G¹ | : |
| 1499 | 4- OCH₃CF₃ | : | : | : | : | : | : | : | G¹ | : |
| 1500 | 3,4-Cl₂ | : | : | : | : | : | : | : | G¹ | : |
| 1501 | 4- CF₃ | : | : | : | : | : | : | : | G¹ | : |
| 1502 | 3-Cl,4-CH₃ | : | : | : | : | : | : | : | G¹ | : |
| 1503 | 3,4-(CH₃)₂ | : | : | : | : | : | : | : | G¹ | : |
| 1504 | 3,4-O-CH₂-O | : | : | : | : | : | : | : | G¹ | : |
| 1505 | 3,4-Cl₂ | : | CH₃ | : | : | : | : | : | G¹ | : |
| 1506 | 4-Cl | : | : | : | : | : | : | : | G¹ | : |
| 1507 | 4- CH₃ | : | : | : | : | : | : | : | G¹ | : |
| 1508 | 4- OCH₂CH₃ | : | : | : | : | : | : | : | G¹ | : |
| 1509 | 3,4- (CH₃)₂ | : | : | : | : | : | : | : | G¹ | : |
| 1510 | 4-Br | : | : | : | : | : | : | : | G¹ | : |
| 1511 | 4- OCH₂CF₃ | : | : | : | : | : | : | : | G¹ | : |
| 1512 | 3-Cl,4-CH₃ | : | : | : | : | : | : | : | G¹ | : |
| 1513 | 4-F | : | : | : | : | : | : | : | G¹ | : |
| 1514 | 4- CF₃ | : | : | : | : | : | : | : | G¹ | : |
| 1515 | 6- OCH₂CF₃ | : | : | : | 3-pyridyl | : | : | : | G¹ | : |
| 1516 | 6-Cl | : | : | : | : | : | : | : | G¹ | : |
| 1517 | H | : | : | : | : | : | : | : | G¹ | : |
| 1518 | 6- CH₃ | : | : | : | : | : | : | : | G¹ | : |
| 1519 | 4-Cl | H | Ethyl | CH₃ | phenyl | S | 0 | 1 | G¹ | CH₃ |
| 1520 | : | : | H | : | : | SO | : | : | G¹ | : |
| 1521 | : | : | : | : | : | SO₂ | : | : | G¹ | : |
| 1522 | : | : | : | : | : | O | : | : | G¹ | : |
| 1523 | 4-Cl | : | : | : | : | S | : | : | G¹ | : |
| 1524 | 4-F | : | : | : | : | : | : | : | G¹ | : |
| 1525 | 4-Br | : | : | : | : | : | : | : | G¹ | : |
| 1526 | 4- CH₃ | : | : | : | : | : | : | : | G¹ | : |
| 1527 | 4- OCH₃ | : | : | : | : | : | : | : | G¹ | : |
| 1528 | 4- OCH₂CH₃ | : | : | : | : | : | : | : | G¹ | : |
| 1529 | 4- OCH₂CF₃ | : | : | : | : | : | : | : | G¹ | : |
| 1530 | 3,4-Cl₂ | : | : | : | : | : | : | : | G¹ | : |
| 1531 | 4- CF₃ | : | : | : | : | : | : | : | G¹ | : |
| 1532 | 3-Cl,4-CH₃ | : | : | : | : | : | : | : | G¹ | : |
| 1533 | 3,4-(CH₃)₂ | : | : | : | : | : | : | : | G¹ | : |
| 1534 | 3,4-O-CH₂-O | : | : | : | : | : | : | : | G¹ | : |
| 1535 | 3,4-Cl₂ | : | CH₃ | : | : | : | : | : | G¹ | : |
| 1536 | 4-Cl | : | : | : | : | : | : | : | G¹ | : |
| 1537 | 4- CH₃ | : | : | : | : | : | : | : | G¹ | : |
| 1538 | 4- OCH₂CH₃ | : | : | : | : | : | : | : | G¹ | : |
| 1539 | 3,4- (CH₃)₂ | : | : | : | : | : | : | : | G¹ | : |
| 1540 | 4-Br | : | : | : | : | : | : | : | G¹ | : |
| 1541 | 4- OCH₂CF₃ | : | : | : | : | : | : | : | G¹ | : |
| 1542 | 3-Cl,4-CH₃ | : | : | : | : | : | : | : | G¹ | : |
| 1543 | 4-F | : | : | : | : | : | : | : | G¹ | : |
| 1544 | 4- CF₃ | : | : | : | : | : | : | : | G¹ | : |
| 1545 | 6- OCH₂CF₃ | : | : | : | 3-pyridyl | : | : | : | G¹ | : |
| 1546 | 6-Cl | : | : | : | : | : | : | : | G¹ | : |
| 1547 | H | : | : | : | : | : | : | : | G¹ | : |
| 1548 | 6- CH₃ | : | : | : | : | : | : | : | G¹ | : |
| 1549 | 4-Cl | H | Ethyl | CH₃ | phenyl | S | 0 | 1 | G¹ | OCH₃ |
| 1550 | : | : | H | : | : | SO | : | : | G¹ | : |
| 1551 | : | : | : | : | : | SO₂ | : | : | G¹ | : |
| 1552 | : | : | : | : | : | O | : | : | G¹ | : |
| 1553 | 4-Cl | : | : | : | : | S | : | : | G¹ | : |
| 1554 | 4-F | : | : | : | : | : | : | : | G¹ | : |
| 1555 | 4-Br | : | : | : | : | : | : | : | G¹ | : |
| 1556 | 4- CH₃ | : | : | : | : | : | : | : | G¹ | : |
| 1557 | 4- OCH₃ | : | : | : | : | : | : | : | G¹ | : |
| 1558 | 4- OCH₂CH₃ | : | : | : | : | : | : | : | G¹ | : |
| 1559 | 4- OCH₂CF₃ | : | : | : | : | : | : | : | G¹ | : |
| 1560 | 3,4-Cl₂ | : | : | : | : | : | : | : | G¹ | : |
| 1561 | 4-CF₃ | : | : | : | : | : | : | : | G¹ | : |
| 1562 | 3-Cl,4-CH₃ | : | : | : | : | : | : | : | G¹ | : |
| 1563 | 3,4-(CH₃)₂ | : | : | : | : | : | : | : | G¹ | : |
| 1564 | 3,4-O-CH₂-O | : | : | : | : | : | : | : | G¹ | : |
| 1565 | 3,4-Cl₂ | : | CH₃ | : | : | : | : | : | G¹ | : |
| 1566 | 4-Cl | : | : | : | : | : | : | : | G¹ | : |
| 1567 | 4- CH₃ | : | : | : | : | : | : | : | G¹ | : |
| 1568 | 4- OCH₂CH₃ | : | : | : | : | : | : | : | G¹ | : |
| 1569 | 3,4- (CH₃)₂ | : | : | : | : | : | : | : | G¹ | : |
| 1570 | 4-Br | : | : | : | : | : | : | : | G¹ | : |
| 1571 | 4- OCH₂CF₃ | : | : | : | : | : | : | : | G¹ | : |
| 1572 | 3-Cl,4-CH₃ | : | : | : | : | : | : | : | G¹ | : |
| 1573 | 4-F | : | : | : | : | : | : | : | G¹ | : |
| 1574 | 4- CF₃ | : | : | : | : | : | : | : | G¹ | : |
| 1575 | 6- OCH₂CF₃ | : | : | : | 3-pyridyl | : | : | : | G¹ | : |
| 1576 | 6-Cl | : | : | : | : | : | : | : | G¹ | : |
| 1577 | H | : | : | : | : | : | : | : | G¹ | : |
| 1578 | 6- CH₃ | : | : | : | : | : | : | : | G¹ | : |
| 1579 | H | H | H | CH₃ | phenyl | NH | 0 | 1 | G¹ | H |
| 1580 | 4-Cl | : | : | : | : | : | : | : | : | : |
| 1581 | 4- CH₃ | : | : | : | : | : | : | : | : | : |
| 1582 | 4- OCH₃ | : | : | : | : | : | : | : | : | : |
| 1583 | H | : | CH₃ | : | : | : | : | : | : | : |
| 1584 | 4-Cl | : | : | : | : | : | : | : | : | : |
| 1585 | 4- CH₃ | : | : | : | : | : | : | : | : | : |
| 1586 | 4- OCH₃ | : | : | : | : | : | : | : | : | : |
| 1587 | 4- OCH₂CH₃ | / | / | CH₃ | : | S | 0 | 0 | : | : |
| 1588 | 4-Cl | / | / | : | : | : | : | : | : | : |
| 1589 | 4- CH₃ | / | / | : | : | : | : | : | : | : |
| 1590 | 4- OCH₃ | / | / | : | : | : | : | : | : | : |
| 1591 | 4- OCH₂CH₃ | / | / | Ethyl | : | S | 0 | 0 | : | : |
| 1592 | 4-Cl | / | / | : | : | : | : | : | : | : |
| 1593 | 4- CH₃ | / | / | : | : | : | : | : | : | : |
| 1594 | 4- OCH₃ | / | / | : | : | : | : | : | : | : |
| 1595 | 4- OCH₂CH₃ | / | / | *i*-Propyl | : | S | 0 | 0 | : | : |
| 1596 | 4-Cl | / | / | : | : | : | : | : | : | : |
| 1597 | 4- CH₃ | / | / | : | : | : | : | : | : | : |
| 1598 | 4- OCH₃ | / | / | : | : | : | : | : | : | : |
| 1599 | 4- OCH₂CH₃ | / | / | *n*-Propyl | : | S | 0 | 0 | : | : |
| 1600 | 4-Cl | / | / | : | : | : | : | : | : | : |
| 1601 | 4- CH₃ | / | / | : | : | : | : | : | : | : |
| 1602 | 4- OCH₃ | / | / | : | : | : | : | : | : | : |
| 1603 | 4- OCH₂CH₃ | / | / | Phenyl | : | S | 0 | 0 | : | : |
| 1604 | 4-Cl | / | / | : | : | : | : | : | : | : |
| 1605 | 4- CH₃ | / | / | : | : | : | : | : | : | : |
| 1606 | 4- OCH₃ | / | / | : | : | : | : | : | : | : |
| 1607 | 4-Cl | / | / | Methyl | : | O | 0 | 0 | : | : |
| 1608 | 4-Cl | / | / | H | : | NH | 0 | 0 | : | : |
| 1609 | 4- OCH₃ | H | H | CH₃ | phenyl | S | 0 | 1 | G³² | H |
| 1610 | 4-Cl | : | : | : | : | : | : | : | G³² | : |
| 1611 | 4- CH₃ | : | : | : | : | : | : | : | G³² | : |
| 1612 | 4-CH₃ | : | CH₃ | : | : | : | : | : | G³² | : |
| 1613 | 4-Cl | : | : | : | : | : | : | : | G³² | : |
| 1614 | 6- CH₃ | : | : | : | 3-pyridyl | : | : | : | G³² | : |
| 1615 | 6-Cl | : | : | : | : | : | : | : | G³² | : |
| 1616 | 4- OCH₃ | H | H | CH₃ | phenyl | S | 0 | 1 | G³³ | H |
| 1617 | 4-Cl | : | : | : | : | : | : | : | G³³ | : |
| 1618 | 4- CH₃ | : | : | : | : | : | : | : | G³³ | : |
| 1619 | 4- CH₃ | : | CH₃ | : | : | : | : | : | G³³ | : |
| 1620 | 4-Cl | : | : | : | : | : | : | : | G³³ | : |
| 1621 | 6- CH₃ | : | : | : | 3-pyridyl | : | : | : | G³³ | : |
| 1622 | 6-Cl | : | : | : | : | : | : | : | G³³ | : |
| 1623 | 4- OCH₃ | H | H | CH₃ | phenyl | S | 0 | 1 | G³⁴ | H |
| 1624 | 4-Cl | : | : | : | : | : | : | : | G³⁴ | : |
| 1625 | 4- CH₃ | : | : | : | : | : | : | : | G³⁴ | : |
| 1626 | 4- CH₃ | : | CH₃ | : | : | : | : | : | G³⁴ | : |
| 1627 | 4-Cl | : | : | : | : | : | : | : | G³⁴ | : |
| 1628 | 6- CH₃ | : | : | : | 3-pyridyl | : | : | : | G³⁴ | : |
| 1629 | 6-Cl | : | : | : | : | : | : | : | G³⁴ | : |
| 1630 | 4- OCH₃ | H | H | CH₃ | phenyl | S | 0 | 1 | G³⁵ | H |
| 1631 | 4-Cl | : | : | : | : | : | : | : | G³⁵ | : |
| 1632 | 4- CH₃ | : | : | : | : | : | : | : | G³⁵ | : |
| 1633 | 4- CH₃ | : | CH₃ | : | : | : | : | : | G³⁵ | : |
| 1634 | 4-Cl | : | : | : | : | : | : | : | G¹⁵ | : |
| 1635 | 6- CH₃ | : | : | : | 3-pyridyl | : | : | : | G³⁵ | : |
| 1636 | 6-Cl | : | : | : | : | : | : | : | G³⁵ | : |
| 1637 | 4-Cl | : | : | : | : | SO₂ | : | : | G³² | : |
| 1638 | 4-Cl | : | : | : | : | SO | : | : | G³³ | : |

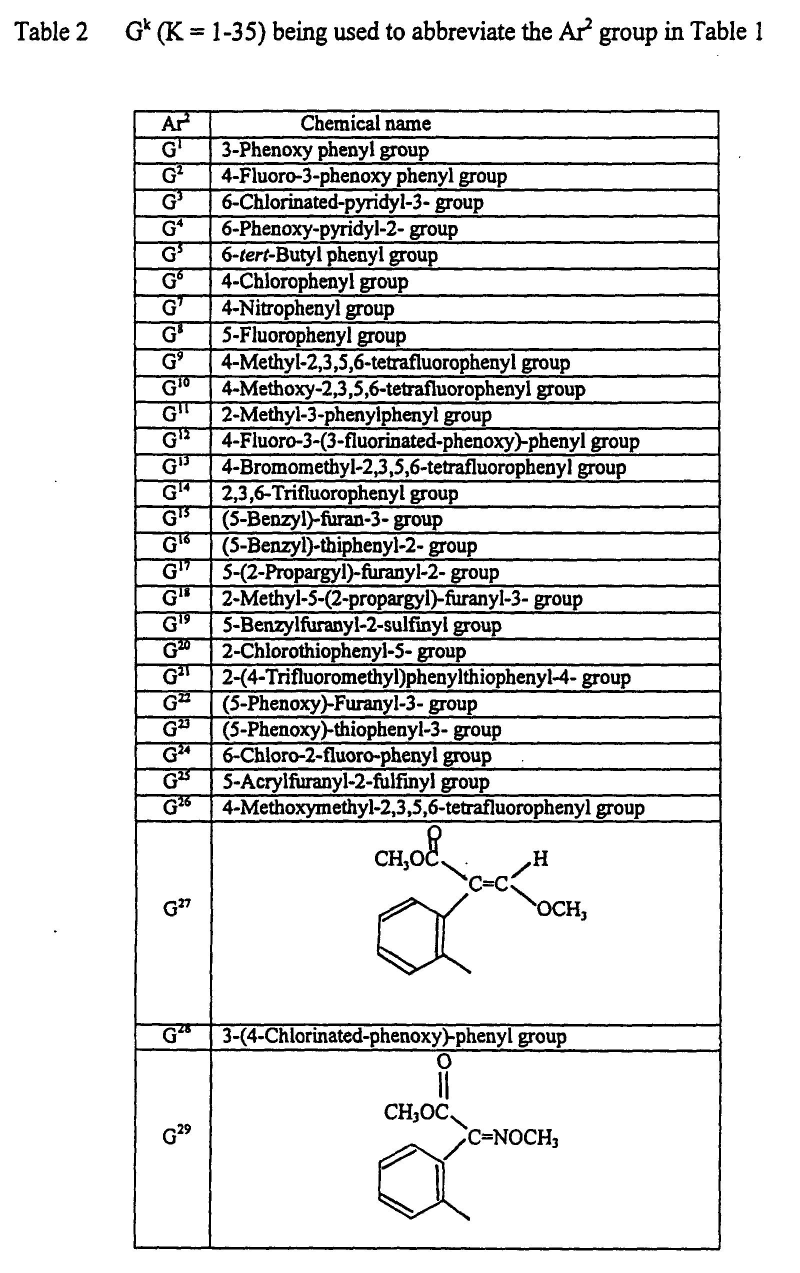

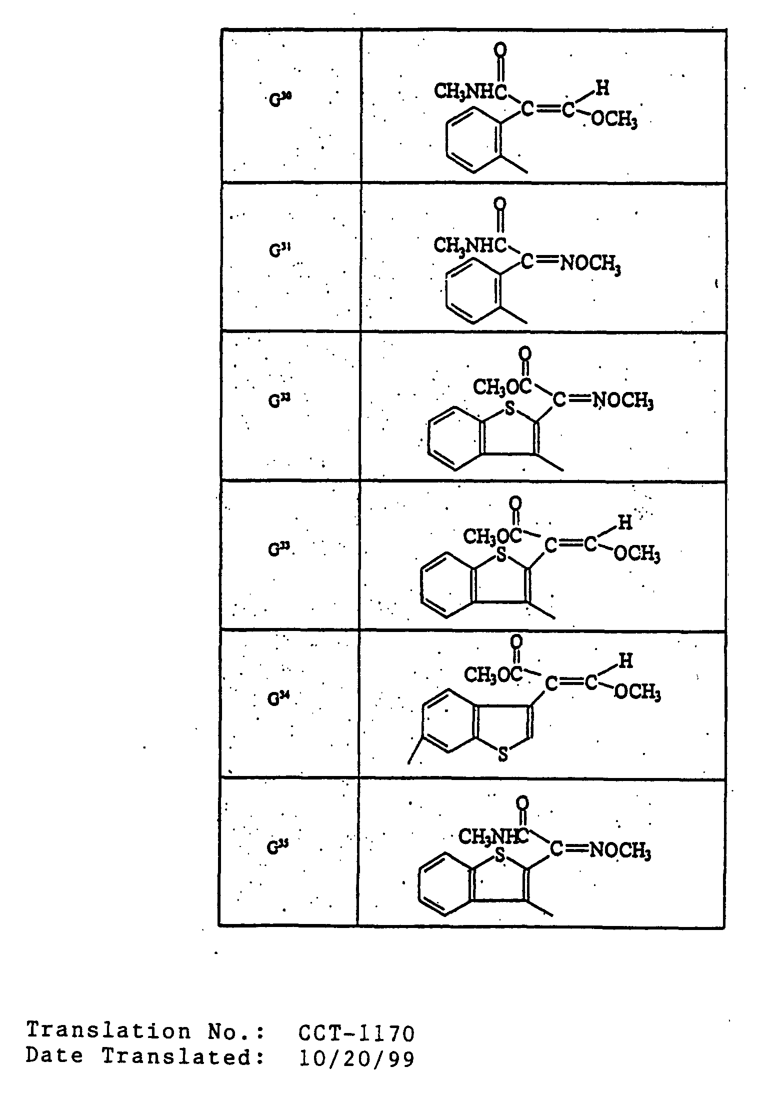

## Claims

1. Biocidal alkyl-substituted-(hetero)aryl-ketoxime-o-ethers, intermediate ketones, and oxime compounds, represented by general formula (I), (II) and (III): 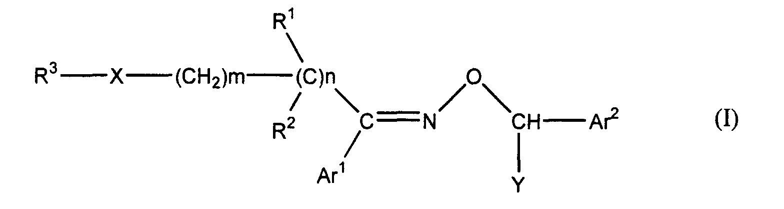 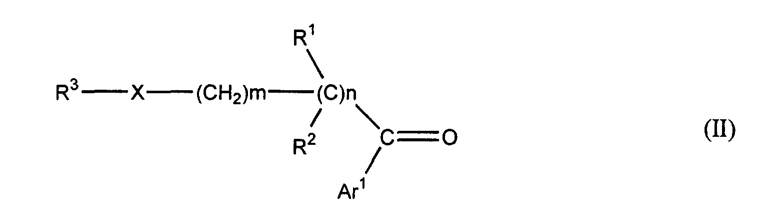 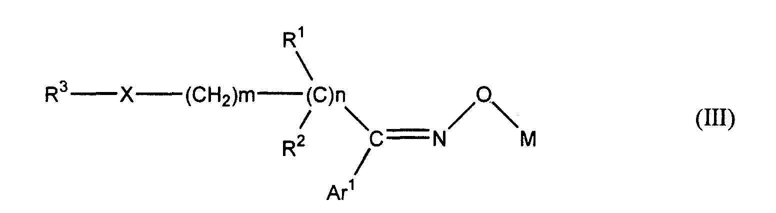 wherein:
Ar1 and Ar2 may be identical or different, and they represent:
(a) C₆ - C₁₂ aryl group or heteroaryl group having no more than 10 carbon atoms, or
(b) the group defined in (a) substituted with no more than five identical or different substituting groups, selected from the following :
halogens, nitro group, cyano group, (C₁ - C₆) alkyl groups, (C₁ - C₆) halogen-substituted alkyl groups, cyano-(C₁ - C₆) alkyl groups, (C₁ - C₆) alkoxy-(C₁- C₆) alkyl groups, (C₁ - C₆)-alkylthio group-(C₁ - C₆) alkyl groups, (C₁ - C₆) alkoxy groups, (C₁ - C₆) alkoxy-(C₁ - C₆) alkoxy groups, (C₁ - C₆) halogen-substituted alkoxy groups, (C₁ - C₆) halogen-substituted-alkoxy-alkyl groups, (C₁ - C₆) alkylthio groups, (C₁ - C₆) halogen-substituted alkylthio groups, (C₁ - C₆) alkyl-sulfonyl groups, (C₁ - C₆) alkyl-sulfinyl groups, (C₁ - C₆) alkoxy-carbonyl groups, (C₁ - C₆) alkylamino group, 2-(C₁ - C₆) alkylamino groups, linear (C₂ - C₆) alkenyl groups, linear (C₂ - C₆) alkenoxy groups, linear (C₂ - C₆) alkenoxy-alkyl groups, linear (C₂ - C₆) halogen-substituted alkenyl groups, linear (C₂ - C₆) halogen-substituted alkenoxy group, linear (C₂ - C₆) halogen-substituted alkenoxy-alkyl groups, (C₂ - C₆) alkynyl groups, (C₂ - C₆) alkenyloxy groups, (C₂ - C₆) halogen-substituted alkynyl groups, (C₂ - C₆) halogen-substituted alkynoxy groups, (C₃ - C₈) cycloalkyl groups, (C₃ - C₈) cycloalkyloxy groups, (C₃ - C₈) cycloalkylamino groups, (C₆ - C₁₂) aryl groups, (C₆ - C₁₂) aryl-thio groups, (C₆ - C₁₂) aryl-(C₁ - C₄) alkyl groups, (C₆ - C₁₂) aryl-oxy-carbonyl groups, (C₆
- C₁₂) aryl-sulfonyl groups, (C₆ - C₁₂) aryl-sulfinyl groups, (C₆ - C₁₂ arylamino group, heteroaryl groups, heteroaryloxy groups, heteroaryl-(C₆ - C₁₂) alkyl group, heteroaryl-thio groups, heteroaryloxy-carbonyl groups, heteroaryl-sulfonyl groups, and heteroaryl-sulfinyl groups, and
provided that:
1) when the substituting group in (b) is an aryl group or heteroaryl group, it may be substituted by one or more identical or different group(s) selected from (C₁ - C₆) alkyl groups, (C₁ - C₆) alkoxy groups, (C₁ - C₆) halogen-substituted alkyl groups, (C₁ - C₆) halogen-substituted alkoxy groups, and halogens, and the heteroaryl group is the group having no more than 10 carbon atoms;
2) the cycloalkyl group described in (b) may be substituted by no more than five identical or different groups selected from halogens and (C₁ - C₄) alkyl groups;
3) when two of the substituting groups described in (b) represent methylene dioxy group or ethylene dioxy group, they may have one or two identical or different substituting group(s) selected from halogens and (C₁ - C₆) alkyl groups; and
4) the aryl group and the heteroaryl group defined in (a) and (b) may be hydrogenated partially or entirely, and one or two CH₂ groups may be substituted with CO;
R₁ and R₂ may be identical or different, and they represent the following:
hydrogen, halogens, (C₁ - C₆) alkyl groups, (C₁ - C₆) halogenated-alkyl groups, cyano-(C₁ - C₆) alkyl groups, (C₁ - C₆) alkoxy-(C₁ - C₆) alkyl groups, (C₁ - C₆) alkylthio-(C₁ - C₆) alkyl groups, (C₁ - C₆) alkoxy-(C₁ - C₆) alkoxy groups, (C₁ - C₆) alkoxy groups, (C₁ - C₆) alkylthio groups, (C₁ - C₆) alkyl-sulfonyl groups, (C₁ - C₆) alkyl-sulfinyl groups, (C₁ - C₆) alkoxy-carbonyl groups, (C₁ - C₆) alkylamino groups, 2-(C₁ - C₆) alkylamino groups, (C₁ - C₆) halogen-substituted alkoxy groups, linear (C₂- C₆) alkenyl groups, linear (C₂ - C₆) alkenoxy groups, linear (C₂ - C₆) halogenated alkenyl groups, linear (C₂ - C₆) halogenated alkenoxy groups, (C₂ - C₆) alkynyl groups, (C₂ - C₆) alkynoxy groups, (C₂ - C₆) halogenated alkynyl groups, (C₂ - C₆) halogenated alkynoxy groups, (C₃ - C₈) cycloalkyl groups, (C₃ - C₈) cycloalkyloxy groups, (C₃ - C₈) cycloalkylamino groups, (C₆ - C₁₂) aryl groups, (C₆ - C₁₂) aryloxy groups, (C₆ - C₁₂) aryl-(C₁ - C₄) alkyl groups, (C₆ - C₁₂) arylthio groups, (C₆ - C₁₂) aryloxy-carbonyl groups, (C₆ - C₁₂) aryl-sulfonyl groups, (C₆ - C₁₂) aryl-sulfinyl groups, (C₆ - C₁₂) arylamino groups, heteroaryl groups, heteroaryloxy groups, heteroaryl-(C₁ - C₄) alkyl groups, heteroarylthio groups, heteroaryloxy-carbonyl groups, heteroarylsulfonyl groups, and heteroaryl sulfinyl groups;
R₃ represents the following:
(a) hydrogen;
(b) (C₁ - C₆) alkyl groups, (C₆ - C₁₂) aryl groups, heteroaryl groups with no more than 10 carbon atoms, linear (C₂ - C₆) alkenyl groups, (C₂ - C₆) alkynyl groups, and (C₁ - C₄) acyl groups;
wherein the groups described in (b) may be substituted by identical or different groups selected from halogens, (C₁ - C₄) alkyl groups, (C₁ - C₄) alkoxy groups, (C₁ - C₄) halogen-substituted alkyl groups, (C₁ - C₄) alkyl-thio groups, and (C₁ - C₄) halogenated alkoxy groups;
X represents O, S, SO, SO₂, NH, or NR^{a};
wherein R^{a} represents a (C₁ - C₄) alkyl group;
Y represents hydrogen, cyano group, halogens, (C₁ - C₄) alkyl group, or (C₁ - C₄) alkoxy group;
M is a hydrogen or a monobasic metallic atom; and
m or n are independently 0, 1, 2 or 3; and
their enantiomers, stereoisomers, and cis and trans forms.

2. The alkyl-substituted-(hetero)aryl-ketoxime-o-ether, intermediate ketones, and oxime compounds according to Claim 1, wherein Ar1 is one of the following groups: 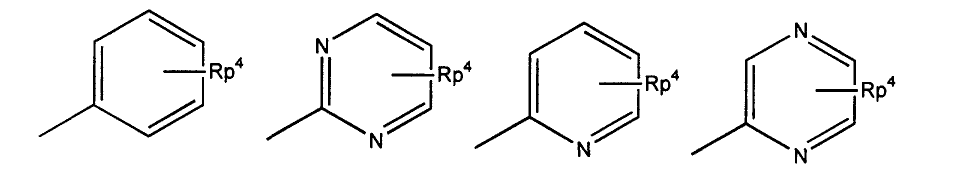 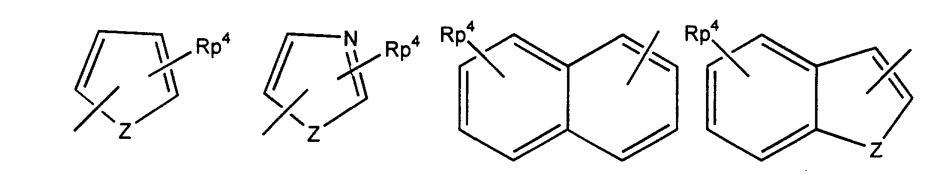 wherein:
Z is O, S or NR⁵, wherein R⁵ is a hydrogen, (C₁ - C₆) alkyl group, (C₁ - C₆) halogen-substituted alkyl group, (C₁ - C₆) alkoxy-(C₁ - C₆) alkyl group, (C₁ - C₆) alkylthio-(C₁ - C₆) alkyl group, cyano-(C₁ - C₆) alkyl group, (C₁ - C₆) alkyloxy carbonyl group, linear (C₂ - C₆) alkenyl group, linear (C₂ - C₆) halogenated alkenyl group, (C₂ - C₆) alkynyl group, (C₂ - C₆) halogenated alkynyl group, (C₃ - C₈) cycloalkyl group optionally substituted by no more than five identical or different groups selected from halogens and (C₁ - C₄) alkyl groups, (C₆ - C₁₂) aryl group, (C₆ - C₁₂) aryl-(C₁ - C₄) alkyl group, (C₆ - C₁₂) aryloxy carbonyl group, and heteroaryl, heteroaryloxy carbonyl, and heteroaryl-(C₁ - C₄) alkyl groups having no more than 10 aryl carbons;
P is an integer 0 to 5; and
each R₄ may be identical or different, and represents halogens, nitro groups, cyano groups, (C₁ - C₆) alkyl groups, (C₁ - C₆) halogenated alkyl groups, cyano-(C₁ - C₆) alkyl groups, (C₁ - C₆) alkoxy-(C₁ - C₆) alkyl groups, (C₁ - C₆) alkylthio-(C₁ - C₆) alkyl groups, (C₁ - C₆) alkoxy groups, (C₁ - C₆) alkoxy-(C₁ - C₆) alkoxy groups, (C₁ - C₆) halogenated alkoxy groups, (C₁ - C₆) halogenated alkoxy-alkyl groups, (C₁ - C₆) alkylthio groups, (C₁ - C₆) halogenated alkylthio groups, (C₁ - C₆) alkyl-sulfonyl groups, (C₁ - C₆) alkyl-sulfinyl groups, (C₁ - C₆) alkoxy-carbonyl groups, (C₁ - C₆) alkylamino groups, 2-(C₁ - C₆) alkylamino groups, linear (C₂ - C₆) alkenyl groups, linear (C₂ - C₆) alkenoxy groups, linear (C₂ - C₆) alkenoxy alkyl groups, linear (C₂ - C₆) halogenated alkenyl groups, linear (C₂ - C₆) halogenated alkenoxy groups, linear (C₂ - C₆) halogenated alkenoxy-alkyl group, (C₂ - C₆) alkynyl groups, (C₂ - C₆) alkenoxy groups, (C₂ - C₆) halogenated alkynyl groups, (C₂ - C₆) halogenated alkynoxy groups, (C₃ - C₈) cycloalkyl groups, (C₃ - C₈) cycloalkyloxy groups, (C₃ - C₈) cycloalkylamino groups, (C₆ - C₁₂) aryl groups, (C₆ - C₁₂) aryloxy groups, (C₆ - C₁₂) arylthio groups, (C₆ - C₁₂) aryl-(C₁ - C₄) alkyl groups, (C₆ - C₁₂) aryloxy carbonyl groups, (C₆ - C₁₂) aryl-sulfonyl groups, (C₆ - C₁₂) arylsulfinyl groups, (C₆ - C₁₂) arylamino groups, heteroaryl groups, heteroaryloxy groups, heteroaryl-(C₁ - C₄) alkyl groups, heteroaryl-thio groups, heteroaryloxy carbonyl groups, heteroaryl-sulfonyl groups, and heteroaryl-sulfinyl groups; provided that:
1) when the substituting group in R⁴ is an aryl group or a hetero-aryl group, it may be substituted by one or more identical or different group(s) selected from (C₁ - C₆) alkyl groups, (C₁ - C₆) alkoxy groups, (C₁ - C₆) halogen-substituted alkyl groups, (C₁ - C₆) halogen-substituted alkoxy groups, and halogens, and the heteroaryl group has no more than 10 carbon atoms;
2) the cycloalkyl group described in R₄ may be substituted by no more than five identical or different groups selected from halogens and (C₁ - C₄) alkyl groups; and
3) when two substituting groups described in R₄ represent methylene dioxy group and ethylene dioxy group they may have one or two identical or different substituting group(s) selected from halogens and (C₁ - C₆) alkyl groups.

3. The alkyl-substituted-(hetero)aryl-ketoxime-o-ethers, intermediate ketones, and oxime compounds according to Claim 1, wherein Ar1 is one of the following groups: 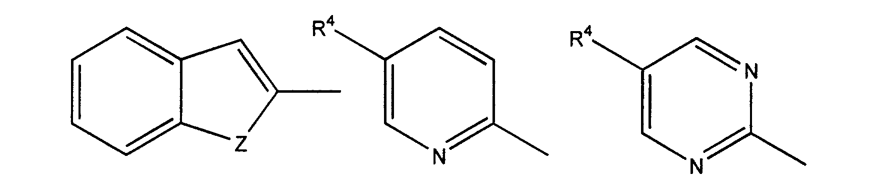 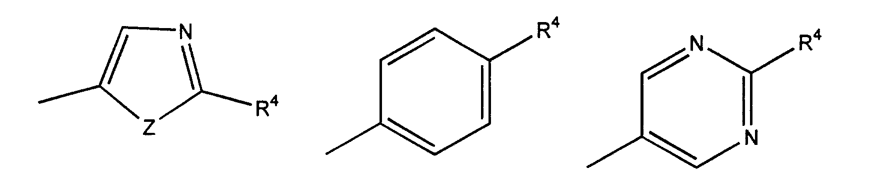 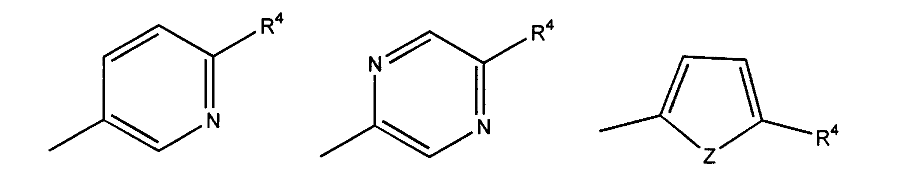 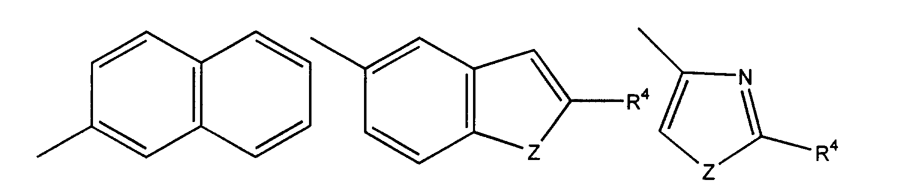 wherein:
Z is O, S or NR⁵, and R⁵ is a hydrogen, (C₁ - C₆) alkyl group, (C₁ - C₆) halogen-substituted alkyl group, (C₁ - C₆) alkoxy-(C₁ - C₆) alkyl group, (C₁ - C₆) alkylthio-(C₁ - C₆) alkyl group, cyano-(C₁ - C₆) alkyl group, (C₁ - C₆) alkyloxy carbonyl group, linear (C₂ - C₆) alkenyl group, linear (C₂ - C₆) halogenated alkenyl group, (C₂ - C₆) alkynyl group, (C₂ - C₆) halogenated alkynyl group, (C₃ - C₈) cycloalkyl group optionally substituted by no more than five identical or different groups selected from halogens and (C₁ - C₄) alkyl groups, (C₆ - C₁₂) aryl group, (C₆ - C₁₂) aryl-(C₁ - C₄) alkyl group, (C₆ - C₁₂) aryloxy carbonyl group, and heteroaryl, heteroaryloxy carbonyl, and heteroaryl-(C₁ - C₄) alkyl groups having no more than 10 aryl carbons; and
each R₄ may be identical or different, and represents halogens, nitro groups, cyano groups, (C₁ - C₆) alkyl groups, (C₁ - C₆) halogenated alkyl groups, cyano-(C₁ - C₆) alkyl groups, (C₁ - C₆) alkoxy-(C₁ - C₆) alkyl groups, (C₁ - C₆) alkylthio-(C₁ - C₆) alkyl groups, (C₁ - C₆) alkoxy groups, (C₁ - C₆) alkoxy-(C₁ - C₆) alkoxy groups, (C₁ - C₆) halogenated alkoxy groups, (C₁ - C₆) halogenated alkoxy-alkyl groups, (C₁ - C₆) alkylthio groups, (C₁ - C₆) halogenated alkylthio groups, (C₁ - C₆) alkyl-sulfonyl groups, (C₁ - C₆) alkyl-sulfinyl groups, (C₁ - C₆) alkoxy-carbonyl groups, (C₁ - C₆) alkylamino groups, 2-(C₁ - C₆) alkylamino groups, linear (C₂ - C₆) alkenyl groups, linear (C₂ - C₆) alkenoxy groups, linear (C₂ - C₆) alkenoxy alkyl groups, linear (C₂ - C₆) halogenated alkenyl groups, linear (C₂ - C₆) halogenated alkenoxy groups, linear (C₂ - C₆) halogenated alkenoxy-alkyl group, (C₂ - C₆) alkynyl groups, (C₂ - C₆) alkenoxy groups, (C₂ - C₆) halogenated alkynyl groups, (C₂ - C₆) halogenated alkynoxy groups, (C₃ - C₈) cycloalkyl groups, (C₃ - C₈) cycloalkyloxy groups, (C₃ - C₈) cycloalkylamino groups, (C₆ - C₁₂) aryl groups, (C₆ - C₁₂) aryloxy groups, (C₆ - C₁₂) arylthio groups, (C₆ - C₁₂) aryl-(C₁ - C₄) alkyl groups, (C₆ - C₁₂) aryloxy carbonyl groups, (C₆ - C₁₂) aryl-sulfonyl groups, (C₆ - C₁₂) arylsulfinyl groups, (C₆ - C₁₂) arylamino groups, heteroaryl groups, heteroaryloxy groups, heteroaryl-(C₁ - C₄) alkyl groups, heteroaryl-thio groups, heteroaryloxy carbonyl groups, heteroaryl-sulfonyl groups, and heteroaryl-sulfinyl groups.

4. The alkyl-substituted-(hetero)aryl-ketoxime-o-ethers, intermediate ketones, and oxime compounds according to Claim 1, wherein:
R₁ and R₂ are identical or different, and are hydrogen, halogens, (C₁ - C₄) alkyl groups, (C₁ - C₄) alkoxyl groups, (C₁ - C₄) halogenated alkyl groups, (C₁ - C₄) alkylthio groups, linear (C₁ - C₄) alkenyl groups, (C₁ - C₄) alkynyl groups, linear (C₁ - C₄) halogenated alkenyl groups, (C₁ - C₄) halogenated-alkynyl groups, (C₁ - C₄) alkyl-sulfonyl groups, (C₁ - C₄) alkyl-sulfinyl groups, or (C₁ - C₄) alkylamino groups.

5. The alkyl-substituted-(hetero)aryl-ketoxime-o-ethers, intermediate ketones, and oxime compounds according to Claim 1, wherein:
R₃ is hydrogen, (C₁ - C₄) alkyl group, (C₁ - C₄) halogenated alkyl group, linear (C₁ - C₄) alkenyl group, (C₁ - C₄) alkynyl group, linear (C₁ - C₄) halogenated-alkenyl group, (C₁ - C₄) halogenated alkynyl group, or (C₆ - C₁₂) aryl group.

6. The alkyl-substituted-(hetero)aryl-ketoxime-o-ethers, intermediate ketones, and oxime compounds according to Claim 1, wherein Ar2 in the general formula (I) represents the following : 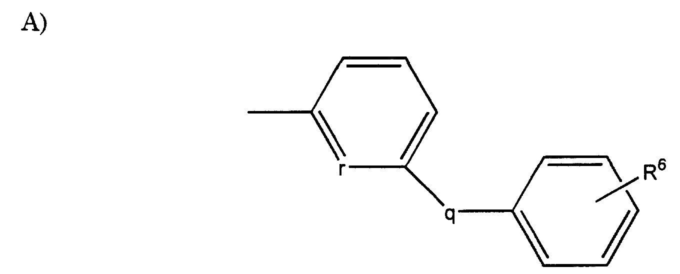 wherein:
r represents N or CH;
q is O, S, NH or CH₂; and
R⁶ is a hydrogen, halogen, acryloxy group, or propargyloxy group; 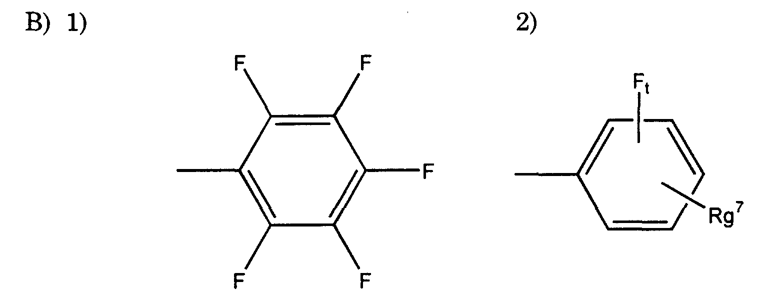 wherein:
F represents fluorine;
t and g are identical or different and represent 0, 1, 2, 3, or 4, and sum of t and g is no larger than 5;
each R⁷ may be identical or different, and represent halogens, (C₁ - C₆) alkyl groups, (C₁ - C₆) halogenated alkyl groups, (C₁ - C₆) alkoxy groups, (C₁ - C₆) alkoxy-(C₁ - C₆) alkyl groups, (C₁ - C₆) alkoxy-(C₁ - C₆) alkoxy groups, (C₁ - C₆) halogenated alkoxy groups, (C₁ - C₆) alkyl-sulfonyl groups, linear (C₂ - C₆) alkenyl groups, linear (C₂ - C₆) alkenoxy groups, linear (C₂ - C₆) alkenoxy-alkyl groups, linear (C₂ - C₆) halogenated-alkenyl groups, linear (C₂ - C₆) halogenated-alkenoxy groups, linear (C₂ - C₆) halogenated-alkenoxy-alkyl groups, (C₂ - C₆) alkynyl groups, (C₂ - C₆) alkenyloxy groups, (C₂ - C₆) halogenated-alkynyl groups, (C₂ - C₆) halogenated- alkynoxy groups, (C₃ - C₈) cycloalkyl groups, (C₃ - C₈) cycloalkyloxy groups, (C₃ - C₈) halogenated-cycloalkyl groups, (C₆ - C₁₂) aryl groups, (C₆ - C₁₂) aryloxy groups, (C₆ - C₁₂) aryl-(C₁ - C₄) alkyl groups, (C₆ - C₁₂) aryl-thio groups, heteroaryl groups having no more than 10 carbon atoms, heteroaryloxy groups, and heteroaryl-(C₁ - C₄) alkyl groups; 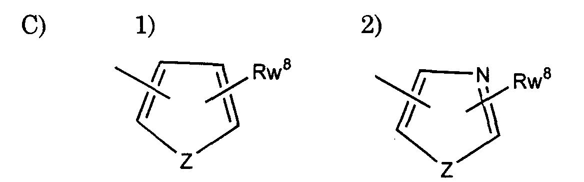
wherein:
Z is O, S, or NR⁵ wherein R⁵ is a hydrogen, (C₁ - C₆) alkyl group, (C₁ - C₆) halogen-substituted alkyl group, (C₁ - C₆) alkoxy-(C₁ - C₆) alkyl group, (C₁ - C₆) alkylthio-(C₁ - C₆) alkyl group, cyano-(C₁ - C₆) alkyl group, (C₁ - C₆) alkyloxy carbonyl group, linear (C₂ - C₆) alkenyl group, linear (C₂ - C₆) halogenated alkenyl group, (C₂ - C₆) alkynyl group, (C₂ - C₆) halogenated alkynyl group, (C₃ - C₈) cycloalkyl group optionally substituted by no more than five identical or different groups selected from halogens and (C₁ - C₄) alkyl groups, (C₆ - C₁₂) aryl group, (C₆ - C₁₂) aryl-(C₁ - C₄) alkyl group, (C₆ - C₁₂) aryloxy carbonyl group, and heteroaryl, heteroaryloxy carbonyl, and heteroaryl-(C₁ - C₄) alkyl groups having no more than 10 aryl carbons;
w represents 0, 1, 2 or 3; and
each R⁸ is identical or different, and represent halogens, (C₁ - C₆) alkyl groups, (C₁ - C₆) halogenated-alkyl groups, (C₁ - C₆) alkoxy groups, (C₁ - C₆) alkoxy-(C₁ - C₆) alkyl groups, (C₁ - C₆) alkoxy-(C₁ - C₆) alkoxy groups, (C₁ - C₆) halogenated alkoxy groups, (C₂ - C₆) linear alkenyl groups, linear (C₂ - C₆) alkenoxy groups, (C₂ - C₆) alkynyl groups, (C₂ - C₆) alkynoxy groups, (C₂ - C₆) halogenated-alkynyl groups, (C₂ - C₆) halogenated alkyloxy groups, (C₆ - C₁₂) aryloxy groups, (C₆ - C₁₂) aryl-(C₁ - C₄) alkyl groups, (C₆ - C₁₂) arylthio groups, heteroaryloxy groups having no more than 10 carbon atoms, heteroaryl-thio groups, and heteroaryl-(C₁ - C₄) alkyl groups; and
wherein the aryl group or the heteroaryl group in R⁸ is optionally substituted by (C₁ - C₆) alkyl groups, (C₁ - C₆) alkoxy groups, (C₁ - C₆) halogenated-alkyl groups, (C₁ - C₆) halogenated-alkoxy groups, and halogens, which may be identical or different. 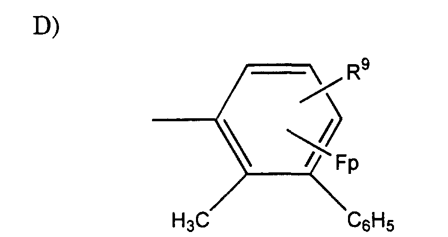
wherein:
F represents fluorine;
R⁹ is a hydrogen, fluorine or a methyl group;
p is 0, 1, or 2; or 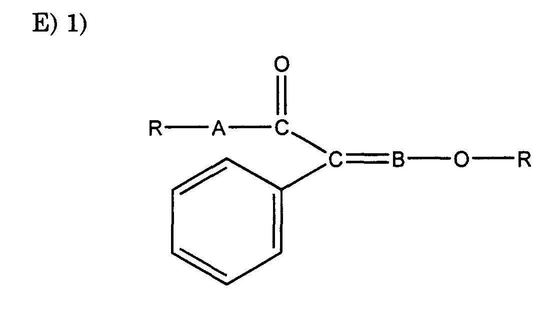 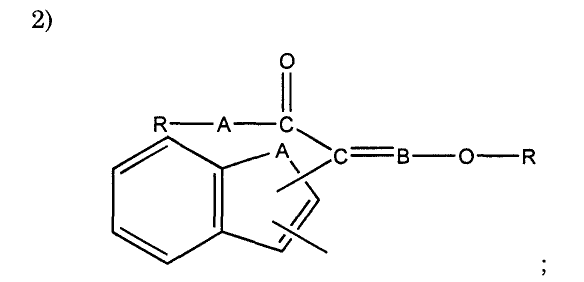
or 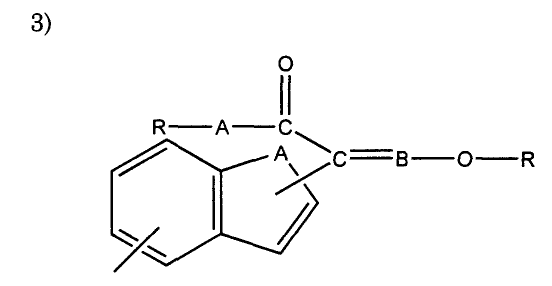 wherein:
R is a (C₁ - C₆) alkyl group or a (C₁ - C₆) halogenated-alkyl group;
A is an oxygen, sulfur or NH; and
B is a nitrogen or CH.

7. A method for preparing a compound represented by the general formula: 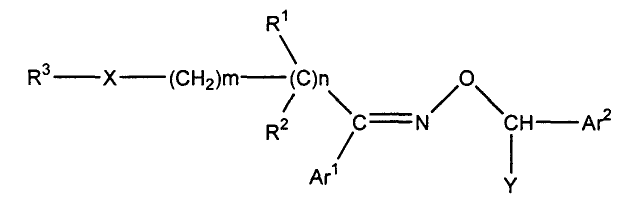 comprising the step of:
reacting a compound represented by the formula: 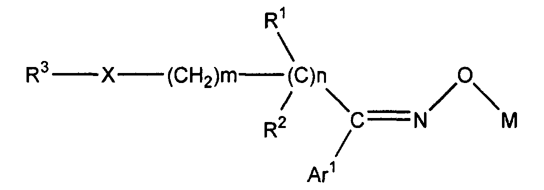 with the compound represented by the formula: 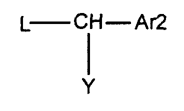
wherein R¹, R², R³, X, m, n, Ar1, M, Ar2, and Y are the same as previously defined in Claim 1 and L represents a releasable group;
in a solvent and optionally in the presence of a base or phase transfer catalyst; and
at atmospheric pressure at a temperature of from 0 to 120 °C for from 1 to 20 hours.

8. A method for preparing a compound represented by the general formula: 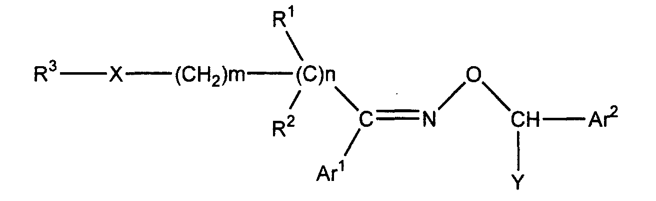 comprising the step of:
reacting a compound represented by the formula: 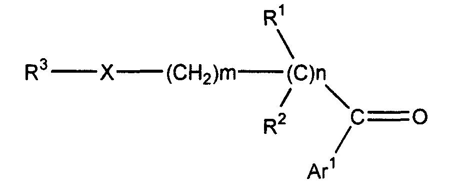 with a compound of the formula: 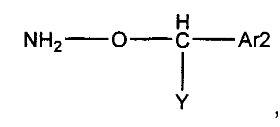 or its salt;
wherein R¹, R², R³, X, m, n, Ar1, Ar2, and Y are the same as previously defined in Claim 1 and L represents a releasable group;
in a solvent in the presence of an appropriate base or phase transfer catalyst.

9. The method of Claim 7 or Claim 8 wherein L is selected from the group consisting of: chlorine, bromine, and -OSO₂; the base is selected from the group consisting of: alkaline metal oxides, alkaline metal carbonates, alkaline metal bicarbonates, alkaline metal alcohol salts, alkaline metals, alkaline metal hydrides, pyridine, and tertiary amines; the phase transfer catalyst is selected from the group consisting of: tetrabutyl ammonium iodide, tetrabutyl ammonium bromide, tetraethyl ammonium bromide, and 18-crown ether-6; and the solvent is selected from the group consisting of: water, tetrahydrofuran, toluene, benzene, acetonitrile, and dimethylsulfoxide.

10. A method for preparing a compound of the formula: 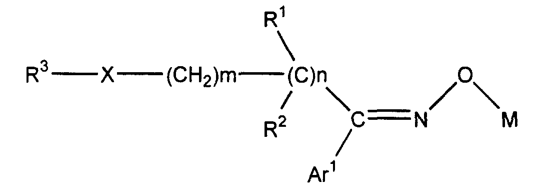 wherein R¹, R², R³, X, m, n, Ar1, and M are the same as previously defined in Claim 1;
comprising the step of reacting a compound of the formula: 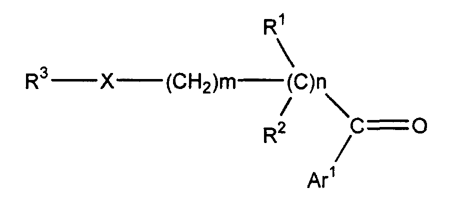 with hydroxylamine hydrochloride or hydroxylamine sulfate in an appropriate solvent under atmospheric pressure at a temperature of from 0 to 100 °C optionally in the presence of a base.

11. A method to prepare a compound of the formula: 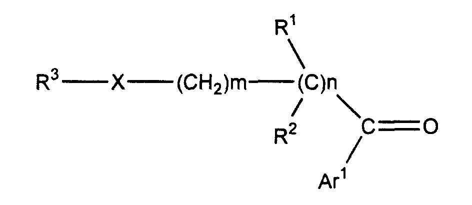 wherein R¹, R², R³, X, m, n, and Ar1 are the same as previously defined in Claim 1;
comprising the step of reacting a compound of the formula: 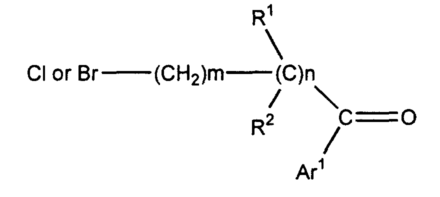 with a salt having an anion of the formula:
R³―X⁻
in an aqueous solution under atmospheric pressure at a temperature from 30 to 100°C for from 2 to 10 hours.

12. A biocidal composition comprising a compound according to Claim 1 and a carrier, and optionally one or more of extenders, emulsifiers, wetting agents, dispersing agents, adhesive agents, and degrading agents.

13. A method for controlling a pest selected from the group consisting of insect pests, fungal pathogens, and weeds, comprising contacting the pest with an effective amount of the biocidal composition of Claim 12.
